# EUROPEAN PATENT APPLICATION

(11) **EP 4 772 637 A1**
(43) Date of publication of application: **08.07.2026**
(21) Application number: 24859978.9
(22) Date of filing: 30.08.2024
(51) Int. Cl.: C12N 15/113, A61K 31/711, A61K 31/712, A61K 31/7125, A61K 47/54, A61P 29/00, A61P 43/00

(54) **ANTISENSE OLIGONUCLEOTIDE OF RASGRP4**

(30) Priority: 30.08.2023 JP 2023139977
(71) Applicant: Stratoimmune Co., Ltd., Fukuoka-shi, Fukuoka 810-0001 (JP); Nissan Chemical Corporation, Tokyo 103-6119 (JP)
(72) Inventor: SEO, Toru, Fukuoka-shi, Fukuoka 810-0001 (JP); IRIYAMA, Yusuke, Funabashi-shi, Chiba 274-8507 (JP); MATSUOKA, Seiya, Funabashi-shi, Chiba 274-8507 (JP); YASUDA, Shinsuke, Tokyo 113-8510 (JP)
(74) Representative: dompatent
(86) International application number: PCT/JP2024/031282
(87) International publication number: WO 2025/047953

(57) **Abstract**

The present invention provides a compound, which is an antisense oligonucleotide which consists of 8 to 80 linked nucleosides and having a nucleobase sequence including at least 8 contiguous nucleobases complementary to a transcript of RasGRP4, as an antisense oligonucleotide capable of controlling RasGRP4 gene expression and treating myositis and rheumatoid arthritis, or a pharmacologically acceptable salt thereof.

## Description

### Technical Field

The present invention relates to an antisense oligonucleotide of RasGRP4.

### Background Art

Polymyositis/dermatomyositis is an autoimmune disease in which inflammation occurs throughout the body, primarily in muscles, skin, and lungs, and mainly causes muscle weakness predominantly in proximal muscles. PM/DM is often accompanied by interstitial pneumonia, which can lead to a poor prognosis. The number of patients with PM/DM in Japan was 17,000 (in 2010), and the prevalence has been reported to be 13.2 per 100,000 population (see, for example, Non Patent Literature 1). Since the exact etiology remains unknown, standard treatment primarily includes administration of a non-specific immunosuppressive agent including glucocorticoids and other immunosuppressive agents. These agents have problems such as side effects or emergence of treatment resistance, and therefore development of a novel therapeutic drug for PM/DM has been required.

RasGRP4 (Ras guanine nucleotide-releasing protein 4) is a guanine exchange factor that activates Ras, and its expression is high in myelocytes such as monocytes, macrophages, neutrophils, mast cells. It is reported that fibroblast-like synoviocytes (FLS) from rheumatoid arthritis patients exhibit increased RasGRP4 expression, that the RasGRP4 mRNA expression level in FLS is positively correlated with cell proliferative capacity, and that when RasGRP4 is knocked down using siRNA, the cell proliferative capacity of FLS is reduced (see, for example, Non Patent Literature 2). The literature further reports that in collagen-induced arthritis rats, administration of RasGRP4 siRNA in the ankle joint significantly reduces the ankle arthritis score and the ankle joint diameter as compared with the control, and also reduces bone destruction and cartilage destruction. It is also reported that RasGRP4 knockout mice exhibit reduced sodium dodecyl sulfate-induced colitis as compared with wild-type mice (see, for example, Non Patent Literature 3).

As a substance that inhibits RasGRP4 gene expression, siRNA (see, for example, Patent Literature 1, Patent Literature 2, and Non Patent Literature 2) is reported.

### Citation List

### Patent Literature

Patent Literature 1: JP 2019-131502 A
Patent Literature 2: JP 2020-015678 A

### Non Patent Literature

Non Patent Literature 1: Mod Rheumatol. 2014, 24(3), pp 477-480
Non Patent Literature 2: Arthritis Rheumatol. 2015, 67(2), pp 396-407
Non Patent Literature 3: J. Biol. Chem. 2012, 287(24), pp 20047-20055

### Summary of Invention

### Technical Problem

An object of the present invention is to provide a novel antisense oligonucleotide that inhibits RasGRP4 gene expression. Another object of the present invention is to provide a novel therapeutic drug for PM/DM.

### Solution to Problem

The present inventors intensively conducted studies in order to find a compound having an antisense effect on RasGRP4, and as a result, have found that a compound of the present invention has an excellent RasGRP4 gene expression inhibitory action, thereby completing the present invention.

That is, the present invention has the following features.
1. A compound comprising an antisense oligonucleotide that inhibits RasGRP4 gene expression, or a pharmacologically acceptable salt thereof.
2. A compound comprising an antisense oligonucleotide which consists of 8 to 80 nucleosides complementary to a portion in a nucleobase sequence selected from the group consisting of nucleobase sequences represented by nucleobase position numbers:
   237 to 252, 244 to 259, 501 to 516, 666 to 681, 683 to 698, 774 to 789, 889 to 904, 939 to 954, 964 to 979, 968 to 983, 969 to 984, 970 to 985, 971 to 986, 972 to 987, 974 to 989, 976 to 991, 977 to 992, 978 to 993, 979 to 994, 980 to 995, 981 to 996, 982 to 997, 1033 to 1048, 1036 to 1051, 1037 to 1052, 1038 to 1053, 1039 to 1054, 1040 to 1055, 1041 to 1056, 1114 to 1129, 1186 to 1201, 1245 to 1260, 1246 to 1261, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1395 to 1410, 1396 to 1411, 1397 to 1412, 1398 to 1413, 1399 to 1414, 1400 to 1415, 1401 to 1416, 1446 to 1461, 1523 to 1538, 1525 to 1540, 1526 to 1541, 1538 to 1553, 1590 to 1605, 1591 to 1606, 1592 to 1607, 1593 to 1608, 1668 to 1683, 1719 to 1734, 1744 to 1759, 1753 to 1768, 1756 to 1771, 1757 to 1772, 1758 to 1773, 1759 to 1774, 1760 to 1775, 1761 to 1776, 1813 to 1828, 1814 to 1829, 1815 to 1830, 1816 to 1831, 1817 to 1832, 1818 to 1833, 1819 to 1834, 1820 to 1835, 1821 to 1836, 1822 to 1837, 1823 to 1838, 1894 to 1909, 2042 to 2057, 2049 to 2064, 2050 to 2065, 2051 to 2066, and 3059 to 3074 of SEQ ID NO: 1, wherein
   the antisense oligonucleotide is at least 80% complementary to the portion in the selected nucleobase sequence of SEQ ID NO: 1, or
   a compound comprising an antisense oligonucleotide which consists of 8 to 80 nucleosides complementary to a portion in a nucleobase sequence selected from the group consisting of nucleobase sequences represented by nucleobase position numbers:
      640 to 655, 967 to 982, 1037 to 1052, 1231 to 1246, 4838 to 4853, 4845 to 4860, 5575 to 5590, 5802 to 5817, 5918 to 5933, 6395 to 6410, 6544 to 6559, 6639 to 6654, 6771 to 6786, 6921 to 6936, 6938 to 6953, 7029 to 7044, 7033 to 7048, 7206 to 7221, 8439 to 8454, 8489 to 8504, 9361 to 9376, 9439 to 9454, 9632 to 9647, 9736 to 9751, 9740 to 9755, 9741 to 9756, 9742 to 9757, 9743 to 9758, 9744 to 9759, 9746 to 9761, 9748 to 9763, 9749 to 9764, 9750 to 9765, 9751 to 9766, 9752 to 9767, 9753 to 9768, 9754 to 9769, 9805 to 9820, 9808 to 9823, 9809 to 9824, 9810 to 9825, 9811 to 9826, 9812 to 9827, 9813 to 9828, 11814 to 11829, 11886 to 11901, 11945 to 11960, 11946 to 11961, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13468 to 13483, 13469 to 13484, 13470 to 13485, 13471 to 13486, 13472 to 13487, 13473 to 13488, 13474 to 13489, 13615 to 13630, 13692 to 13707, 13694 to 13709, 13695 to 13710, 13902 to 13917, 13903 to 13918, 13904 to 13919, 13905 to 13920, 14094 to 14109, 14145 to 14160, 14170 to 14185, 14179 to 14194, 14182 to 14197, 14183 to 14198, 14184 to 14199, 14185 to 14200, 14186 to 14201, 14187 to 14202, 14868 to 14883, 15529 to 15544, 15530 to 15545, 15531 to 15546, 15532 to 15547, 15533 to 15548, 15534 to 15549, 15535 to 15550, 15536 to 15551, 15537 to 15552, 15538 to 15553, 15539 to 15554, 15705 to 15720, 15787 to 15802, 15969 to 15984, 15976 to 15991, 15977 to 15992, 15978 to 15993, 16578 to 16593, and 17775 to 17790 of SEQ ID NO: 2, wherein
      the antisense oligonucleotide is at least 80% complementary to the portion in the selected nucleobase sequence of SEQ ID NO: 2, or a pharmacologically acceptable salt thereof.
3. The compound or the pharmacologically acceptable salt thereof according to 2, comprising an antisense oligonucleotide which consists of 8 to 80 nucleosides complementary to a portion in a nucleobase sequence selected from the group consisting of nucleobase sequences represented by nucleobase position numbers:
   237 to 252, 244 to 259, 501 to 516, 666 to 681, 683 to 698, 774 to 789, 889 to 904, 939 to 954, 964 to 979, 968 to 983, 969 to 984, 970 to 985, 971 to 986, 972 to 987, 974 to 989, 976 to 991, 977 to 992, 978 to 993, 979 to 994, 980 to 995, 981 to 996, 982 to 997, 1033 to 1048, 1036 to 1051, 1037 to 1052, 1038 to 1053, 1039 to 1054, 1040 to 1055, 1041 to 1056, 1114 to 1129, 1186 to 1201, 1245 to 1260, 1246 to 1261, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1395 to 1410, 1396 to 1411, 1397 to 1412, 1398 to 1413, 1399 to 1414, 1400 to 1415, 1401 to 1416, 1446 to 1461, 1523 to 1538, 1525 to 1540, 1526 to 1541, 1538 to 1553, 1590 to 1605, 1591 to 1606, 1592 to 1607, 1593 to 1608, 1668 to 1683, 1719 to 1734, 1744 to 1759, 1753 to 1768, 1756 to 1771, 1757 to 1772, 1758 to 1773, 1759 to 1774, 1760 to 1775, 1761 to 1776, 1813 to 1828, 1814 to 1829, 1815 to 1830, 1816 to 1831, 1817 to 1832, 1818 to 1833, 1819 to 1834, 1820 to 1835, 1821 to 1836, 1822 to 1837, 1823 to 1838, 1894 to 1909, 2042 to 2057, 2049 to 2064, 2050 to 2065, 2051 to 2066, and 3059 to 3074 of SEQ ID NO: 1, wherein
   the antisense oligonucleotide is at least 80% complementary to the portion in the selected nucleobase sequence of SEQ ID NO: 2.
4. The compound or the pharmacologically acceptable salt thereof according to 2, comprising an antisense oligonucleotide which consists of 8 to 80 nucleosides complementary to a portion in a nucleobase sequence selected from the group consisting of nucleobase sequences represented by nucleobase position numbers:
   640 to 655, 967 to 982, 1037 to 1052, 1231 to 1246, 4838 to 4853, 4845 to 4860, 5575 to 5590, 5802 to 5817, 5918 to 5933, 6395 to 6410, 6544 to 6559, 6639 to 6654, 6771 to 6786, 6921 to 6936, 6938 to 6953, 7029 to 7044, 7033 to 7048, 7206 to 7221, 8439 to 8454, 8489 to 8504, 9361 to 9376, 9439 to 9454, 9632 to 9647, 9736 to 9751, 9740 to 9755, 9741 to 9756, 9742 to 9757, 9743 to 9758, 9744 to 9759, 9746 to 9761, 9748 to 9763, 9749 to 9764, 9750 to 9765, 9751 to 9766, 9752 to 9767, 9753 to 9768, 9754 to 9769, 9805 to 9820, 9808 to 9823, 9809 to 9824, 9810 to 9825, 9811 to 9826, 9812 to 9827, 9813 to 9828, 11814 to 11829, 11886 to 11901, 11945 to 11960, 11946 to 11961, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13468 to 13483, 13469 to 13484, 13470 to 13485, 13471 to 13486, 13472 to 13487, 13473 to 13488, 13474 to 13489, 13615 to 13630, 13692 to 13707, 13694 to 13709, 13695 to 13710, 13902 to 13917, 13903 to 13918, 13904 to 13919, 13905 to 13920, 14094 to 14109, 14145 to 14160, 14170 to 14185, 14179 to 14194, 14182 to 14197, 14183 to 14198, 14184 to 14199, 14185 to 14200, 14186 to 14201, 14187 to 14202, 14868 to 14883, 15529 to 15544, 15530 to 15545, 15531 to 15546, 15532 to 15547, 15533 to 15548, 15534 to 15549, 15535 to 15550, 15536 to 15551, 15537 to 15552, 15538 to 15553, 15539 to 15554, 15705 to 15720, 15787 to 15802, 15969 to 15984, 15976 to 15991, 15977 to 15992, 15978 to 15993, 16578 to 16593, and 17775 to 17790 of SEQ ID NO: 2, wherein
   the antisense oligonucleotide is at least 80% complementary to the portion in the selected nucleobase sequence of SEQ ID NO: 2.
5. The compound or the pharmacologically acceptable salt thereof according to 2, comprising an antisense oligonucleotide which consists of 8 to 80 nucleosides, and having a nucleobase sequence including at least 8 contiguous nucleobases of any one of nucleobase sequences of SEQ ID NOs: 3 to 123.
6. The compound or the pharmacologically acceptable salt thereof according to 5, comprising an antisense oligonucleotide having a nucleobase sequence including any one of nucleobase sequences of SEQ ID NOs: 3 to 123.
7. The compound or the pharmacologically acceptable salt thereof according to 5 or 6, comprising an antisense oligonucleotide having any one of nucleobase sequences of SEQ ID NOs: 3 to 123.
8. The compound or the pharmacologically acceptable salt thereof according to any one of 5 to 7, comprising an antisense oligonucleotide which consists of 8 to 80 nucleosides and having a nucleobase sequence including at least 8 contiguous nucleobases of any one nucleobase sequence selected from the group consisting of SEQ ID NOs:
   4, 5, 6, 8, 10, 11, 12, 13, 14, 15, 16, 17, 19, 20, 21, 22, 23, 24, 25, 26, 27, 28, 29, 30, 31, 32, 34, 35, 36, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 71, 72, 73, 74, 75, 76, 77, 78, 79, 80, 81, 82, 83, 84, 85, 86, 87, 95, 96, 97, 99, 100, 102, 103, 104, 105, 106, 107, 108, 109, 110, 111, 112, 113, 114, 115, 116, 117, 118, 119, 120, 121, 122, and 123.
9. The compound or the pharmacologically acceptable salt thereof according to any one of 1 to 8, wherein the antisense oligonucleotide includes a phosphorothioate bond.
10. The compound or the pharmacologically acceptable salt thereof according to any one of 1 to 9, wherein the antisense oligonucleotide includes at least one nucleoside selected from the group consisting of a 2'-modified nucleoside and a 2'-4'-bridged nucleoside.
11. The compound or the pharmacologically acceptable salt thereof according to 10, wherein the 2'-4'-bridged nucleoside is at least one selected from the group consisting of LNA, ENA, cEt, AmNA, scpBNA, and GuNA.
12. The compound or the pharmacologically acceptable salt thereof according to 11, wherein the 2'-4'-bridged nucleoside is LNA.
13. The compound or the pharmacologically acceptable salt thereof according to any one of 10 to 12, wherein the 2'-modified nucleoside is at least one selected from the group consisting of a 2'-O-MCE nucleoside, a 2'-O-MOE nucleoside, a 2'-O-NMA nucleoside, and a 2'-O-Me nucleoside.
14. The compound or the pharmacologically acceptable salt thereof according to 13, wherein the 2'-modified nucleoside is a 2'-O-MCE nucleoside.
15. The compound or the pharmacologically acceptable salt thereof according to any one of 1 to 14, wherein the antisense oligonucleotide includes 5-methylcytosine.
16. The compound or the pharmacologically acceptable salt thereof according to any one of 1 to 15, wherein
   the antisense oligonucleotide includes a gap segment, a 5' wing segment, and a 3' wing segment,
   the gap segment includes at least two nucleosides selected from 5'-modified nucleosides and deoxyribonucleosides, and a 5'-terminal and a 3'-terminal of the gap segment are independently 5'-modified nucleosides or deoxyribonucleosides,
   the nucleoside at the 3'-terminal of the 5' wing segment is a 2'-modified nucleoside or a 2'-4'-bridged nucleoside, and is linked to the 5'-terminal of the gap segment, and
   the nucleoside at the 5'-terminal of the 3' wing segment is a 2'-modified nucleoside or a 2'-4'-bridged nucleoside, and is linked to the 3'-terminal of the gap segment.
17. The compound or the pharmacologically acceptable salt thereof according to 16, wherein
   the gap segment includes 5 to 30 deoxyribonucleosides,
   the 5' wing segment and the 3' wing segment each independently include 1 to 10 sugar-modified nucleosides independently selected from the group consisting of a 2'-modified nucleoside and a 2'-4'-bridged nucleoside,
   each of the wing segments includes at least one phosphorothioate bond, and
   each of cytosines of the gap segment and each wing segment is replaced with 5-methylcytosine.
18. The compound or the pharmacologically acceptable salt thereof according to 17, wherein
   the gap segment includes 8 to 12 deoxyribonucleosides,
   the 5' wing segment and the 3' wing segment each independently include 2 to 5 sugar-modified nucleosides independently selected from the group consisting of LNA and a 2'-O-MCE nucleoside, and include at least one 2'-O-MCE nucleoside, and the gap segment includes at least one phosphorothioate bond.
19. The compound or the pharmacologically acceptable salt thereof according to any one of 16 to 18, wherein the 5' wing segment and the 3' wing segment are each independently selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, VVVL, VLLVV, and VVLLV, in which L represents a 2'-4'-bridged nucleoside, and V represents a 2'-modified nucleoside.
20. The compound or the pharmacologically acceptable salt thereof according to any one of 1 to 19, wherein the antisense oligonucleotide consists of 15 to 25 nucleosides.
21. The compound or the pharmacologically acceptable salt thereof according to any one of 1 to 20, wherein the compound comprising the antisense oligonucleotide includes a functional molecule.
22. The compound or the pharmacologically acceptable salt thereof according to 21, wherein the functional molecule is a group derived from a molecule having a function of delivering an antisense oligonucleotide to a target site.
23. The compound or the pharmacologically acceptable salt thereof according to 21 or 22, wherein the functional molecule is a lipid selected from the group consisting of cholesterol, a vitamin, a steroid, C5-30 saturated fatty acids, and C5-30 unsaturated fatty acids.
24. The compound or the pharmacologically acceptable salt thereof according to any one of 1 to 20, wherein the compound consists of the antisense oligonucleotide.
25. The salt according to any one of 1 to 21, wherein the pharmacologically acceptable salt is a sodium salt.
26. A medicinal drug comprising, as an active ingredient, the compound or the pharmacologically acceptable salt according to any one of 1 to 25.
27. A drug for treating, preventing, and/or ameliorating a disease or a condition for which a RasGRP4 gene expression inhibitory action is effective, comprising, as an active ingredient, the compound or the pharmacologically acceptable salt according to any one of 1 to 25.
28. A RasGRP4 gene expression inhibitor comprising, as an active ingredient, the compound or the pharmacologically acceptable salt according to any one of 1 to 25.

### Description of Embodiments

It is understood that both the above summary and the following detailed description are exemplary and explanatory only and do not limit the present invention as claimed. In this description, the use of a singular form shall include a plural form unless otherwise specifically stated.

In this description, an antisense oligonucleotide or an antisense oligonucleotide compound is used interchangeably, and these may be described as "ASO".

Hereinafter, the present invention will be described in more detail.

The following terms have the following meanings unless otherwise specified.

The expression "may be substituted" means having no substituent or having a substituent.

The "nucleoside" is a term well known to a person skilled in the art, and is generally understood as a molecule to which a sugar and a nucleobase are bonded and which can be a unit constituting a nucleic acid. Herein, the nucleoside is a broader concept, and includes a ribonucleoside, a deoxyribonucleoside, and a sugar-modified nucleoside described later. The nucleobase may be modified.

The "ribonucleoside" means a molecule having a nucleobase on a carbon atom at the 1-position of ribose. The ribonucleoside in the present invention may be a naturally occurring ribonucleoside or a ribonucleoside in which a nucleobase moiety of a naturally occurring ribonucleoside is modified. A plurality of modifications may be made to one ribonucleoside in combination. The modified ribonucleoside is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

The "deoxyribonucleoside" means a molecule having a nucleobase on a carbon atom at the 1-position of 2-deoxyribose. The deoxyribonucleoside in the present invention may be a naturally occurring deoxyribonucleoside or a deoxyribonucleoside in which a nucleobase moiety of a naturally occurring deoxyribonucleoside is modified. A plurality of modifications may be made to one deoxyribonucleoside in combination. The modified deoxyribonucleoside is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A and the like.

The "modified sugar" means
(Z1) a molecule in which ribose or 2-deoxyribose is partially substituted with one or more substituents,
(Z2) a pentasaccharide or a hexasaccharide different from ribose and 2-deoxyribose (for example, hexitol, threose, or the like),
(Z3) a molecule in which the entire ribose or 2-deoxyribose or a tetrahydrofuran ring thereof is replaced with a 5- to 7-membered saturated or unsaturated ring (for example, cyclohexane, cyclohexene, morpholine, or the like) or a partial structure in which a 5- to 7-membered ring can be formed by a hydrogen bond (for example, a peptide structure), or
(Z4) a molecule in which ribose or 2-deoxyribose is replaced with an alkylene glycol having 2 to 6 carbon atoms (for example, ethylene glycol, propylene glycol, or the like).

The modified sugar includes a "2-modified sugar", a "2-4-bridged sugar", and a "5-modified sugar" described later.

Examples of the modified sugar and the sugar-modified nucleoside described later include sugars, sugar-modified nucleosides, and the like disclosed as being preferably used in an antisense method in JP H10-304889 A, WO 2005/021570 A, JP H10-195098 A, JP 2002-521310 A, WO 2007/143315 A, WO 2008/043753 A, WO 2008/029619 A, WO 2008/049085 A, WO 2017/142054 A, and the like (hereinafter, these literatures are referred to as "literatures related to an antisense method"). The modified sugar and the sugar-modified nucleoside are also disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

Examples of a modified sugar that is partially substituted with one substituent include ribose or 2-deoxyribose substituted at any position of a sugar moiety with one or more (preferably one or two) of the following substituents (i) or (ii).
(i) a C₁₋₆ alkyl group Here, when substitution with two or more C₁₋₆ alkyl groups is contained, two or more C₁₋₆ alkyl groups may form a 3- to 6-membered ring together.
(ii) a C₁₋₆ alkyl group substituted with at least one selected from the group consisting of a halogen atom, a C₁₋₆ alkoxy group, a halo C₁₋₆ alkoxy group, a mono- or di-C₁₋₆ alkylamino group, a 5- to 10-membered heterocyclic group, a carboxy group, a carbamoyl group, and a N-substituted carbamoyl group.

Here, examples of the N-substituted carbamoyl group include a N-methyl-carbamoyl group and a N-ethyl-carbamoyl group, and the methyl group and the ethyl group of the N-methyl-carbamoyl group and the N-ethyl-carbamoyl group may be substituted with a 5- to 10-membered heterocyclic group or a mono- or di-C₁₋₆ alkylamino group. Specific examples of the N-substituted carbamoyl group include a N-methylcarbamoyl group, a N-ethylcarbamoyl group, a N-dimethylaminoethyl-carbamoyl group, a N-(morpholinoethyl)carbamoyl group, a N-(2-pyridylethyl)carbamoyl group, a N-((benzimidazol-1-yl)ethyl)carbamoyl group, and the like.

The "sugar-modified nucleoside" means a molecule having the "modified sugar" instead of a sugar moiety of a deoxyribonucleoside or a ribonucleoside. For example, the sugar-modified nucleoside includes a "2'-modified nucleoside" and a "2'-4'-bridged nucleoside" described later, and also includes a "5'-modified nucleoside". When the modified sugar is (Z3) as defined above, the sugar-modified nucleoside also includes a molecule in which a modified sugar and a nucleobase are bonded to each other via a methylene chain or the like.

The "2-modified sugar" means a non-bridged sugar in which an oxygen atom or a carbon atom at the 2-position of ribose is modified, and includes "2-O-Me", "2-O-MOE", "2-O-MCE", "2-O-NMA", "2-O-AP", "2-F", "2-DMAECE", "2-MorECE", "2-PyECE", and "2-BimECE". The 4-position and the 5-position of the "2-modified sugar" are preferably not modified.

The "2'-modified nucleoside" means a molecule having a nucleobase at the 1-position of the 2-modified sugar, and examples thereof include a "2'-O-Me nucleoside", a "2'-O-MOE nucleoside", a "2'-O-MCE nucleoside", a "2'-O-NMA nucleoside", a "2'-O-AP nucleoside", a "2'-F nucleoside", a "2'-DMAECE nucleoside", a "2'-MorECE nucleoside", a "2'-PyECE nucleoside", and a "2'-BimECE nucleoside".

The "2-O-Me" (also referred to as 2-O-methyl) means a sugar in which a hydroxy group at the 2-position of ribose is replaced with a methoxy group.

The "2'-O-Me nucleoside" (also referred to as 2'-O-methyl nucleoside) means a molecule having a nucleobase at the 1-position of "2-O-Me".

The "2-O-MOE" (also referred to as 2-O-methoxyethyl) means a sugar in which a hydroxy group at the 2-position of ribose is replaced with a 2-methoxyethyloxy group.

The "2'-O-MOE nucleoside" (also referred to as 2'-O-methoxyethyl nucleoside) means a molecule having a nucleobase at the 1-position of "2-O-MOE".

The "2-O-MCE" (also referred to as 2-O-methylcarbamoylethyl) means a sugar in which a hydroxy group at the 2-position of ribose is replaced with a methylcarbamoylethyloxy group.

The "2'-O-MCE nucleoside" (also referred to as 2'-O-methylcarbamoylethyl nucleoside) means a molecule having a nucleobase at the 1-position of "2-O-MCE".

The "2-O-NMA" means a sugar in which a hydroxy group at the 2-position of ribose is replaced with a [2-(methylamino)-2-oxoethyl]oxy group.

The "2'-O-NMA nucleoside" means a molecule having a nucleobase at the 1-position of "2-O-NMA".

The "2-O-AP" means a sugar in which a hydroxy group at the 2-position of ribose is replaced with a 3-aminopropyloxy group.

The "2'-O-AP nucleoside" means a molecule having a nucleobase at the 1-position of "2-O-AP".

The "2-F" means a sugar in which a hydroxy group at the 2-position of ribose is replaced with a fluorine atom.

The "2'-F nucleoside" means a molecule having a nucleobase at the 1-position of "2-F".

The "2-DMAECE", "2-MorECE", "2-PyECE", and "2-BimECE" are sugars in which a hydroxy group at the 2-position of ribose is replaced with a structure represented by the following DMAECE, MorECE, PyECE, and BimECE, respectively. In the following structure, the wavy line indicates a bonding position to a carbon atom to which a hydroxy group at the 2-position of ribose is bonded.

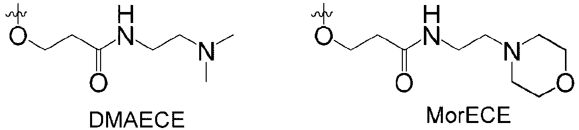

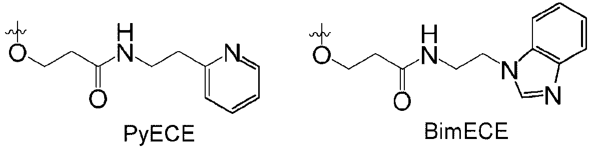

The "2'-DMAECE nucleoside", "2'-MorECE nucleoside", "2'-PyECE nucleoside", and "2'-BimECE nucleoside" mean molecules having a nucleobase at the 1-position of "2-DMAECE", "2-MorECE", "2-PyECE", and "2-BimECE", respectively.

The "2-4-bridged sugar" means a sugar substituted with a bridging unit at two positions of the 2-position and the 4-position of ribose. Examples of the bridging unit include a C₂₋₆ alkylene group (the alkylene group has no substituent or has one or more substituents selected from the group consisting of a halogen atom, an oxo group, and a thioxo group, and one or two methylene groups of the alkylene group are not replaced or independently replaced with a group selected from the group consisting of -O-, -NR¹-(R¹ represents a hydrogen atom, a C₁₋₆ alkyl group, or a halo C₁₋₆ alkyl group), and -S-). The 5-position of the "2-4-bridged sugar" is preferably not modified.

The "2'-4'-bridged nucleoside" (2',4'-BNA) means a molecule having a nucleobase at the 1-position of the 2-4-bridged sugar. Examples thereof include β-D-methyleneoxy (4'-CH₂-O-2') BNA or α-L-methyleneoxy (4'-CH₂-O-2') BNA also referred to as LNA (locked nucleic acid (registered trademark)), ethyleneoxy (4'-(CH₂)₂-O-2') BNA also referred to as ENA, β-D-thio (4'-CH₂-S-2') BNA, aminooxy (4'-CH₂-ON(R¹¹)-2') BNA (R¹¹ is a hydrogen atom or methyl), oxyamino (4'-CH₂-N(R¹²)-O-2') BNA (R¹² is a hydrogen atom or methyl) also referred to as 2',4'-BNA^{NC}, 2',4'-BNA^{COC}, 3'-amino-2',4'-BNA, 5'-methyl BNA, (4'-CH(CH₃)-O-2') BNA also referred to as cEt, (4'-CH(CH₂OCH₃)-O-2') BNA also referred to as cMOE-BNA, amide-based BNA (4'-C(=O)-N(R¹³)-2') BNA (R¹³ is a hydrogen atom or methyl) also referred to as AmNA, (4'-C (spiro-cyclopropyl)-O-2') BNA also referred to as scpBNA, (4'-CH₂-N(R¹⁴)-2') BNA (R¹⁴ is C(=NHR¹⁵⁺)NHR¹⁶, R¹⁵ and R¹⁶ are each independently a hydrogen atom, methyl, ethyl, isopropyl, or t-butyl) also referred to as GuNA, (4'-CH₂-N(R¹⁷)-2') BNA(R¹⁷ is a hydrogen atom or methyl) also referred to as amino-LNA, other BNAs known to a person skilled in the art, and the like.

Specific examples of the 2'-4'-bridged nucleoside are represented by, for example, the following structural formulae. In the formulae, Base is a nucleobase.

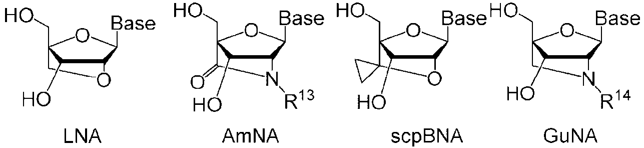

AmNA[Me] is AmNA in which R¹³ is methyl.

GuNA[tBu] is GuNA in which R¹⁵ is t-butyl and R¹⁶ is H.

The "5-modified sugar" means a non-bridged sugar in which the 5-position of 2-deoxyribose is modified, and includes "5-CP", "5-methyl", and "5-aminopropyl". The 2-position and the 4-position of the "5-modified sugar" are preferably not modified.

The "5'-modified nucleoside" means a molecule having a nucleobase at the 1-position of the "5-modified sugar", and includes, for example, a "5'-CP nucleoside", a "5'-methyl nucleoside", and a "5'-aminopropyl nucleoside".

The "5-CP" is a sugar in which the 5-position of 2-deoxyribose is substituted with two methyl groups, and the two methyl groups form cyclopropane together.

The "5'-CP nucleoside" is a molecule represented by the following structural formula. In the formulae, Base is a nucleobase.

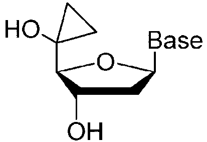

The "5-methyl" is a sugar in which the 5-position of 2-deoxyribose is substituted with a methyl group.

The "5-aminopropyl" is a sugar in which the 5-position of 2-deoxyribose is substituted with a 3-aminopropyl group.

The "5'-methyl nucleoside" and "5'-aminopropyl nucleoside" mean molecules having a nucleobase at the 1-position of "5-methyl" and "5-aminopropyl", respectively.

In the "deoxyribonucleoside", "ribonucleoside", "2'-modified nucleoside", "2'-4'-bridged nucleoside", and the like, examples of a bond between a carbon atom at the 1'-position and a nucleobase include an α-glycosidic bond and a β-glycosidic bond, but the bond is usually a β-glycosidic bond. Therefore, β-D-methyleneoxy BNA is usually used as the LNA.

"n-" means normal, "s-" means secondary, and "t-" means tertiary.

The "halogen atom" means a fluorine atom, a chlorine atom, a bromine atom, or an iodine atom.

The "C₁₋₆ alkyl group" means a linear or branched saturated hydrocarbon group having 1 to 6 carbon atoms, and examples thereof include a methyl group, an ethyl group, a n-propyl group, an isopropyl group, a n-butyl group, an isobutyl group, a s-butyl group, a t-butyl group, a n-pentyl group, an isopentyl group, a neopentyl group, a n-hexyl group, an isohexyl group, and the like.

The "halo C₁₋₆ alkyl group" means a group in which a hydrogen atom at any position of the "C₁₋₆ alkyl group" is substituted with one or more of the "halogen atoms".

The "C₁₋₆ alkylene group" means a divalent group obtained by removing one hydrogen atom at any position from a linear or branched saturated hydrocarbon group having 1 to 6 carbon atoms, and examples thereof include a methylene group, an ethylene (ethanediyl) group, a propane-1,3-diyl (trimethylene) group, a propane-2,2-diyl group, a 2,2-dimethyl-propane-1,3-diyl group, a hexane-1,6-diyl (hexamethylene) group, a 3-methylbutane-1,2-diyl group, and the like.

The "C₂₋₆ alkylene group" means a linear or branched divalent group having 2 to 6 carbon atoms in the "C₁₋₆ alkylene group", and examples thereof are similar to those of the "C₁₋₆ alkylene group" except for a methylene group.

The "C₁₋₆ alkoxy group" means a group in which the "C₁₋₆ alkyl group" is bonded to an oxy group.

The "halo C₁₋₆ alkoxy group" means a group in which a hydrogen atom at any position of the "C₁₋₆ alkoxy group" is substituted with one or more of the "halogen atoms".

The "mono- or di-C₁₋₆ alkylamino group" means a group in which one hydrogen atom of an amino group is replaced with one "C₁₋₆ alkyl group" or a group in which two hydrogen atoms of an amino group are replaced with two identical or different "C₁₋₆ alkyl groups", and examples thereof include methylamino, ethylamino, propylamino, isopropylamino, butylamino, dimethylamino, diethylamino, dipropylamino, dibutylamino, a N-ethyl-N-methylamino group, and the like.

The "3- to 6-membered ring" means a monocyclic saturated or unsaturated hydrocarbon ring having 3 to 6 carbon atoms, and examples thereof include cyclopropane, cyclobutane, cyclohexane, and the like.

The "5- to 10-membered heterocyclic group" means a 5- to 10-membered monocyclic or fused polycyclic aromatic or non-aromatic heterocyclic group containing 1 to 4 heteroatoms selected from a nitrogen atom, a sulfur atom, and an oxygen atom as ring-constituting atoms in addition to a carbon atom.

Preferable examples of the "5- to 10-membered heterocyclic group" include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, triazolyl, tetrazolyl, triazinyl, benzothiophenyl, benzofuranyl, benzimidazolyl, benzoxazolyl, benzisoxazolyl, benzothiazolyl, benzoisothiazolyl, benzotriazolyl, imidazolopyridinyl, thienopyridinyl, pyrrolopyridinyl, pyrazolopyridinyl, oxazolopyridinyl, thiazolopyridinyl, imidazopyrazinyl, imidazolopyrimidinyl, aziridinyl, oxiranyl, azetidinyl, oxetanyl, thietanyl, tetrahydrothienyl, tetrahydrofuranyl, pyrrolinyl, pyrrolidinyl, oxopyrrolidinyl, imidazolinyl, oxoimidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, tetrahydropyridazinyl, dihydropyranyl, tetrahydropyranyl, tetrahydrothiopyranyl, morpholinyl, thiomorpholinyl, and the like.

The "oxo group" refers to a group (=O) obtained by substitution with an oxygen atom via a double bond. When an oxo group is substituted on a carbon atom, the oxo group forms a carbonyl group together with the carbon atom. The "thioxo group" refers to a group (=S) obtained by substitution with a sulfur atom via a double bond. When a thioxo group is substituted on a carbon atom, the thioxo group forms a thiocarbonyl group together with the carbon atom.

The "nucleobase" is a purine base or a pyrimidine base, and may be a naturally occurring nucleobase or one obtained by modifying a naturally occurring nucleobase. Examples of the naturally occurring nucleobase herein include adenine (A), guanine (G), thymine (T), cytosine (C), and uracil (U). The "nucleobase" includes the naturally occurring nucleobase and the "modified nucleobase" described later.

Examples of modification of a nucleobase in the "modified nucleobase" herein include halogenation, methylation, ethylation, n-propylation, isopropylation, cyclopropylation, n-butylation, isobutylation, s-butylation, t-butylation, cyclobutylation, hydroxylation, amination, thiation, demethylation, and the like. More specific examples thereof include: 5-methylation, 5-fluorination, 5-bromination, 5-iodination, and N4-methylation of cytosine; 2-thiation, 5-demethylation, 5-fluorination, 5-bromination, and 5-iodination of thymine; 2-thiation, 5-fluorination, 5-bromination, and 5-iodination of uracil; N6-methylation and 8-bromination of adenine; N2-methylation and 8-bromination of guanine; and the like. Examples of modification of a nucleobase moiety in a nucleoside are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

The nucleobase in a nucleoside is more preferably at least one selected from the group consisting of adenine, guanine, thymine, cytosine, uracil, and 5-methylcytosine.

The "5-methylcytosine" means a cytosine having a methyl group at the 5-position.

The "nucleobase sequence" means a sequence from a 5' side to a 3' side of nucleobases of each nucleoside contained in an oligonucleotide.

The "contiguous nucleobases" means a sequence from a 5' side to a 3' side of nucleobases in a contiguous part of the "nucleobase sequence".

The "internucleoside bond" means a group or a bond that forms a covalent bond between adjacent nucleosides in an oligonucleotide. The "internucleoside bond" includes a phosphodiester bond and a "modified internucleoside bond" described below.

The "modified internucleoside bond" means a modified phosphodiester bond, and examples thereof include a phosphorothioate bond, a methyl phosphonate bond (including a chiral-methyl phosphonate bond), a methyl thiophosphonate bond, a phosphorodithioate bond, a phosphoramidate bond, a phosphorodiamidate bond, a phosphoramidothioate bond, a boranophosphate bond, and the like. Examples of modification of a phosphodiester bond are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, and the like, and can be used for a modified phosphodiester bond.

The "oligonucleotide" means a molecule having a structure in which two or more identical or different "nucleosides" are linked by the independently selected "internucleoside bond" (for example, a phosphodiester bond or a modified phosphodiester bond).

The "DNA" means a polynucleotide or an oligonucleotide in which the two or more identical or different "deoxyribonucleosides" are linked by the independently selected "internucleoside bond".

The "RNA" means a polynucleotide or an oligonucleotide in which the two or more identical or different "ribonucleosides" are linked by the independently selected "internucleoside bond".

The "antisense effect" means that the target RNA selected corresponding to a target gene hybridizes with, for example, an oligonucleotide having a sequence complementary to a partial sequence of the target RNA to control a function of the target RNA. For example, when the target RNA is mRNA, the antisense effect means inhibition of translation of the target RNA by hybridization, a splicing function converting effect such as exon skipping, degradation of the target RNA by recognition of a portion that has hybridized, and the like.

In the present invention, the target RNA is "RasGRP4 mRNA" or "RasGRP4 pre-mRNA".

The "antisense oligonucleotide" (ASO) is an oligonucleotide having the antisense effect. Examples thereof include DNA, a gapmer, a mixmer, and the like, but are not limited thereto, and may be RNA, an oligonucleotide designed to usually have the antisense effect, or the like. The ASO of the present invention is a single-stranded antisense oligonucleotide.

The "hybridize" means an action of forming a double strand between oligonucleotides including sequences complementary to each other or portions thereof, and a phenomenon that oligonucleotides including sequences complementary to each other or portions thereof form a double strand therebetween.

The "complementary" means that two nucleobases can form a Watson-Crick base pair (natural base pair) or a non-Watson-Crick base pair (Hoogsteen base pair, and the like) via a hydrogen bond. Two oligonucleotides or portions thereof can "hybridize" with each other when their sequences are complementary to each other. In order for two oligonucleotides or portions thereof to hybridize with each other, they need not be completely complementary to each other, but the complementarity between two oligonucleotides or portions thereof to hybridize with each other is preferably 70% or more, more preferably 80% or more, and still more preferably 90% or more (for example, 95%, 96%, 97%, 98%, or 99% or more). The complementarity between sequences is determined by using a computer program that automatically identifies a partial sequence of an oligonucleotide. For example, GENETYX (manufactured by GENETYX Corporation) is one such software.

The "gapmer" means an oligonucleotide including a "gap segment", a "5' wing segment", and a "3' wing segment" described later.

The "gap segment" is a region including "at least four contiguous nucleosides recognized by RNaseH", and is not particularly limited as long as it includes four or more contiguous nucleosides and is recognized by RNaseH. However, the contiguous nucleosides are preferably independently selected from a deoxyribonucleoside and a sugar-modified nucleoside. In the present invention, the nucleosides at the 5'-terminal and the 3'-terminal of the gap segment are independently preferably 5'-modified nucleosides or deoxyribonucleosides, more preferably deoxyribonucleosides or 5'-CP nucleosides, and particularly preferably deoxyribonucleosides.

The "5' wing segment" is a region linked to a 5' side of the gap segment and including "at least one nucleoside" without including the "at least four contiguous nucleosides recognized by RNaseH". Here, a sugar moiety of a nucleoside at a 3'-terminal of the 5' wing segment is different from a sugar moiety of a nucleoside at a 5'-terminal of the gap segment. Due to the difference in sugar moiety, a boundary between the 5' wing segment and the gap segment is confirmed. In the present invention, preferably as one aspect, the difference between the sugar moieties is determined by the presence or absence of modification at the 2-position of the corresponding sugar. As defined above, the "2-modified sugar" is a non-bridged sugar in which an oxygen atom or a carbon atom at the 2-position of ribose is modified, and the "2-4-bridged sugar" is a sugar substituted with a bridging unit at two positions of the 2-position and the 4-position of ribose in the above definition, and thus both are modified at the 2-position. (For example, the nucleoside at the 5'-terminal of the gap segment is a deoxyribonucleoside, and the nucleoside at the 3'-terminal of the 5' wing segment is a sugar-modified nucleoside. This sugar-modified nucleoside is a 2'-4'-bridged nucleoside or a 2-'modified nucleoside. Also, for example, the nucleoside at the 5'-terminal of the gap segment is a 5'-CP nucleoside and the nucleoside at the 3'-terminal of the 5' wing segment is a 2'-4'-bridged nucleoside or a 2'-modified nucleoside.) The nucleoside at the 3'-terminal of the 5' wing segment is generally a sugar-modified nucleoside, preferably a 2'-4'-bridged nucleoside or a 2'-modified nucleoside, more preferably a 2'-4'-bridged nucleoside. The 5' wing segment is not particularly limited as long as it satisfies the above definition, but the at least one nucleoside is preferably independently selected from a deoxyribonucleoside and a sugar-modified nucleoside, and includes at least one sugar-modified nucleoside, preferably includes at least one 2'-4'-bridged nucleoside or 2'-modified nucleoside, and particularly preferably includes a 2'-4'-bridged nucleoside.

The "3' wing segment" is a region linked to a 3' side of the gap segment and including "at least one nucleoside" without including the "at least four contiguous nucleosides recognized by RNaseH". Here, a sugar moiety of a nucleoside at a 5'-terminal of the 3' wing segment is different from a sugar moiety of a nucleoside at a 3'-terminal of the gap segment. Due to the difference in sugar moiety, a boundary between the 3' wing segment and the gap segment is confirmed. In the present invention, preferably as one aspect, the difference between the sugar moieties is determined by the presence or absence of modification at the 2-position of the corresponding sugar. (For example, the nucleoside at the 3'-terminal of the gap segment is a deoxyribonucleoside, and the nucleoside at the 5'-terminal of the 3' wing segment is a sugar-modified nucleoside. This sugar-modified nucleoside is a 2'-4'-bridged nucleoside or a 2-'modified nucleoside. Also, for example, the nucleoside at the 3'-terminal of the gap segment is a 5'-CP nucleoside and the nucleoside at the 5'-terminal of the 3' wing segment is a 2'-4'-bridged nucleoside or a 2'-modified nucleoside.) The nucleoside at the 5'-terminal of the 3' wing segment is generally a sugar-modified nucleoside, preferably a 2'-4'-bridged nucleoside or a 2'-modified nucleoside, more preferably a 2'-4'-bridged nucleoside. The 3' wing segment is not particularly limited as long as it satisfies the above definition, but the at least one nucleoside is preferably independently selected from a deoxyribonucleoside and a sugar-modified nucleoside, and includes at least one sugar-modified nucleoside, preferably includes at least one 2'-4'-bridged nucleoside or 2'-modified nucleoside, and particularly preferably includes a 2'-4'-bridged nucleoside.

As one typical example, a portion in which 2'-modified nucleosides or 2'-4'-bridged nucleosides are contiguous from the 5'-terminal is the 5' wing segment, and the boundary where the nucleoside at the 3'-terminal of the 5' wing segment connects to another nucleoside (a deoxyribonucleoside, a 5'-modified nucleoside, a ribonucleoside, or the like) other than the 2'-modified nucleosides or the 2'-4'-bridged nucleosides is the boundary between the 5' wing segment and the gap segment.

As one typical example, a portion in which 2'-modified nucleosides or 2'-4'-bridged nucleosides are contiguous from the 3'-terminal is the 3' wing segment, and the boundary where the nucleoside at the 5'-terminal of the 3' wing segment connects to another nucleoside (a deoxyribonucleoside, a 5'-modified nucleoside, a ribonucleoside, or the like) other than the 2'-modified nucleosides or the 2'-4'-bridged nucleosides is the boundary between the 3' wing segment and the gap segment.

The "RNaseH" is generally known as a ribonuclease that recognizes a double strand in which DNA and RNA in a living body hybridize with each other, and cleaves the RNA to generate single-stranded DNA. RNaseH can recognize not only a double strand in which DNA and RNA hybridize with each other but also a double strand in which at least one of a nucleobase moiety, a phosphodiester bond moiety, and a sugar moiety of at least one of DNA and RNA is modified. For example, RNaseH can also recognize a double strand in which DNA modified with a phosphorothioate bond and RNA hybridize with each other.

Thus, DNA can be recognized by RNaseH when DNA hybridizes with RNA. In addition, RNA can be cleaved by RNaseH when RNA hybridizes with DNA. The same applies to a case where at least one of a nucleobase moiety, a phosphodiester bond moiety, and a sugar moiety is modified in at least one of DNA and RNA. For example, a typical example is an oligonucleotide in which a phosphodiester bond moiety of DNA is modified with phosphorothioate, and the like.

Examples of modification of DNA and/or RNA that can be recognized by RNaseH are described, for example, in Nucleic Acids Research, 2014, 42, pp 5378-5389, Bioorganic & Medicinal Chemistry Letters, 2008, 18, pp 2296-2300, Molecular BioSystems, 2009, 5, pp 838-843, Nucleic Acid Therapeutics, 2015, 25, pp 266-274, The Journal of Biological Chemistry, 2004, 279, pp 36317-36326, and the like.

RNaseH used in the present invention is preferably mammalian RNaseH, more preferably human RNaseH, and particularly preferably human RNaseH1.

At least four contiguous nucleosides recognized by RNaseH are not particularly limited as long as they include four or more contiguous nucleosides and are recognized by RNaseH, but examples thereof include "at least four contiguous deoxyribonucleosides", and the like. The number of nucleosides constituting "at least four contiguous nucleosides recognized by RNaseH" is, for example, 5 to 20, preferably 5 to 15, more preferably 8 to 14, still more preferably 9 to 13, and particularly preferably 10.

A person skilled in the art can determine whether or not certain at least four contiguous nucleosides are "at least four contiguous nucleosides recognized by RNaseH" by the structure of a sugar moiety of the contiguous nucleosides.

The "RasGRP4" (RAS Guanyl Releasing Protein 4) means any protein of RasGRP4. The "RasGRP4 mRNA" means mRNA encoding RasGRP4 protein. The "RasGRP4 pre-mRNA" means pre-mRNA encoding RasGRP4 protein. The "RasGRP4 nucleic acid" means any nucleic acid encoding RasGRP4, and includes, for example, "RasGRP4 mRNA" and "RasGRP4 pre-mRNA".

The "RasGRP4 mRNA" is represented, for example, by SEQ ID NO: 1. The "RasGRP4 pre-mRNA" is represented, for example, by SEQ ID NO: 2. The "RasGRP4 mRNA" and the "RasGRP4 pre-mRNA" each have nucleosides linked to each other by a phosphodiester bond, and usually thymine is replaced with uracil. The "RasGRP4 mRNA" and the "RasGRP4 pre-mRNA" do not have a modified sugar, a modified nucleobase, or a modified internucleoside bond in addition to the nucleosides.

The "gene expression" means that coding information of a gene is converted into a structure in a cell or a function thereof. Such a structure is not limited, but examples thereof include products of transcription and translation (mRNA, pre-mRNA, protein, and the like).

The ASO of the present invention targets mRNA and/or pre-mRNA of RasGRP4. The ASO of the present invention does not need to hybridize with the entire mRNA and/or pre-mRNA of RasGRP4, and usually hybridizes with at least a part thereof. For example, a part of mRNA and/or pre-mRNA of RasGRP4 hybridizes with an oligonucleotide having a sequence complementary to a partial sequence of mRNA and/or pre-mRNA of RasGRP4 (a gapmer or an oligonucleotide designed to usually have an antisense effect, or the like) to control RasGRP4 gene expression. In addition, the entire ASO does not need to hybridize, and a part of the ASO does not need to hybridize.

The complementarity between a nucleobase sequence of the ASO of the present invention and a partial sequence of mRNA and/or pre-mRNA of RasGRP4 is preferably 80% or more, more preferably 85% or more, or 90% or more (for example, 95%, 96%, 97%, 98%, or 99% or more). In order for the ASO of the present invention to hybridize with at least a part of a partial sequence of mRNA and/or pre-mRNA of RasGRP4, the sequences do not need to be completely complementary to each other, but are more preferably completely complementary to each other.

The ASO of the present invention includes a gap segment, a 5' wing segment, and a 3' wing segment.

The gap segment comprises at least five nucleosides independently selected from the group consisting of a deoxyribonucleoside, a ribonucleoside, and a sugar-modified nucleoside, and includes at least two deoxyribonucleosides selected from a deoxyribonucleoside and a 5'-modified nucleoside, and the 3'-terminals and 5'-terminals thereof are each independently a deoxyribonucleoside or a 5'-modified nucleoside. The gap segment preferably includes at least two deoxyribonucleosides. The gap segment preferably includes "at least four contiguous deoxyribonucleosides".

The number of nucleosides included in the gap segment is preferably 5 to 20, more preferably 5 to 15, still more preferably 8 to 15, even still more preferably 9 to 13, and particularly preferably 10.

The nucleoside included in the gap segment is preferably independently selected from the group consisting of a deoxyribonucleoside and a sugar-modified nucleoside, more preferably selected from a deoxyribonucleoside and a 5'-modified nucleoside, and still more preferably selected from a deoxyribonucleoside.

The 3'-terminal and the 5'-terminal of the gap segment are independently preferably deoxyribonucleosides or 5'-modified nucleosides. In one aspect, particularly preferably, the 3'-terminal and the 5'-terminal of the gap segment are deoxyribonucleosides. In another aspect, particularly preferably, the 5'-terminal of the gap segment is a 5'-modified nucleoside, and the 3'-terminal of the gap segment is a deoxyribonucleoside.

The sugar-modified nucleoside included in the gap segment may be any of a 2'-modified nucleoside, a 2'-4'-bridged nucleoside, and a 5'-modified nucleoside. The 2'-modified nucleoside included in the gap segment is more preferably selected from a 2'-O-Me nucleoside, a 2'-O-MOE nucleoside, a 2'-O-AP nucleoside, a 2'-F nucleoside, a 2'-O-NMA nucleoside, and a 2'-O-MCE nucleoside, and is still more preferably a 2'-O-Me nucleoside.

The sugar-modified nucleoside included in the gap segment is preferably a 5'-modified nucleoside, and more preferably a 5'-CP nucleoside.

As the positions of the 2'-modified nucleoside and the 2'-4'-bridged nucleoside in the gap segment, the 3'-terminal and the 5'-terminal of the gap segment are excluded, and there are no other limitations.

The position of the 5'-modified nucleoside in the gap segment is not particularly limited, but the 5'-modified nucleoside is preferably included on a 5' side in the gap segment.

When the gap segment includes one or more sugar-modified nucleosides, the number of sugar-modified nucleosides included in the gap segment is preferably 1 to 3.

When the gap segment includes one or more 5'-modified nucleosides, the number of 5'-modified nucleosides included in the gap segment is particularly preferably 1 to 3.

An internucleoside bond (an internucleoside bond between the wing segment and the gap segment is not included, the same applies hereinafter) included in the gap segment is preferably at least one independently selected from a phosphodiester bond and a modified phosphodiester bond, and more preferably at least one independently selected from a phosphodiester bond and a phosphorothioate bond. The internucleoside bond included in the gap segment preferably includes at least one phosphorothioate bond, more preferably 50% or more of the internucleoside bonds are phosphorothioate bonds, still more preferably 75% or more of the internucleoside bonds are phosphorothioate bonds, even still more preferably 80% or more of the internucleoside bonds are phosphorothioate bonds, even yet still more preferably 90% or more of the internucleoside bonds are phosphorothioate bonds, and particularly preferably all of the internucleoside bonds are phosphorothioate bonds.

The internucleoside bond on a 5' side of the 5'-modified nucleoside is preferably a phosphodiester bond or a phosphorothioate bond, and more preferably a phosphodiester bond. When a 3' side of a 5'-modified nucleoside is bonded to a 5'-modified nucleoside, the internucleoside bond is preferably a phosphodiester bond. When a 3' side of a 5'-modified nucleoside is bonded to a nucleoside other than the 5'-modified nucleoside, the internucleoside bond is preferably a phosphodiester bond or a phosphorothioate bond, more preferably a phosphorothioate bond.

The 5' wing segment comprises at least one nucleoside independently selected from the group consisting of a deoxyribonucleoside, a ribonucleoside, and a sugar-modified nucleoside, provided that a 3'-terminal of the 5' wing segment bonded to the gap segment is a sugar-modified nucleoside, and does not include "at least four contiguous nucleosides recognized by RNaseH." The 3'-terminal bonded to the gap segment is preferably a 2'-modified nucleoside or a 2'-4'-bridged nucleoside.

The 3' wing segment comprises at least one nucleoside independently selected from the group consisting of a deoxyribonucleoside, a ribonucleoside, and a sugar-modified nucleoside, provided that a 5'-terminal of the 3' wing segment bonded to the gap segment is a sugar-modified nucleoside, and does not include "at least four contiguous nucleosides recognized by RNaseH." The 5'-terminal bonded to the gap segment is preferably a 2'-modified nucleoside or a 2'-4'-bridged nucleoside.

At least one of the 5' wing segment and the 3' wing segment preferably includes at least one 2'-4'-bridged nucleoside.

The 5' wing segment and the 3' wing segment each preferably do not include "at least four contiguous deoxyribonucleosides".

Hereinafter, properties common to the 5' wing segment and the 3' wing segment will be described with reference simply to a wing segment.

The number of nucleosides included in each wing segment is preferably 1 to 10, more preferably 1 to 7, still more preferably 2 to 6, even still more preferably 2 to 5, even yet still more preferably 3 to 4, and particularly preferably 3.

Each wing segment preferably includes a nucleoside having increased affinity for a partial sequence of RasGRP4 mRNA and/or RasGRP4 pre-mRNA or a nucleoside having increased resistance to a nucleolytic enzyme due to substitution or the like. The nucleoside included in each wing segment is more preferably independently selected from a sugar-modified nucleoside and a deoxyribonucleoside, still more preferably independently selected from a 2'-4'-bridged nucleoside, a 2'-modified nucleoside, and a deoxyribonucleoside. Each wing segment even more preferably includes at least one 2'-4'-bridged nucleoside and particularly preferably includes at least one 2'-4'-bridged nucleoside and at least one 2'-modified nucleoside.

The 2'-4'-bridged nucleoside included in each wing segment is preferably at least one independently selected from the group consisting of LNA, cEt, ENA, BNA^{NC}, AmNA, GuNA, and scpBNA, and more preferably at least one independently selected from the group consisting of LNA, AmNA, and GuNA. Each wing segment preferably includes at least one LNA, more preferably 1 to 3 LNAs, further preferably 2 or 3 LNAs, and particularly preferably 3 LNAs.

The GuNA included in each wing segment is particularly preferably GuNA[tBu]. The AmNA included in each wing segment is particularly preferably AmNA[Me].

The 2'-modified nucleoside included in each wing segment is preferably at least one independently selected from the group consisting of a 2'-O-Me nucleoside, a 2'-O-MOE nucleoside, a 2'-O-AP nucleoside, a 2'-F nucleoside, a 2'-O-NMA nucleoside, a 2'-O-MCE nucleoside, a 2'-DMAECE nucleoside, a 2'-MorECE nucleoside, a 2'-PyECE nucleoside, and a 2'-BimECE nucleoside, more preferably at least one independently selected from the group consisting of a 2'-O-Me nucleoside, a 2'-O-MOE nucleoside, a 2'-O-NMA nucleoside, and a 2'-O-MCE nucleoside, and still more preferably a 2'-O-MCE nucleoside.

In another aspect, the 2'-modified nucleoside is independently selected from the group consisting of a 2'-O-MCE nucleoside and a 2'-MorECE nucleoside. In a certain embodiment, the 2'-modified nucleoside included in the 5' wing segment is independently selected from the group consisting of a 2'-O-MCE nucleoside and a 2'-MorECE nucleoside, and the 2'-modified nucleoside included in the 3' wing segment is a 2'-O-MCE nucleoside.

Preferably, each wing segment comprises 1 to 10 nucleosides independently selected from the group consisting of a sugar-modified nucleoside and a deoxyribonucleoside, and includes at least one 2'-4'-bridged nucleoside. More preferably, it comprises 2 to 6 nucleosides independently selected from the group consisting of a 2'-4'-bridged nucleoside and a 2'-modified nucleoside and includes at least one 2'-4'-bridged nucleoside and at least one 2'-modified nucleoside. Still more preferably, it comprises 2 to 5 nucleosides independently selected from the group consisting of a 2',4'-BNA and a 2'-modified nucleoside and includes at least two 2'-4'-bridged nucleosides and at least one 2'-modified nucleoside. Particularly preferably, it comprises 3 nucleosides independently selected from the group consisting of a 2'-4'-bridged nucleoside and a 2'-modified nucleoside and includes two 2'-4'-bridged nucleosides and one 2'-modified nucleoside.

In a certain embodiment, the 5' wing segment comprises 5 nucleosides independently selected from the group consisting of a 2'-4'-bridged nucleoside and a 2'-modified nucleoside and includes two 2'-4'-bridged nucleosides and three 2'-modified nucleosides, and the 3' wing segment comprises 3 nucleosides independently selected from the group consisting of a 2'-4'-bridged nucleoside and a 2'-modified nucleoside and includes two 2'-4'-bridged nucleosides and one 2'-modified nucleoside.

More specifically, each wing segment comprises 1 to 10 (preferably 2 to 6, more preferably 2 to 5, still more preferably 3) nucleosides independently selected from the group consisting of LNA, AmNA, GuNA, scpBNA, a 2'-O-Me nucleoside, a 2'-O-MOE nucleoside, a 2'-O-AP nucleoside, a 2'-F nucleoside, a 2'-O-NMA nucleoside, a 2'-O-MCE nucleoside, a 2'-DMAECE nucleoside, a 2'-MorECE nucleoside, a 2'-PyECE nucleoside, a 2'-BimECE nucleoside and a deoxyribonucleoside, and includes at least one selected from the group consisting of LNA, AmNA, and GuNA, more preferably includes at least one LNA, and still more preferably includes at least one LNA and at least one 2'-O-MCE nucleoside. It may or may not further include a 2'-MorECE nucleoside.

A preferable LNA included in each wing segment is β-D-methyleneoxy BNA.

In some embodiments of the present invention, the 5' wing segment and the 3' wing segment each independently include 2, 3, 4, 5, or 6 sugar-modified nucleosides, and the gap segment includes 8, 9, 10, 11, 12, 13, or 14 deoxyribonucleosides. The number of nucleosides in such a gapmer can be represented by (the number of nucleosides in the 5' wing segment)-(the number of nucleosides in the gap segment)-(the number of nucleosides in the 3' wing segment), and includes, for example, 5-10-5, 5-11-4, 4-11-5, 4-12-4, 3-14-3, 6-8-6, 3-12-3, 2-12-2, 2-11-3, 3-11-2, 3-10-3, 4-10-4, 3-10-4, 4-10-3, 3-9-3, 4-9-4, 3-9-4, 4-9-3, 3-8-3, 3-8-4, 4-8-3, and 4-8-4.

In some embodiments, the ASO includes at least 11, 12, 13, 14, or 15 contiguous nucleosides, the gap segment includes at least 5, 6, 7, 8, or 9 contiguous nucleosides, and the 5' wing segment and the 3' wing segment are any of the following (i) to (vi).
(i) The 5' wing segment and the 3' wing segment each independently include three or four selected from a 2'-O-MCE nucleoside and LNA, and include at least one 2'-O-MCE nucleoside and at least one LNA.
(ii) The 5' wing segment and the 3' wing segment each independently include one, two, three, four, five, or six LNAs. Particularly preferably, the 5' wing segment and the 3' wing segment each independently include 2 to 4 LNAs.
(iii) The 5' wing segment and the 3' wing segment each independently include one, two, three, four, five, or six 2'-O-MCE nucleosides.
(iv) The 5' wing segment includes three or four selected from a 2'-O-MCE nucleoside and LNA, and includes at least one 2'-O-MCE nucleoside and at least one LNA. The 3' wing segment includes 2 to 4 LNAs, particularly preferably 3 LNAs.
(v) The 5' wing segment includes 2 to 4 LNAs, particularly preferably 3 LNAs. The 3' wing segment includes three or four selected from a 2'-O-MCE nucleoside and LNA, and includes at least one 2'-O-MCE nucleoside and at least one LNA.
(vi) The 5' wing segment includes three to five selected from a 2'-O-MCE nucleoside, LNA, and a 2'-MorECE nucleoside, and includes at least one 2'-O-MCE nucleoside, at least one LNA, and at least one 2'-MorECE nucleoside. The 3' wing segment includes three to five selected from a 2'-O-MCE nucleoside, LNA, and a 2'-MorECE nucleoside, and includes at least one 2'-O-MCE nucleoside and at least one LNA.
(vii) The 5' wing segment includes three to five selected from a 2'-O-MCE nucleoside, LNA, and a 2'-MorECE nucleoside, and includes at least one 2'-O-MCE nucleoside, at least one LNA, and at least one 2'-MorECE nucleoside. The 3' wing segment includes three to five selected from a 2'-O-MCE nucleoside and LNA, and includes at least one 2'-O-MCE nucleoside and at least one LNA.

The arrangement of sugar modifications of the nucleosides constituting the 5' wing segment and the 3' wing segment in the ASO of the present invention is each preferably selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, VVVL, VLLVV, and VVLLV, more preferably selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, and VVVL, and still more preferably selected from the group consisting of VLL, VLLV, and LLV.

The arrangement of sugar modifications in the 5' wing segment is preferably VLL or VLLV, and the arrangement of sugar modifications in the 3' wing segment is preferably LLV or VLLV.

The arrangement of sugar modifications in the 5' wing segment is particularly preferably VLL, and the arrangement of sugar modifications in the 3' wing segment is particularly preferably LLV.

Here, L and V in the 5' wing segment and the 3' wing segment are sugar-modified nucleosides having different modified sugars, and a left side represents a 5' side and a right side represents a 3' side. Preferably, L's each independently represent a 2'-4'-bridged nucleoside and V's each independently represent a 2'-modified nucleoside.

More preferably, L represents LNA and V's each independently represent a 2'-O-MCE nucleoside or a 2'-MorECE nucleoside.

Particularly preferably, L represents LNA and V represents a 2'-O-MCE nucleoside.

In another aspect, particularly preferably, L represents LNA and V represents a 2'-MorECE nucleoside.

In another aspect, particularly preferably, the arrangement of sugar modifications in the 5' wing segment is VLL(Mo), and the arrangement of sugar modifications in the 3' wing segment is LLV.

In another aspect, particularly preferably, the arrangement of sugar modifications in the 5' wing segment is VLL(Mo)(Mo), and the arrangement of sugar modifications in the 3' wing segment is LLV.

In another aspect, particularly preferably, the arrangement of sugar modifications in the 5' wing segment is VLLVV, and the arrangement of sugar modifications in the 3' wing segment is LLV.

Here, the left side represents the 5' side, the right side represents the 3' side, preferably L represents LNA, V represents a 2'-O-MCE nucleoside, and (Mo) represents a 2'-MorECE nucleoside.

When the number of nucleosides included in the 5' wing segment is 2 or more (for example, 2 to 10), an internucleoside bond (an internucleoside bond between the wing segment and the gap segment is not included, the same applies hereinafter) included in the 5' wing segment is preferably at least one independently selected from a phosphodiester bond and a modified phosphodiester bond, and more preferably at least one independently selected from a phosphodiester bond and a phosphorothioate bond. The internucleoside bond included in the 5' wing segment preferably includes at least one phosphorothioate bond, preferably 50% or more of the internucleoside bonds are phosphorothioate bonds, more preferably 60% or more of the internucleoside bonds are phosphorothioate bonds, still more preferably 75% or more of the internucleoside bonds are phosphorothioate bonds, even still more preferably 80% or more of the internucleoside bonds are phosphorothioate bonds, even yet still more preferably 90% or more of the internucleoside bonds are phosphorothioate bonds, and particularly preferably all of the internucleoside bonds are phosphorothioate bonds.

The 3' wing segment is similar to the 5' wing segment.

In another aspect, from a viewpoint that toxicity can be reduced, all of the internucleoside bonds included in each wing segment may be phosphodiester bonds, and some of the internucleoside bonds may be phosphodiester bonds.

An internucleoside bond at a 5'-terminal included in the 5' wing segment is preferably a phosphorothioate bond. An internucleoside bond at a 3'-terminal included in the 3' wing segment is preferably a phosphorothioate bond. The internucleoside bonds other than the internucleoside bond at the 5'-terminal included in the 5' wing segment may be each a phosphodiester bond or a phosphorothioate bond. However, preferably, 50% or more of the internucleoside bonds are phosphodiester bonds, more preferably, 60% or more of the internucleoside bonds are phosphodiester bonds, still more preferably, 75% or more of the internucleoside bonds are phosphodiester bonds, even still more preferably, 80% or more of the internucleoside bonds are phosphodiester bonds, even yet still more preferably, 90% or more of the internucleoside bonds are phosphodiester bonds, and particularly preferably, all of the internucleoside bonds are phosphodiester bonds. The internucleoside bonds other than the internucleoside bond at the 3'-terminal included in the 3' wing segment may be each a phosphodiester bond or a phosphorothioate bond. However, preferably, 50% or more of the internucleoside bonds are phosphodiester bonds, more preferably, 60% or more of the internucleoside bonds are phosphodiester bonds, still more preferably, 75% or more of the internucleoside bonds are phosphodiester bonds, even still more preferably, 80% or more of the internucleoside bonds are phosphodiester bonds, even yet still more preferably, 90% or more of the internucleoside bonds are phosphodiester bonds, and particularly preferably, all of the internucleoside bonds are phosphodiester bonds. Among the internucleoside bonds included in each wing segment, an internucleoside bond located closest to the gap segment is preferably a phosphodiester bond.

For example, when the 5' wing segment includes two internucleoside bonds, the internucleoside bond at the 5'-terminal is preferably a phosphorothioate bond, and the internucleoside bond at a side of the gap segment is preferably a phosphodiester bond.

When the 5' wing segment includes three internucleoside bonds, the internucleoside bond at the 5'-terminal is preferably a phosphorothioate bond, and the other two internucleoside bonds included in the 5' wing segment are each independently preferably a phosphodiester bond or a phosphorothioate bond, and particularly preferably a phosphodiester bond.

When the 5' wing segment includes four internucleoside bonds, the internucleoside bond at the 5'-terminal is preferably a phosphorothioate bond, and the other three internucleoside bonds included in the 5' wing segment are each independently preferably a phosphodiester bond or a phosphorothioate bond, and particularly preferably a phosphodiester bond.

When the 3' wing segment includes two internucleoside bonds, the internucleoside bond at the 3'-terminal is preferably a phosphorothioate bond, and the internucleoside bond at a side of the gap segment is preferably a phosphodiester bond.

When the 3' wing segment includes three internucleoside bonds, the internucleoside bond at the 3'-terminal is preferably a phosphorothioate bond, and the other two internucleoside bonds included in the 3' wing segment are each independently preferably a phosphodiester bond or a phosphorothioate bond, and particularly preferably a phosphodiester bond.

When the 3' wing segment includes four internucleoside bonds, the internucleoside bond at the 3'-terminal is preferably a phosphorothioate bond, and the other three internucleoside bonds included in the 3' wing segment are each independently preferably a phosphodiester bond or a phosphorothioate bond, and particularly preferably a phosphodiester bond.

When the number of nucleosides included in the 5' wing segment is 1, the number of internucleoside bonds included in the 5' wing segment is 0, and when the number of nucleosides included in the 3' wing segment is 1, the number of internucleoside bonds included in the 3' wing segment is 0.

In the ASO of the present invention, preferably, the 3'-terminal of the 5' wing segment and the 5'-terminal of the gap segment are linked to each other by forming a phosphodiester bond or a modified phosphodiester bond, and the 5'-terminal of the 3' wing segment and the 3'-terminal of the gap segment are linked to each other by forming a phosphodiester bond or a modified phosphodiester bond. More preferably, the 3'-terminal of the 5' wing segment and the 5'-terminal of the gap segment are linked to each other by forming a modified phosphodiester bond, and the 5'-terminal of the 3' wing segment and the 3'-terminal of the gap segment are linked to each other by forming a modified phosphodiester bond. Still more preferably, the 3'-terminal of the 5' wing segment and the 5'-terminal of the gap segment are linked to each other by forming a phosphorothioate bond, and the 5'-terminal of the 3' wing segment and the 3'-terminal of the gap segment are linked to each other by forming a phosphorothioate bond.

In another preferable embodiment of the ASO of the present invention, the 3'-terminal of the 5' wing segment and the 5'-terminal of the gap segment are linked to each other by forming a phosphodiester bond, and the 5'-terminal of the 3' wing segment and the 3'-terminal of the gap segment are linked to each other by forming a phosphorothioate bond.

The present invention includes an ASO in which the 3'-terminal of the 5' wing segment and the 5'-terminal of the gap segment are linked to each other by forming a phosphorothioate bond, and the 5'-terminal of the 3' wing segment and the 3'-terminal of the gap segment are linked to each other by forming a phosphodiester bond. The present invention includes an ASO in which the 3'-terminal of the 5' wing segment and the 5'-terminal of the gap segment are linked to each other by forming a phosphodiester bond, and the 5'-terminal of the 3' wing segment and the 3'-terminal of the gap segment are linked to each other by forming a phosphodiester bond.

The ASO of the present invention is more preferably complementary to at least 8 contiguous nucleobases in a nucleobase sequence selected from the group consisting of nucleobase sequences represented by nucleobase position numbers:
237 to 252, 244 to 259, 501 to 516, 666 to 681, 683 to 698, 774 to 789, 889 to 904, 939 to 954, 964 to 979, 968 to 983, 969 to 984, 970 to 985, 971 to 986, 972 to 987, 974 to 989, 976 to 991, 977 to 992, 978 to 993, 979 to 994, 980 to 995, 981 to 996, 982 to 997, 1033 to 1048, 1036 to 1051, 1037 to 1052, 1038 to 1053, 1039 to 1054, 1040 to 1055, 1041 to 1056, 1114 to 1129, 1186 to 1201, 1245 to 1260, 1246 to 1261, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1395 to 1410, 1396 to 1411, 1397 to 1412, 1398 to 1413, 1399 to 1414, 1400 to 1415, 1401 to 1416, 1446 to 1461, 1523 to 1538, 1525 to 1540, 1526 to 1541, 1538 to 1553, 1590 to 1605, 1591 to 1606, 1592 to 1607, 1593 to 1608, 1668 to 1683, 1719 to 1734, 1744 to 1759, 1753 to 1768, 1756 to 1771, 1757 to 1772, 1758 to 1773, 1759 to 1774, 1760 to 1775, 1761 to 1776, 1813 to 1828, 1814 to 1829, 1815 to 1830, 1816 to 1831, 1817 to 1832, 1818 to 1833, 1819 to 1834, 1820 to 1835, 1821 to 1836, 1822 to 1837, 1823 to 1838, 1894 to 1909, 2042 to 2057, 2049 to 2064, 2050 to 2065, 2051 to 2066, and 3059 to 3074 of RasGRP4 mRNA represented by SEQ ID NO: 1. It is preferably complementary to 8 to 16, more preferably 12 to 16, still more preferably 15 to 16, and even still more preferably 16 nucleobases in the nucleobase sequence.

The ASO of the present invention is more preferably complementary to at least 8 contiguous nucleobases in a nucleobase sequence selected from the group consisting of nucleobase sequences represented by nucleobase position numbers:
964 to 979, 971 to 986, 974 to 989, 979 to 994, 980 to 995, 981 to 996, 982 to 997, 1033 to 1048, 1036 to 1051, 1037 to 1052, 1038 to 1053, 1039 to 1054, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1396 to 1411, 1397 to 1412, 1399 to 1414, 1400 to 1415, 1446 to 1461, 1523 to 1538, 1525 to 1540, 1538 to 1553, 1591 to 1606, 1593 to 1608, 1668 to 1683, 1758 to 1773, 1759 to 1774, 1760 to 1775, 1761 to 1776, 1813 to 1828, 1815 to 1830, 1817 to 1832, 1821 to 1836, 1822 to 1837, 1823 to 1838, 1818 to 1833, 1894 to 1909, and 2051 to 2066 of RasGRP4 mRNA represented by SEQ ID NO: 1. It is preferably complementary to 8 to 16, more preferably 12 to 16, still more preferably 15 to 16, and even still more preferably 16 nucleobases in the nucleobase sequence.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 979 to 994 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1038 to 1053 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1761 to 1776 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 2051 to 2066 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 980 to 995 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 981 to 996 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1036 to 1051 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1037 to 1052 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1039 to 1054 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1249 to 1264 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1253 to 1268 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1368 to 1383 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1396 to 1411 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1397 to 1412 of RasGRP4 mRNA represented by SEQ ID NO: 1.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 1400 to 1415 of RasGRP4 mRNA represented by SEQ ID NO: 1.

The complementarity between the ASO of the present invention and a corresponding portion of RasGRP4 mRNA represented by SEQ ID NO: 1 (for example, each of the above-described target regions) is at least 90% or more, and more preferably 95%, 96%, 97%, 98%, or 99% or more.

The ASO of the present invention preferably consists of 8 to 80 nucleosides complementary to at least a part of each of the above-described target regions of RasGRP4 mRNA represented by SEQ ID NO: 1.

The ASO of the present invention preferably consists of 12 to 30 nucleosides complementary to at least a part of each of the above-described target regions of RasGRP4 mRNA represented by SEQ ID NO: 1. It consists of more preferably 12 to 20, still more preferably 14 to 20, even still more preferably 15 to 18, even yet still more preferably 16 to 18 and particularly preferably 16 nucleosides.

The ASO of the present invention is more preferably complementary to at least 8 contiguous nucleobases in a nucleobase sequence selected from the group consisting of nucleobase sequences represented by nucleobase position numbers:
640 to 655, 967 to 982, 1037 to 1052, 1231 to 1246, 4838 to 4853, 4845 to 4860, 5575 to 5590, 5802 to 5817, 5918 to 5933, 6395 to 6410, 6544 to 6559, 6639 to 6654, 6771 to 6786, 6921 to 6936, 6938 to 6953, 7029 to 7044, 7033 to 7048, 7206 to 7221, 8439 to 8454, 8489 to 8504, 9361 to 9376, 9439 to 9454, 9632 to 9647, 9736 to 9751, 9740 to 9755, 9741 to 9756, 9742 to 9757, 9743 to 9758, 9744 to 9759, 9746 to 9761, 9748 to 9763, 9749 to 9764, 9750 to 9765, 9751 to 9766, 9752 to 9767, 9753 to 9768, 9754 to 9769, 9805 to 9820, 9808 to 9823, 9809 to 9824, 9810 to 9825, 9811 to 9826, 9812 to 9827, 9813 to 9828, 11814 to 11829, 11886 to 11901, 11945 to 11960, 11946 to 11961, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13468 to 13483, 13469 to 13484, 13470 to 13485, 13471 to 13486, 13472 to 13487, 13473 to 13488, 13474 to 13489, 13615 to 13630, 13692 to 13707, 13694 to 13709, 13695 to 13710, 13902 to 13917, 13903 to 13918, 13904 to 13919, 13905 to 13920, 14094 to 14109, 14145 to 14160, 14170 to 14185, 14179 to 14194, 14182 to 14197, 14183 to 14198, 14184 to 14199, 14185 to 14200, 14186 to 14201, 14187 to 14202, 14868 to 14883, 15529 to 15544, 15530 to 15545, 15531 to 15546, 15532 to 15547, 15533 to 15548, 15534 to 15549, 15535 to 15550, 15536 to 15551, 15537 to 15552, 15538 to 15553, 15539 to 15554, 15705 to 15720, 15787 to 15802, 15969 to 15984, 15976 to 15991, 15977 to 15992, 15978 to 15993, 16578 to 16593, and 17775 to 17790 of RasGRP4 pre-mRNA represented by SEQ ID NO: 2. It is preferably complementary to 8 to 16, more preferably 12 to 16, still more preferably 15 to 16, and even still more preferably 16 nucleobases in the nucleobase sequence.

The ASO of the present invention is more preferably complementary to at least 8 contiguous nucleobases in a nucleobase sequence selected from the group consisting of nucleobase sequences represented by nucleobase position numbers:
6639 to 6654, 7033 to 7048, 7206 to 7221, 9361 to 9376, 9632 to 9647, 9736 to 9751, 9743 to 9758, 9746 to 9761, 9751 to 9766, 9752 to 9767, 9753 to 9768, 9754 to 9769, 9805 to 9820, 9808 to 9823, 9809 to 9824, 9810 to 9825, 9811 to 9826, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13469 to 13484, 13470 to 13485, 13472 to 13487, 13473 to 13488, 13615 to 13630, 13692 to 13707, 13694 to 13709, 13903 to 13918, 13905 to 13920, 14094 to 14109, 14184 to 14199, 14185 to 14200, 14186 to 14201, 14187 to 14202, 14868 to 14883, 15529 to 15544, 15531 to 15546, 15533 to 15548, 15534 to 15549, 15537 to 15552, 15538 to 15553, 15539 to 15554, 15705 to 15720, 15977 to 15992, and 15978 to 15993 of RasGRP4 pre-mRNA represented by SEQ ID NO: 2. It is preferably complementary to 8 to 16, more preferably 12 to 16, still more preferably 15 to 16, and even still more preferably 16 nucleobases in the nucleobase sequence.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 7206 to 7221 of RasGRP4 pre-mRNA represented by SEQ ID NO: 2.

The ASO of the present invention is particularly preferably complementary to 8 to 16 (particularly preferably 16) contiguous nucleobases in a nucleobase sequence represented by nucleobase position numbers: 9751 to 9766 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9810 to 9825 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 14187 to 14202 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 15978 to 15993 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9752 to 9767 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9753 to 9768 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9808 to 9823 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9809 to 9824 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 9811 to 9826 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 11949 to 11964 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 11953 to 11968 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 13441 to 13456 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 13469 to 13484 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 13470 to 13485 of RasGRP4 pre-mRNA represented by SEQ ID NO: 2.

The ASO of the present invention is particularly preferably complementary to 8 to 16 (preferably 10 to 16, 12 to 16, 13 to 16, 14 to 16, or 15 to 16, particularly preferably 16) contiguous nucleobases in a nucleobase sequence represented by nucleobase position numbers: 13473 to 13488 of RasGRP4 pre-mRNA represented by SEQ ID NO: 2.

The complementarity between the ASO of the present invention and a corresponding portion of RasGRP4 pre-mRNA represented by SEQ ID NO: 2 (for example, each of the above-described target regions) is at least 90% or more, and more preferably 95%, 96%, 97%, 98%, or 99% or more.

The ASO of the present invention preferably consists of 8 to 80 nucleosides complementary to at least a part of each of the above-described target regions of RasGRP4 mRNA represented by SEQ ID NO: 1.

The ASO of the present invention preferably consists of 12 to 30 nucleosides complementary to at least a part of each of the above-described target regions of RasGRP4 pre-mRNA represented by SEQ ID NO: 2. It consists of more preferably 12 to 20, still more preferably 14 to 20, even still more preferably 15 to 18, even yet still more preferably 16 to 18 and particularly preferably 16 nucleosides.

The nucleobase sequence included in the ASO of the present invention preferably includes at least 11, more preferably 12, 13, 14, 15, or 16 contiguous nucleobases of any of nucleobase sequences of SEQ ID NOs: 3 to 123.

The nucleobase sequence included in the ASO of the present invention more preferably includes any of nucleobase sequences of SEQ ID NOs: 3 to 123.

The nucleobase sequence included in the ASO of the present invention preferably has a nucleobase sequence including at least 11, more preferably 12, 13, 14, 15, or 16 contiguous nucleobases of any one nucleobase sequence in a nucleobase sequence group A, still more preferably includes any one nucleobase sequence in the nucleobase sequence group A, and even still more preferably has any one nucleobase sequence in the nucleobase sequence group A.

The nucleobase sequence included in the ASO of the present invention more preferably has a nucleobase sequence including at least 11, more preferably 12, 13, 14, 15, or 16 contiguous nucleobases of any one nucleobase sequence in a nucleobase sequence group B, still more preferably includes any one nucleobase sequence in the nucleobase sequence group B, and even still more preferably has any one nucleobase sequence in the nucleobase sequence group B.

In another embodiment, the nucleobase sequence included in the ASO of the present invention more preferably has a nucleobase sequence including at least 11, 12, 13, 14, 15, or 16 contiguous nucleobases of any one nucleobase sequence in a nucleobase sequence group C, and still more preferably includes any one nucleobase sequence in the nucleobase sequence group C, and even still more preferably has any one nucleobase sequence in the nucleobase sequence group C in another aspect.

In another embodiment, the nucleobase sequence included in the ASO of the present invention more preferably has a nucleobase sequence including at least 11, 12, 13, 14, 15, or 16 contiguous nucleobases of any one nucleobase sequence in a nucleobase sequence group D, and still more preferably includes any one nucleobase sequence in the nucleobase sequence group D, and even still more preferably has any one nucleobase sequence in the nucleobase sequence group C in another aspect.

In such an aspect, the ASO of the present invention consists of 12 to 30, more preferably 12 to 20, still more preferably 14 to 20, even still more preferably 15 to 18, even yet still more preferably 16 to 18, and particularly preferably 16 nucleosides.

Nucleobase sequence group A:
a group including SEQ ID NOs: 4, 5, 6, 8, 10, 11, 12, 13, 14, 15, 16, 17, 19, 20, 21, 22, 23, 24, 25, 26, 27, 28, 29, 30, 31, 32, 34, 35, 36, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 71, 72, 73, 74, 75, 76, 77, 78, 79, 80, 81, 82, 83, 84, 85, 86, 87, 95, 96, 97, 99, 100, 102, 103, 104, 105, 106, 107, 108, 109, 110, 111, 112, 113, 114, 115, 116, 117, 118, 119, 120, 121, 122, and 123

The mRNA residual rate for these sequences was 80% or less.

Nucleobase sequence group B:
a group including SEQ ID NOs: 11, 12, 13, 14, 17, 19, 20, 21, 22, 23, 24, 25, 26, 28, 29, 30, 31, 44, 47, 48, 50, 52, 53, 57, 62, 63, 64, 65, 66, 67, 73, 74, 75, 76, 77, 78, 79, 81, 82, 84, 86, 97, 104, 105, 106, 107, 108, 110, 112, 115, 116, 117, and 121

The mRNA residual rate for these sequences was 30% or less.

Nucleobase sequence group C:
a group including SEQ ID NOs: 12, 14, 28, 31, 62, 63, 65, 66, 67, 75, 78, 79, 81, 82, 105, and 108

Nucleobase sequence group D: a group including SEQ ID NOs: 21, 28, and 82. These are nucleobase sequences corresponding to Manufacturing Example 4.

In an ASO having a nucleobase sequence including at least 11 contiguous nucleobases of any one nucleobase sequence in the nucleobase sequence groups A, B, C, and D, each wing segment preferably has at least one 2'-4'-bridged nucleoside, more preferably has at least one LNA, and more preferably further has at least one 2'-O-MCE nucleoside. Particularly preferably, the 3' wing segment and the 5' wing segment have two LNAs and one 2'-O-MCE nucleoside.

Among the nucleobase sequence groups A, B, C, and D, any one nucleobase sequence in the nucleobase sequence group C is particularly preferable.

The arrangement of sugar modifications in the 5' wing segment in the sequence is particularly preferably VLL, and the arrangement of sugar modifications in the 3' wing segment is particularly preferably LLV.

Here, L and V in the 5' wing segment and the 3' wing segment are sugar-modified nucleosides having different modified sugars, and a left side represents a 5' side and a right side represents a 3' side. Preferably, L represents a 2'-4'-bridged nucleoside and V represents a 2'-modified nucleoside. Particularly preferably, L represents LNA and V represents a 2'-O-MCE nucleoside.

The gap segment in the sequence particularly preferably comprises 10 deoxyribonucleosides.

A functional molecule may be directly or indirectly bonded to the ASO of the present invention. The bond between the functional molecule and the ASO may be direct or indirect via another substance. However, the oligonucleotide and the functional molecule are preferably bonded to each other by a covalent bond, an ionic bond, or a hydrogen bond. More preferably, the functional molecule and the ASO are directly bonded to each other by a covalent bond or bonded to each other via a linker (linking group) by a covalent bond from a viewpoint of high bonding stability.

When the functional molecule is bonded to the ASO by a covalent bond, the functional molecule is preferably directly or indirectly bonded to a 3'-terminal or a 5'-terminal of the ASO molecule. The bonding between the linker or the functional molecule and a terminal nucleoside of the ASO molecule is selected depending on the functional molecule.

The linker or the functional molecule and the terminal nucleoside of the ASO molecule are preferably linked to each other by a phosphodiester bond or a modified phosphodiester bond.

The linker or the functional molecule may be directly linked to an oxygen atom at the 3'-position of a nucleoside at the 3'-terminal or an oxygen atom at the 5'-position of a nucleoside at the 5'-terminal of the ASO molecule.

The structure of "functional molecule" (conjugate) is not particularly limited, and bonding of the functional molecule to the ASO imparts a desired function to the ASO. Examples of the desired function include a labeling function, a purification function, and a function of delivery to a target site. Examples of a molecule that imparts a labeling function include compounds such as a fluorescent protein, luciferase. Examples of a molecule that imparts a purification function include compounds such as biotin, avidin, His tag peptide, GST tag peptide, FLAG tag peptide.

In addition, a molecule having a function of delivering the ASO to a target site is preferably bonded as the functional molecule from a viewpoint of highly specifically and efficiently delivering the ASO to a target site (for example, skeletal muscle, skin, joint, or lung) with very effectively controlling RasGRP4 gene expression by the ASO.

Examples of the functional molecule capable of efficiently delivering the ASO of the present application to skeletal muscle, skin, or the like include a lipid. Examples of the lipid include: cholesterol; vitamins such as vitamin E (tocopherols and tocotrienols), vitamin A, vitamin D, vitamin K; steroids such as glucocorticoids, mineralocorticoids, estrogens, androgens, progesterone; fatty acids such as C5-30 saturated fatty acids, C5-30 unsaturated fatty acids; intermediate metabolites such as acylcarnitine, acyl-CoA; glycolipids; glycerides; derivatives thereof, and the like.

The lipid used is preferably a fatty acid such as a C5-30 saturated fatty acid, a C5-30 unsaturated fatty acid. Here, the saturated C5-30 fatty acid is a linear or branched saturated fatty acid having 5 to 30 carbon atoms, and specific examples thereof include palmitic acid, stearic acid, and the like. The C5-30 unsaturated fatty acid is a linear or branched unsaturated fatty acid having at least one carbon-carbon double bond and 5 to 30 carbon atoms, and specific examples thereof include oleic acid, linoleic acid, α-linolenic acid, γ-linolenic acid, arachidonic acid, icosapentaenoic acid, docosahexaenoic acid, and the like.

The lipid used is more preferably a C15-25 saturated fatty acid or a C15-25 unsaturated fatty acid, and still more preferably a C15-25 saturated fatty acid. The C15-25 saturated fatty acid is a linear or branched saturated fatty acid having 15 to 25 carbon atoms, and the C15-25 unsaturated fatty acid is a linear or branched unsaturated fatty acid having at least one carbon-carbon double bond and 15 to 25 carbon atoms. The lipid used is more preferably a linear fatty acid among C15-25 saturated fatty acids and C15-25 unsaturated fatty acids, still more preferably a linear C15-25 saturated fatty acid, even still more preferably a linear C15-20 saturated fatty acid, and particularly preferably palmitic acid. The C15-20 saturated fatty acid is a saturated fatty acid having 15 to 20 carbon atoms among the C15-25 saturated fatty acids.

The other lipids used are preferably cholesterol or vitamin E, more preferably tocopherols, still more preferably tocopherol, and particularly preferably α-tocopherol, from a viewpoint of improving the accumulation in skeletal muscle.

As a functional molecule capable of delivering the ASO of the present invention to the liver with high specificity, a sugar derivative having an interaction with an asialoglycoprotein receptor may be used.

The "asialoglycoprotein receptor" is present on a surface of a liver cell, and has an action of recognizing a galactose residue of asialoglycoprotein and taking in and decomposing a molecule of the asialoglycoprotein into the cell. The "sugar derivative having an interaction with an asialoglycoprotein receptor" is preferably a compound that has a structure similar to that of a galactose residue and is taken into a cell by an interaction with an asialoglycoprotein receptor, and examples thereof include a GalNAc (N-acetylgalactosamine) derivative, a galactose derivative, and a lactose derivative.

Examples of a functional molecule capable of efficiently delivering the ASO to an organ with high specificity by interacting with various proteins on a surface of a cell of the organ include a ligand of a receptor, an antibody, and a peptide or a protein of fragments thereof.

The linker via which the functional molecule and the ASO are bonded to each other only needs to exhibit the function of the functional molecule as the ASO molecule, and therefore is not particularly limited as long as the linker stably bonds the functional molecule and the oligonucleotide to each other. Examples of the linker include a group derived from an oligonucleotide including 1 to 20 nucleosides, a group derived from a polypeptide including 1 to 20 amino acids, an alkylene including 1 to 20 carbon atoms, and an alkenylene including 2 to 20 carbon atoms. The group derived from an oligonucleotide including 1 to 20 nucleosides is a divalent group obtained by removing a hydrogen atom or the like from each of a 3'-terminal and a 5'-terminal of an oligonucleotide including 1 to 20 nucleosides (a nucleoside when the number of nucleosides is 1). For the group derived from an oligonucleotide including 1 to 20 nucleosides, for example, WO 2017/053995 A can be referred to. WO 2017/053995 A describes, for example, a 3-base linker having a TCA motif, a 1- to 5-base linker having no TCA motif, and the like. The group derived from a polypeptide including 1 to 20 amino acids is a divalent group obtained by removing two groups selected from hydroxy, a hydrogen atom, amino, and the like from a polypeptide including 1 to 20 amino acids (an amino acid when the number of amino acids is 1).

The functional molecule may also be bonded to any of the above-described sequences. For example, it is preferable that the lipid is bonded to an ASO having a nucleobase sequence selected from the nucleobase sequence group A, B, C, or D. Particularly preferably, it is preferable that a C15-25 saturated fatty acid (preferably a C15-20 saturated fatty acid or palmitic acid) is bonded to an ASO having a nucleobase sequence selected from the nucleobase sequence group D. A particularly preferable compound to which a lipid is bonded is any of C16-BN-0019, C16-BN-0026, C16-BN-0181, C16-BN-0196, and C16-BN-0211, and more particularly preferably any of C16-BN-0181, C16-BN-0196, and C16-BN-0211.

A compound comprising the ASO of the present invention may include a prodrug moiety.

The prodrug is a derivative of a pharmaceutical compound having a group capable of being chemically or metabolically decomposed, and is a compound guided to a pharmacologically active pharmaceutical compound by solvolysis or decomposition in vivo under physiological conditions. A method for selecting and a method for manufacturing an appropriate prodrug derivative are described, for example, in Design of Prodrugs (Elsevier, Amsterdam, 1985). Examples of a prodrug moiety such as an acyloxy derivative manufactured by reacting a hydroxy group of an ASO (or a compound comprising an ASO) with a suitable acyl halide, a suitable acid anhydride, or a suitable halogenated alkyloxycarbonyl compound include -O-C(=O)C₂H₅, -O-C(=O)(t-Bu), -O-C(=O)C₁₅H₃₁, -O-C(=O)-(m-CO₂Na-Ph), -O-C(=O)CH₂CH₂CO₂Na, - OC(=O)CH(NH₂)CH₃, -O-C(=O)CH₂N(CH₃)₂, -O-CH₂OC(=O)CH₃, -NH-C(=O)C₂H₅, - NH-C(=O)(t-Bu), -NH-C(=O)C₁₅H₃₁, -NH-C(=O)-(m-CO₂Na-Ph), -NH-C(=O)CH₂CH₂CO₂Na, -NHC(=O)CH(NH₂)CH₃, -NH-C(=O)CH₂N(CH₃)₂, -NH-CH₂OC(=O)CH₃, and the like.

The prodrug of the compound comprising the ASO of the present invention also includes, for example, a complex obtained by hybridization of two or more oligonucleotides. Examples of such a preferable structure (prodrug) include a doublestranded oligonucleotide (for example, WO 2013/089283 A, WO 2017/068791 A, WO 2017/068790 A, or WO 2018/003739 A) comprising an oligonucleotide complementary to an ASO (or a compound comprising an ASO), in which the complementary oligonucleotide includes a ribonucleoside (for example, RNA), a peptide nucleic acid (PNA), or a deoxyribonucleoside (for example, DNA), an oligonucleotide in which an RNA complementary to an ASO is bonded by a linker (for example, WO 2017/131124 A or WO 2018/143475 A), and the like. The linker may be an oligonucleotide linker or a linker including a non-nucleoside structure. Examples of the preferable structure (prodrug) also include an oligonucleotide in which an RNA complementary to an ASO is directly linked (WO 2019/022196 A).

The compound of the present invention includes a compound in which two identical or different ASOs are linked to each other. For a structure in which two ASOs are linked to each other, for example, WO 2017/131124 A and WO 2018/143475 A can be referred to.

The compound comprising an ASO includes those present via tautomerism thereof or geometric isomerism thereof, and also includes those present as a mixture thereof or a mixture of isomers thereof. When an asymmetric center is present, or when an asymmetric center is generated as a result of isomerization, the compound comprising an ASO also includes those present as each optical isomer and those present as a mixture thereof at any ratio. In a case of a compound having two or more asymmetric centers, diastereomers due to optical isomerism of each of the asymmetric centers are also present. The present invention also includes those including all these types at any ratio. An optically active form can be obtained by a well-known method for this purpose.

For example, when the compound comprising the ASO of the present invention includes a modified phosphodiester bond (for example, a phosphorothioate bond) and the phosphorus atom is an asymmetric atom, both forms of an oligonucleotide in which the stereochemistry of the phosphorus atom is controlled and an oligonucleotide in which the stereochemistry of the phosphorus atom is not controlled are included in the scope of the present invention.

The compound comprising the ASO, a prodrug thereof, or a pharmacologically acceptable salt thereof of the present invention can be present in any crystalline form depending on manufacturing conditions, and can be present in any hydrate, but these crystalline forms, hydrates, and mixtures thereof are also included in the scope of the present invention. In addition, it may be present as a solvate containing an organic solvent such as acetone, ethanol, 1-propanol, or 2-propanol, but all of these forms are included in the scope of the present invention.

The compound comprising the ASO of the present invention can be converted into a pharmacologically acceptable salt or can be liberated from a generated salt, as necessary. Examples of the pharmacologically acceptable salt of the compound comprising the ASO include salts with an alkali metal (lithium, sodium, potassium, or the like), an alkaline earth metal (calcium or the like), magnesium, ammonium, an organic base (triethylamine, trimethylamine, or the like), an amino acid (glycine, lysine, glutamic acid, or the like), an inorganic acid (hydrochloric acid, hydrobromic acid, phosphoric acid, sulfuric acid, or the like), or an organic acid (acetic acid, citric acid, maleic acid, fumaric acid, tartaric acid, benzenesulfonic acid, methanesulfonic acid, p-toluenesulfonic acid, or the like).

In particular, a partial structure represented by -P(=O)(OH)- may be converted into an anionic partial structure represented by -P(=O)(O⁻)- to form a salt with an alkali metal (lithium, sodium, potassium, or the like), an alkaline earth metal (calcium or the like), magnesium, ammonium, or the like. A partial structure represented by - P(=O)(SH)- that forms a phosphorothioate bond may be converted into an anionic partial structure represented by -P(=O)(S⁻)- to form a salt with an alkali metal, an alkaline earth metal, ammonium, or the like similarly. The same applies to other modified phosphodiester bonds.

The pharmacologically acceptable salt is particularly preferably a sodium salt.

The compound comprising the ASO of the present invention can be prepared by appropriately selecting a method known to a person skilled in the art. For example, a person skilled in the art can synthesize the compound comprising the ASO of the present invention by designing a nucleoside sequence of the ASO based on information of a nucleoside sequence of a target RNA, and using a commercially available automatic nucleic acid synthesizer (manufactured by Applied Biosystems, manufactured by Beckman, manufactured by GeneDesign, Inc., or the like). The compound comprising the ASO of the present invention can also be synthesized by a reaction using an enzyme. Examples of the enzyme include polymerase, ligase, restriction enzyme, and the like, but are not limited thereto. That is, the method for manufacturing the compound comprising the ASO according to the present embodiment can include a step of extending a nucleoside chain at a 3'-terminal or a 5'-terminal.

Many methods for bonding a functional molecule and an oligonucleotide to each other are well known in the present field. For example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to. For example, a functional molecule and a linker are bonded to each other by a known method, then the bonded product is guided to an amidite form with an amidite reagent or an H-phosphonate form with an H-phosphonate reagent, and can be bonded to an oligonucleotide.

By purifying the resulting oligonucleotide by reverse phase column chromatography or the like, a compound comprising an ASO can be prepared.

The compound comprising the ASO or a pharmacologically acceptable salt thereof of the present invention can effectively inhibit RasGRP4 gene expression. The disease that can be treated, prevented, and ameliorated by the nucleic acid pharmaceutical utilizing the compound comprising the ASO or a pharmacologically acceptable salt thereof of the present invention is not particularly limited as long as it is a disease for which a RasGRP4 gene expression inhibitory action is effective, and examples include polymyositis, dermatomyositis, rheumatoid arthritis, an inflammatory bowel disease, systemic lupus erythematosus, asthma, mastocytosis, mast cell leukemia, neutrophilic inflammation in vasculitis syndromes, thrombus formation due to macrophage activation and NETs (neutrophil extracellular traps) release from neutrophils in COVID-19, a collagen disease, an autoimmune disease, an auto inflammatory disease, an allergic disease, and the like, among which polymyositis and/or dermatomyositis are included.

The present invention can provide a composition containing the compound comprising the ASO as an active ingredient, for example, for inhibiting RasGRP4 gene expression by an antisense effect. In particular, the compound comprising the ASO of the present invention can also provide a pharmaceutical composition for treating, preventing, and/or ameliorating a disease, for which inhibition of RasGRP4 gene expression is effective, such as polymyositis, dermatomyositis, rheumatoid arthritis, an inflammatory bowel disease, systemic lupus erythematosus, asthma, mastocytosis, mast cell leukemia, neutrophilic inflammation in vasculitis syndromes, thrombus formation due to macrophage activation and NETs release from neutrophils in COVID-19, a collagen disease, an autoimmune disease, an autoinflammatory disease, or an allergic disease.

A pharmaceutical composition containing the compound comprising the ASO or a pharmacologically acceptable salt thereof of the present invention can be formulated by a known pharmaceutical method. For example, the pharmaceutical composition can be used transintestinally (orally or the like) or non-transintestinally as an injection, a capsule, a tablet, a pill, a liquid, a powder, a granule, a fine granule, a film coating agent, a pellet, a troche, a sublingual agent, a chewing agent, a buccal agent, a paste, a syrup, a suspension agent, an elixir agent, an emulsion, a coating agent, an ointment, a plaster, a cataplasm, a transdermal absorption formulation, a lotion, an inhalation agent, an aerosol agent, a suppository, or the like.

In the formulation, the pharmaceutical composition can be appropriately combined with a pharmacologically acceptable carrier or a carrier acceptable as food/drink, specifically, sterile water, physiological saline, vegetable oil, a solvent, a base, an emulsifier, a suspension agent, a surfactant, a pH adjuster, a stabilizer, a flavoring agent, a fragrance, an excipient, a vehicle, a preservative, a binder, a diluent, an isotonizing agent, a soothing agent, an extender, a disintegrant, a buffer, a coating agent, a lubricant, a coloring agent, a sweetening agent, a thickening agent, a corrective, a solubilizing agent, other additives, or the like.

An administration form of the composition containing the compound comprising the ASO or a pharmacologically acceptable salt thereof of the present invention is not particularly limited, and examples thereof include transintestinal (oral or the like) and non-transintestinal administration. More preferable examples thereof include intravenous administration, intraarterial administration, intraarticular administration, intraperitoneal administration, subcutaneous administration, intradermal administration, airway administration, rectal administration, intramuscular administration, intrathecal administration, intracerebroventricular administration, nasal administration, intravitreal administration, and administration by infusion. Still more preferable examples thereof include intravenous administration, intraarticular administration, and subcutaneous administration, and particularly preferable examples thereof include subcutaneous administration.

A formulation for subcutaneous administration, intraarticular administration, intravenous administration, or the like, containing a compound comprising the ASO or a pharmacologically acceptable salt thereof of the present invention, can be produced by a known conventional method. For example, a preparation obtained by adding an additive such as a buffer or an isotonizing agent to the compound comprising the ASO or a pharmacologically acceptable salt thereof of the present invention is completely dissolved in water for injection, and then the resulting preparation solution is sterilized by filtration. A prefilled syringe formulation can be manufactured by filling the preparation solution into a sterilized syringe, a formulation for injection can be manufactured by filling the preparation solution into a sterilized vial, and a formulation for preparation at the time of use can be manufactured by filling the preparation solution into a sterilized vial and then freeze-drying the preparation solution. Instead of the filtration sterilization, terminal sterilization such as autoclaving or gamma irradiation may be performed.

Various mammalian diseases can be treated, prevented and/or ameliorated by the composition containing the compound comprising the ASO or a pharmacologically acceptable salt thereof of the present invention. For example, diseases in mammals including but not limited to a human, a cow, a sheep, a goat, a horse, a dog, a cat, a guinea pig, rat, a rabbit, a chimpanzee, a bovine, an ovine, an equine, a canine, a feline, and a rodent species such as a mouse can be treated, prevented and/or ameliorated. The mammal is particularly preferably a human.

When the composition containing the compound comprising the ASO or a pharmacologically acceptable salt thereof of the present invention is administered to or ingested by a mammal including a human, a dose thereof or an intake thereof is preferably an effective amount. The effective amount means a sufficient amount of the compound to exhibit a desired pharmacological effect in an individual who needs a drug, changes depending on the age, weight, symptom, and health condition of an individual whose disease is treated, prevented, and/or ameliorated, blending in the composition, and the like, and is appropriately selected. The dose or the intake is preferably 0.0001 mg/kg/day to 100 mg/kg/day in terms of ASO.

Examples of a preferable method using the ASO of the present invention include those described below.

A method for controlling RasGRP4 gene expression, the method including a step of bringing a cell into contact with the compound comprising the ASO or a pharmacologically acceptable salt thereof of the present invention.

A method for controlling RasGRP4 gene expression in a mammal, the method including a step of administering a pharmaceutical composition containing the compound comprising the ASO or a pharmacologically acceptable salt thereof of the present invention to the mammal.

Use of the compound comprising the ASO or a pharmacologically acceptable salt thereof of the present invention for controlling RasGRP4 gene expression in a mammal.

Use of the compound comprising the ASO or a pharmacologically acceptable salt thereof of the present invention for manufacturing a drug for controlling RasGRP4 gene expression in a mammal.

### Examples

Hereinafter, the present invention will be described more specifically with reference to Examples, but the embodiments of the present invention are not limited to the following Examples. In Examples, "Cmpd No" means a compound number. The sequence represented by "SEQ No. of BASE SEQ" is expressed independently of any modification of a sugar moiety, an internucleoside bond, or a nucleobase moiety. In the chemical structure, a modified form of a sugar moiety, an internucleoside bond, or a nucleobase moiety of an oligonucleotide is shown, and a unique sequence number (SEQ No. of compd.) is assigned to the compound.

In the notation of a chemical structure in Examples, unless otherwise specified, "(L)" means LNA (β-D-methyleneoxy BNA) nucleoside, "(Mo)" means 2'-MorECE nucleoside, "(V)" means 2'-O-MCE nucleoside, a lower case alphabet (excluding "o" in the "5(Mo)") means a deoxyribonucleoside, a capital letter (excluding "L" in the (L), "V" in (V), and "M" in (Mo)) means a ribonucleoside, "^" means a phosphorothioate bond, "5(x)" means that a nucleobase of the deoxyribonucleoside is 5-methylcytosine, and "5" in "5(V)", "5(L)", and "5(Mo)" means that a nucleobase of the nucleoside is 5-methylcytosine.

Note that in the notation of a chemical structure in Examples, when "^" is not described between two adjacent nucleosides, the internucleoside bond between the two nucleosides is a phosphodiester bond. For example, in the notation with "5(L)A(L)", the internucleoside bond between 2'-O-4'-C-Locked 5-methylcytidine and 2'-O-4'-CLocked adenosine is a phosphodiester bond, and in the notation with "5(L)g", the internucleoside bond between 2'-O-4'-C-Locked 5-methylcytidine and deoxyguanosine is a phosphodiester bond.

### (Manufacturing Example 1)

Antisense oligonucleotides (compounds represented by chemical structures corresponding to compound numbers) described in Tables 1 to 5 were prepared using an automatic nucleic acid synthesizer nS-8II (manufactured by GeneDesign, Inc.) or NTS M-8-SE (manufactured by Nihon Techno Service Co., Ltd.). Note that the title rows in Tables 2 and 3 are the same as that in Table 1, and thus are omitted.

**[Table 1]**

| Compd. No. | Chemical structure |
|---|---|
| BN-0001 | T(V)^G(L)^G(L)^g^g^5(x)^a^t^g^t^5(x)^t^t^G(L)^T(L)^G(V) |
| BN-0002 | 5(V)^5(L)^T(L)^t^g^5(x)^t^g^a^t^t^t^5(x)^5(L)^5(L)^G(V) |
| BN-0003 | T(V)^5(L)^A(L)^g^5(x)^5(x)^a^g^t^a^5(x)^5(x)^t^G(L)^A(L)^5(V) |
| BN-0004 | 5(V)^T(L)^T(L)^t^t^t^g^5(x)^5(x)^5(x)^a^g^g^5(L)^5(L)^T(V) |
| BN-0005 | A(V)^5(L)^T(L)^5(x)^5(x)^a^g^g^t^a^g^g^t^G(L)^A(L)^G(V) |
| BN-0006 | T(V)^G(L)^A(L)^t^a^g^5(x)^5(x)^t^g^g^a^a^G(L)^G(L)^A(V) |
| BN-0007 | T(V)^5(L)^A(L)^g^5(x)^a^5(x)^5(x)^a^t^5(x)^a^5(x)^5(L)^T(L)^G(V) |
| BN-0008 | G(V)^A(L)^A(L)^t^g^a^a^5(x)^t^t^g^t^5(x)^5(L)^A(L)^G(V) |
| BN-0009 | G(V)^A(L)^A(L)^a^t^t^5(x)^t^g^5(x)^a^g^5(x)^T(L)^G(L)^G(V) |
| BN-0010 | G(V)^T(L)^G(L)^t^t^g^a^a^a^t^t^5(x)^t^G(L)^5(L)^A(V) |
| BN-0011 | T(V)^5(L)^5(L)^t^t^g^a^g^t^5(x)^t^g^g^A(L)^G(L)^A(V) |
| BN-0012 | G(V)^G(L)^G(L)^a^g^t^5(x)^5(x)^t^t^g^a^g^T(L)^5(L)^T(V) |
| BN-0013 | G(V)^5(L)^G(L)^t^a^g^t^t^t^t^g^t^G(L)^G(L)^G(V) |
| BN-0014 | A(V)^G(L)^G(L)^t^5(x)^5(x)^t^t^g^a^g^g^t^G(L)^5(L)^A(V) |
| BN-0015 | T(V)^T(L)^5(L)^a^g^5(x)^t^t^g^g^g^t^a^G(L)^G(L)^T(V) |
| BN-0016 | G(V)^5(L)^A(L)^g^5(x)^a^g^a^t^5(x)^5(x)^t^5(x)^A(L)^T(L)^T(V) |
| BN-0017 | T(V)^G(L)^T(L)^a^g^a^a^g^a^g^g^t^5(x)^5(L)^A(L)^G(V) |
| BN-0018 | T(V)^5(L)^5(L)^g^t^g^t^a^g^a^a^g^a^G(L)^G(L)^T(V) |
| BN-0019 | 5(V)^5(L)^G(L)^g^g^5(x)^a^t^a^a^g^a^a^A(L)^G(L)^5(V) |
| BN-0020 | G(V)^T(L)^G(L)^5(x)^a^t^t^g^a^a^g^g^g^G(L)^G(L)^A(V) |
| BN-0021 | A(V)^5(L)^5(L)^a^g^5(x)^t^g^5(x)^t^5(x)^5(x)^a^5(L)^A(L)^T(V) |
| BN-0022 | G(V)^A(L)^A(L)^5(x)^a^5(x)^a^g^a^5(x)^t^5(x)^5(x)^A(L)^5(L)^5(V) |
| BN-0023 | 5(V)^G(L)^5(L)^t^5(x)^a^a^a^g^t^5(x)^5(x)^t^5(L)^5(L)^T(V) |
| BN-0024 | 5(V)^T(L)^5(L)^t^g^5(x)^ t^g^a^a^g^g^a^T(L)^5(L)^5(V) |
| BN-0025 | 5(V)^5(L)^A(L)^a^5(x)^t^t^g^g^a^g^5(x)^a^G(L)^A(L)^T(V) |
| BN-0026 | T(V)^T(L)^5(L)^g^g^a^a^g^g^t^g^a^5(x)^5(L)^T(L)^5(V) |
| BN-0027 | A(V)^G(L)^5(L)^5(x)^t^t^g^5(x)^t^t^g^g^t^G(L)^A(L)^5(V) |
| BN-0028 | G(V)^G(L)^5(L)^5(x)^t^5(x)^t^t^5(x)^t^t^a^5(x)^A(L)^T(L)^T(V) |
| BN-0029 | G(V)^G(L)^A(L)^t^t^5(x)^a^g^t^5(x)^t^g^g^G(L)^T(L)^5(V) |
| BN-0030 | G(V)^G(L)^G(L)^g^a^a^a^g^g^a^a^g^a^G(L)^G(L)^5(V) |
| BN-0031 | G(V)^G(L)^A(L)^a^a^g^a^g^g^a^a^5(x)^t^G(L)^5(L)^5(V) |
| BN-0032 | 5(V)^T(L)^5(L)^5(x)^5(x)^t^g^a^a^g^5(x)^a^t^5(L)^5(L)^A(V) |
| BN-0033 | A(V)^G(L)^5(L)^t^5(x)^a^a^a^5(x)^t^5(x)^5(x)^a^G(L)^G(L)^G(V) |
| BN-0034 | A(V)^G(L)^G(L)^a^g^t^g^g^t^t^t^a^a^G(L)^A(L)^G(V) |
| BN-0035 | A(V)^G(L)^T(L)^g^a^5(x)^t^t^a^5(x)^5(x)^t^a^A(L)^G(L)^G(V) |
| BN-0036 | G(V)^5(L)^5(L)^a^t^g^a^5(x)^5(x)^t^t^g^5(x)^T(L)^G(L)^A(V) |
| BN-0037 | 5(V)^A(L)^T(L)^g^a^5(x)^5(x)^t^t^g^g^a^5(x)^A(L)^G(L)^G(V) |
| BN-0038 | G(V)^G(L)^A(L)^t^a^t^g^a^g^g^t^5(x)^a^G(L)^5(L)^A(V) |
| BN-0039 | G(V)^A(L)^G(L)^t^t^5(x)^t^g^g^t^5(x)^a^a^G(L)^G(L)^5(V) |
| BN-0040 | T(V)^T(L)^G(L)^g^a^a^g^t^g^g^g^a^t^T(L)^A(L)^T(V) |
| BN-0041 | G(V)^T(L)^G(L)^5(x)^t^g^a^g^5(x)^t^a^g^g^G(L)^G(L)^G(V) |
| BN-0042 | T(V)^5(L)^A(L)^g^5(x)^ 5(x)^a^g^t^a^5(x)^5(x)^t^G(L)^A(L)^G(V) |
| BN-0043 | G(V)^G(L)^5(L)^t^g^a^5(x)^t^5(x)^a^5(x)^a^g^G(L)^T(L)^5(V) |
| BN-0044 | G(V)^G(L)^5(L)^t^g^5(x)^t^5(x)^a^t^t^g^g^G(L)^A(L)^G(V) |
| BN-0045 | A(V)^5(L)^5(L)^g^t^g^a^t^a^g^5(x)^5(x)^t^G(L)^G(L)^A(V) |
| BN-0046 | T(V)^G(L)^G(L)^5(x)^5(x)^t^5(x)^t^t^t^a^a^5(x)^T(L)^5(L)^A(V) |
| BN-0047 | A(V)^G(L)^5(L)^a^5(x)^5(x)^a^t^5(x)^a^5(x)^5(x)^t^G(L)^5(L)^A(V) |
| BN-0048 | 5(V)^T(L)^5(L)^g^a^g^a^g^t^5(x)^a^5(x)^A(L)^5(L)^A(V) |
| BN-0049 | T(V)^T(L)^G(L)^g^g^g^a^a^t^a^g^a^5(x)^A(L)^G(L)6G(V) |
| BN-0050 | A(V)^G(L)^G(L)^5(x)^a^t^g^t^g^t^g^g^a^5(L)^5(L)^A(V) |

**[Table 2]**

| | |
|---|---|
| BN-0051 | A(V)^G(L)^5(L)^t^g^g^t^g^g^a^g^5(x)^5(x)^T(L)^5(L)^T(V) |
| BN-0052 | 5(V)^T(L)^G(L)^5(x)^a^g^5(x)^t^g^g^t^g^g^A(L)^G(L)^5(V) |
| BN-0053 | T(V)^5(L)^T(L)^g^5(x)^a^g^5(x)^t^g^g^t^g^G(L)^A(L)^G(V) |
| BN-0054 | T(V)^T(L)^5(L)^t^g^5(x)^a^g^5(x)^t^g^g^t^G(L)^G(L)^A(V) |
| BN-0055 | A(V)^T(L)^T(L)^5(x)^t^g^5(x)^a^g^ 5(x)^t^g^g^T(L)^G(L)^G(V) |
| BN-0056 | A(V)^A(L)^T(L)^t^5(x)^t^g^5(x)^a^g^5(x)^t^g^G(L)^T(L)^G(V) |
| BN-0057 | T(V)^T(L)^G(L)^a^a^a^t^t^5(x)^t^g^5(x)^a^G(L)^5(L)^T(V) |
| BN-0058 | G(V)^T(L)^T(L)^g^a^a^a^t^t^5(x)^t^g^5(x)^A(L)^G(L)^5(V) |
| BN-0059 | T(V)^G(L)^T(L)^t^g^a^a^a^t^t^5(x)^t^g^5(L)^A(L)^G(V) |
| BN-0060 | 5(V)^G(L)^T(L)^g^t^t^g^a^a^a^t^t^5(x)^T(L)^G(L)^5(V) |
| BN-0061 | G(V)^5(L)^G(L)^t^g^t^t^g^a^a^a^t^t^5(L)^T(L)^G(V) |
| BN-0062 | A(V)^G(L)^5(L)^g^t^g^t^t^g^a^a^a^t^T(L)^5(L)^T(V) |
| BN-0063 | G(V)^A(L)^G(L)^t^5(x)^5(x)^t^t^g^a^g^t^5(x)^T(L)^G(L)^G(V) |
| BN-0064 | G(V)^G(L)^A(L)^g^t^5(x)^5(x)^t^t^g^a^g^t^5(L)^T(L)^G(V) |
| BN-0065 | T(V)^G(L)^G(L)^g^a^g^t^5(x)^5(x)^t^t^g^a^G(L)^T(L)^5(V) |
| BN-0066 | A(V)^T(L)^G(L)^g^g^a^g^t^5(x)^5(x)^t^t^g^A(L)^G(L)^T(V) |
| BN-0067 | 5(V)^A(L)^T(L)^g^g^g^a^g^t^5(x)^5(x)^t^t^G(L)^A(L)^G(V) |
| BN-0068 | T(V)^T(L)^G(L)^g^a^g^5(x)^t^t^t^g^g^g^G(L)^A(L)^G(V) |
| BN-0069 | T(V)^G(L)^G(L)^g^t^a^g^g^t^g^5(x)^a^g^G(L)^5(L)^G(V) |
| BN-0070 | T(V)^T(L)^G(L)^g^g^t^a^g^g^t^g^5(x)^a^G(L)^G(L)^5(V) |
| BN-0071 | 5(V)^T(L)^T(L)^g^g^g^t^a^g^g^t^g^5(x)^A(L)^G(L)^G(V) |
| BN-0072 | G(V)^5(L)^T(L)^t^g^g^g^t^a^g^g^t^g^5(L)^A(L)^G(V) |
| BN-0073 | A(V)^G(L)^5(L)^t^t^g^g^g^t^a^g^g^t^G(L)^5(L)^A(V) |
| BN-0074 | 5(V)^A(L)^G(L)^5(x)^t^t^g^g^g^t^a^g^g^T(L)^G(L)^5(V) |
| BN-0075 | T(V)^5(L)^A(L)^g^5(x)^t^t^g^g^g^t^a^g^G(L)^T(L)^G(V) |
| BN-0076 | G(V)^T(L)^T(L)^5(x)^a^g^5(x)^t^t^g^g^g^t^A(L)^G(L)^G(V) |
| BN-0077 | 5(V)^5(L)^G(L)^t^g^t^a^g^a^a^g^a^g^G(L)^T(L)^5(V) |
| BN-0078 | 5(V)^A(L)^T(L)^a^a^g^a^a^a^g^5(x)^t^5(x)^A(L)^T(L)^A(V) |
| BN-0079 | G(V)^5(L)^A(L)^t^a^a^g^a^a^a^g^5(x)^t^5(L)^A(L)^T(V) |
| BN-0080 | G(V)^G(L)^5(L)^a^t^a^a^g^a^a^a^g^5(x)^T(L)^5(L)^A(V) |
| BN-0081 | G(V)^G(L)^G(L)^5(x)^a^t^a^a^g^a^a^a^g^5(L)^T(L)^5(V) |
| BN-0082 | 5(V)^G(L)^G(L)^g^5(x)^a^t^a^a^g^a^a^a^G(L)^5(L)^T(V) |
| BN-0083 | 5(V)^5(L)^5(L)^g^g^g^5(x)^a^t^a^a^g^a^A(L)^A(L)^G(V) |
| BN-0084 | 5(V)^A(L)^G(L)^5(x)^t^g^5(x)^t^5(x)^5(x)^a^5(x)^a^T(L)^G(L)^5(V) |
| BN-0085 | 5(V)^A(L)^5(L)^5(x)^a^g^5(x)^t^g^5(x)^t^5(x)^5(x)^A(L)^5(L)^A(V) |
| BN-0086 | 5(V)^5(L)^A(L)^5(x)^5(x)^a^g^5(x)^t^g^5(x)^t^5(x)^5(L)^A(L)^5(V) |
| BN-0087 | T(V)^5(L)^5(L)^a^5(x)^5(x)^a^g^5(x)^t^g^5(x)^t^5(L)^5^(L)^A(V) |
| BN-0088 | G(V)^A(L)^A(L)^5(x)^a^5(x)^a^g^a5(x)^t^t^5(x)^A(L)^G(L)^A(V) |
| BN-0089 | A(V)^A(L)^A(L)^g^t^5(x)^5(x)^t^5(x)^5(x)^t^g^a^G(L)^A(L)^G(V) |
| BN-0090 | 5(V)^A(L)^A(L)^a^g^t^5(x)^5(x)^t^5(x)^5(x)^t^g^A(L)^G(L)^A(V) |
| BN-0091 | T(V)^5(L)^A(L)^a^a^g^t^5(x)^5(x)^t^5(x)^5(x)^t^G(L)^A(L)^G(V) |
| BN-0092 | 5(V)^T(L)^5(L)^a^a^a^g^t^5(x)^5(x)^t^5(x)^5(x)^T(L)^G(L)^A(V) |
| BN-0093 | G(V)^5(L)^T(L)^5(x)^a^a^a^g^t^5(x)^5(x)^t^5(x)^5(L)^T(L)^G(V) |
| BN-0094 | T(V)^5(L)^G(L)^5(x)^t^5(x)^a^a^a^g^t^5(x)^5(x)^T(L)^5(L)^5(V) |
| BN-0095 | G(V)^T(L)^5(L)^g^5(x)^t^5(x)^a^a^a^g^t^5(x)^5(L)^T(L)^5(V) |
| BN-0096 | 5(V)^T(L)^5(L)^t^5(x)^t^g^5(x)^t^g^a^a^g^G(L)^A(L)^T(V) |
| BN-0097 | T(V)^G(L)^G(L)^a^a^g^g^t^g^t^g^5(x)^a^G(L)^G(L)^A(V) |
| BN-0098 | G(V)^T(L)^G(L)^a^5(x)^5(x)^t^5(x)^a^t^g^g^a^A(L)^G(L)^G(V) |
| BN-0099 | G(V)^G(L)^T(L)^g^a^5(x)^5(x)^t^5(x)^a^t^g^g^A(L)^A(L)^G(V) |
| BN-0100 | A(V)^A(L)^G(L)^g^t^g^a^5(x)^5(x)^t^5(x)^a^t^G(L)^G(L)^A(V) |

**[Table 3]**

| | |
|---|---|
| BN-0101 | G(V)^A(L)^A(L)^g^g^t^g^a^5(x)^5(x)^t^5(x)^a^T(L)^G(L)^G(V) |
| BN-0102 | G(V)^G(L)^A(L)^a^g^g^t^g^a^5(x)^5(x)^t^5(x)^A(L)^T(L)^G(V) |
| BN-0103 | 5(V)^G(L)^G(L)^a^a^g^g^t^g^a^5(x)^5(x)^t^5(L)^A(L)^T(V) |
| BN-0104 | T(V)^5(L)^G(L)^g^a^a^g^g^t^g^a^5(x)^5(x)^T(L)^5(L)^A(V) |
| BN-0105 | T(V)^A(L)^G(L)^a^g^a^t^t^t^g^g^a^g^G(L)^A(L)^T(V) |
| BN-0106 | T(V)^G(L)^5(L)^t^t^g^g^t^g^a^5(x)^a^5(x)^5(L)^5(L)^5(V) |
| BN-0107 | T(V)^T(L)^G(L)^5(x)^t^t^g^g^t^g^a^5(x)^a^5(L)^5(L)^5(V) |
| BN-0108 | 5(V)^T(L)^T(L)^g^5(x)^t^t^g^g^t^g^a^5(x)^A(L)^5(L)^5(V) |
| BN-0109 | 5(V)^5(L)^T(L)^t^g^5(x)^t^t^g^g^t^g^a^5(L)^A(L)^5(V) |
| BN-0110 | G(V)^5(L)^5(L)^t^t^g^5(x)^t^t^g^g^t^g^A(L)^5(L)^A(V) |
| BN-0111 | T(V)^A(L)^G(L)^5(x)^5(x)^t^t^g^5(x)^t^t^g^g^T(L)^G(L)^A(V) |
| BN-0112 | G(V)^T(L)^A(L)^g^5(x)^5(x)^t^t^g^5(x)^t^t^g^G(L)^T(L)^G(V) |
| BN-0113 | G(V)^G(L)^T(L)^a^g^5(x)^5(x)^t^t^g^5(x)^t^t^G(L)^G(L)^T(V) |
| BN-0114 | 5(V)^G(L)^G(L)^t^a^g^5(x)^5(x)^t^t^g^5(x)^t^T(L)^G(L)^G(V) |
| BN-0115 | G(V)^5(L)^G(L)^g^t^a^g^5(x)^5(x)^t^t^g^5(x)^T(L)^T(L)^G(V) |
| BN-0116 | 5(V)^T(L)^A(L)^g^g^t^5(x)^t^t^a^5(x)^5(x)^a^5(L)^A(L)^G(V) |
| BN-0117 | 5(V)^T(L)^G(L)^g^g^t^5(x)^5(x)^a^g^g^5(x)^a^T(L)^G(L)^G(V) |
| BN-0118 | A(V)^T(L)^T(L)^5(x)^a^g^t^5(x)^t^g^g^g^t^5(L)^5(L)^A(V) |
| BN-0119 | G(V)^A(L)^T(L)^t^5(x)^a^g^t^5(x)^t^g^g^g^T(L)^5(L)^5(V) |
| BN-0120 | T(V)^G(L)^G(L)^5(x)^5(x)^t^t^a^t^t^5(x)^t^t^G(L)^5(L)^T(V) |
| BN-0121 | 5(V)^A(L)^G(L)^g^g^g^t^g^t^t^g^g^g^G(L)^T(L)^T(V) |

**[Table 4]**

| | |
|---|---|
| BN-0124 | G(L)^T(L)^G(V)^t^t^g^a^a^a^t^t^5(x)^t^G(V)^5(L)^A(L) |
| BN-0125 | G(L)^G(L)^G(V)^a^g^t^5(x)^5(x)^t^t^g^a^g^T(V)^5(L)^T(L) |
| BN-0126 | T(L)^T(L)^5(V)^g^g^a^a^g'g^t^g^a^5(x)^5(V)^T(L)^5(L) |
| BN-0127 | G(L)^G(L)^A(V)^t^t^5(x)^a^g^t^5(x)^t^g^g^G(V)^T(L)^5(L) |
| BN-0128 | 5(L)^G(L)^T(V)^g^t^t^g^a^a^a^t^t^5(x)^T(V)^G(L)^5(L) |
| BN-0129 | G(L)^5(L)^G(V)^t^g^t^t^g^a^a^a^t^t^5(V)^T(L)^G(L) |
| BN-0130 | G(L)^A(L)^G(V)^t^5(x)^5(x)^t^t^g^a^g^t^5(x)^T(V)^G(L)^G(L) |
| BN-0131 | G(L)^G(L)^A(V)^g^t^5(x)^5(x)^t^t^g^a^g^t^5(V)^T(L)^G(L) |
| BN-0132 | T(L)^G(L)^G(V)^g^a^g^t^5(x)^5(x)^t^t^g^a^G(V)^T(L)^5(L) |
| BN-0133 | G(L)^T(L)^T(V)^5(x)^a^g^5(x)^t^t⁻g^g^g^t⁻A(V)^G(L)^G(L) |
| BN-0134 | 5(L)^5(L)^G(V)^t^g^t^a^g^a^a^g^a^g^G(V)^T(L)^5(L) |
| BN-0135 | G(L)^5(L)^A(V)^t^a^a^g^a^a^a^g^5(x)^t^5(V)^A(L)^T(L) |
| BN-0136 | G(L)^G(L)^5(V)^a^t^a^a^g^a^a^a^g^5(x)^T(V)^5(L)^A(L) |
| BN-0137 | 5(L)^G(L)^G(V)^a^a^g^g^t^g^a^5(x)^5(x)^t^5(V)^A(L)^T(L) |
| BN-0138 | T(L)^G(L)^5(V)^t^t^g^g^t^g^a^5(x)^a^5(x)^5(V)^5(L)^5(L) |
| BN-0139 | G(V)^T(L)^G(L)^t^t^g^a^a^a^t^t^5(x)^T(V)^G(L)^5(L)^A(V) |
| BN-0140 | G(V)^G(L)^G(L)^a^g^t^5(x)^5(x)^t^t^g^a^G(V)^T(L)^5(L)^T(V) |
| BN-0141 | T(V)^T(L)^5(L)^g^g^a^a^g^g^t^g^a^5(V)^5(L)^T(L)^5(V) |
| BN-0142 | G(V)^G(L)^A(L)^t^t^5(x)^a^g^t^5(x)^t^g^G(V)^G(L)^T(L)^5(V) |
| BN-0143 | 5(V)^G(L)^T(L)^g^t^t^g^a^a^a^t^t^5(V)^T(L)^G(L)^5(V) |
| BN-0144 | G(V)^5(L)^G(L)^t^g^t^t^g^a^a^a^t^T(V)^5(L)^T(L)^G(V) |
| BN-0145 | G(V)^A(L)^G(L)^t^5(x)^5(x)^t^t^g^a^g^t^5(V)^T(L)^G(L)^G(V) |
| BN-0146 | G(V)^G(L)^A(L)^g^t^5(x)^5(x)^t^t^g^a^g^T(V)^5(L)^T(L)^G(V) |
| BN-0147 | T(V)^G(L)^G(L)^g^a^g^t^5(x)^5(x)^t^t^g^A(V)^G(L)^T(L)^5(V) |
| BN-0148 | G(V)^T(L)^T(L)^5(x)^a^g^5(x)^t^t^g^g^g^T(V)^A(L)^G(L)^G(V) |
| BN-0149 | 5(V)^5(L)^G(L)^t^g^t^a^g^a^a^g^a^G(V)^G(L)^T(L)^5(V) |
| BN-0150 | G(V)^5(L)^A(L)^t^a^a^g^a^a^a^g^5(x)^T(V)^5(L)^A(L)^T(V) |
| BN-01 51 | G(V)^G(L)^5(L)^a^t^a^a^g^a^a^a^g^5(V)^T(L)^5(L)^A(V) |
| BN-0152 | 5(V)^G(L)^G(L)^a^a^g^g^t^g^a^5(x)^5(x)^T(V)^5(L)^A(L)^T(V) |
| BN-0153 | T(V)^G(L)^5(L)^t^t^g^g^t^g^a^5(x)^a^5(V)^5(L)^5(L)^5(V) |
| BN-0154 | G(V)^T(L)^G(L)^T(V)^t^g^a^a^a^t^t^5(x)^t^G(L)^5(L)^A(V) |
| BN-0155 | G(V)^G(L)^G(L)^A(V)^g^t^5(x)^5(x)^t^t^g^a^g^T(L)^5(L)^T(V) |
| BN-0156 | T(V)^T(L)^5(L)^G(V)^g^a^a^g^g^t^g^a^5(x)^5(L)^T(L)^5(V) |
| BN-0157 | G(V)^G(L)^A(L)^T(V)^t^5(x)^a^g^t^5(x)^t^g^g^G(L)^T(L)^5(V) |
| BN-0158 | 5(V)^G(L)^T(L)^G(V)^t^t^g^a^a^a^t^t^5(x)^T(L)^G(L)^5(V) |
| BN-0159 | G(V)^5(L)^G(L)^T(V)^g^t^t^g^a^a^a^t^t^5(L)^T(L)^G(V) |
| BN-0160 | G(V)^A(L)^G(L)^T(V)^5(x)^5(x)^t^t^g^a^g^t^5(x)^T(L)^G(L)^G(V) |
| BN-0161 | G(V)^G(L)^A(L)^G(V)^t^5(x)^5(x)^t^t^g^a^g^t^5(L)^T(L)^G(V) |
| BN-0162 | T(V)^G(L)^G(L)^G(V)^a^g^t^5(x)^5(x)^t^t^g^a^G(L)^T(L)^5(V) |
| BN-0163 | G(V)^T(L)^T(L)^5(V)^a^g^5(x)^t^t^g^g^g^t^A(L)^G(L)^G(V) |
| BN-0164 | 5(V)^5(L)^G(L)^T(V)^g^t^a^g^a^a^g^a^g^G(L)^T(L)^5(V) |
| BN-0165 | G(V)^5(L)^A(L)^T(V)^a^a^g^a^a^a^g^5(x)^t^5(L)^A(L)^T(V) |
| BN-0166 | G(V)^G(L)^5(L)^A(V)^t^a^a^g^a^a^a^g^5(x)^T(L)^5(L)^A(V) |
| BN-0167 | 5(V)^G(L)^G(L)^A(V)^a^g^g^t^g^a^5(x)^5(x)^t^5(L)^A(L)^T(V) |
| BN-0168 | T(V)^G(L)^5(L)^T(V)^t^g^g^t^g^a^5(x)^a^5(x)^5(L)^5(L)^5(V) |
| BN-0169 | G(V)^T(L)^G(L)^T(V)^T(V)^g^a^a^a^t^t^5(x)^t^G(L)^5(L)^A(V) |
| BN-0170 | G(V)⁻G(L)^G(L)^A(V)^G(V)^t^5(x)^5(x)^t^t^g^a^g^T(L)^5(L)^T(V) |
| BN-01 71 | T(V)^T(L)^5(L)^G(V)^G(V)^a^a^g^g^t^g^a^5(x)^5(L)^T(L)^5(V) |
| BN-0172 | G(V)^G(L)^A(L)^T(V)^T(V)^5(x)^a^g^t^5(x)^t^g^g^G(L)^T(L)^5(V) |
| BN-0173 | 5(V)^G(L)^T(L)^G(V)^T(V)^t^g^a^a a^t^t^5(x)^T(L)^G(L)^5(V) |

**[Table 5]**

| | |
|---|---|
| BN-0174 | G(V)^5(L)^G(L)^T(V)^G(V)^t^t^g^a^a^a^t^t^5(L)^T(L)^G(V) |
| BN-0175 | G(V)^A(L)^G(L)^T(V)^5(V)^5(x)^t^t^g^a^g^t^5(x)^T(L)^G(L)^G(V) |
| BN-0176 | G(V)^G(L)^A(L)^G(V)^T(V)^5(x)^5(x)^t^t^g^a^g^t^5(L)^T(L)^G(V) |
| BN-0177 | T(V)^G(L)^G(L)^G(V)^A(V)^g^t^5(x)^5(x)^t^t^g^a^G(L)^T(L)^5(V) |
| BN-0178 | G(V)^T(L)^T(L)^5(V)^A(V)^g^5(x)^t^t^g^g^g^t^A(L)^G(L)^G(V) |
| BN-0179 | 5(V)^5(L)^G(L)^T(V)^G(V)^t^a^g^a^a^g^a^g^G(L)^T(L)^5(V) |
| BN-0180 | G(V)^5(L)^A(L)^T(V)^A(V)^a^g^a^a^a^ ^5(x)^t^5(L)^A(L)^T(V) |
| BN-0181 | G(V)^G(L)^5(L)^A(V)^T(V)^a^a^g^a^a^a^g^5(x)^T(L)^5(L)^A(V) |
| BN-0182 | 5(V)^G(L)^G(L)^A(V)^A(V)^g^g^t^g^a^5(x)^5(x)^t^5(L)^A(L)^T(V) |
| BN-0183 | T(V)^G(L)^5(L)^T(V)^T(V)^g^g^t^g^a^5(x)^a^5(x)^5(L)^5(L)^5(V) |
| BN-0184 | G(V)^T(L)G(L)t^t^g^a^a^a^t^t^5(x)^t^G(L)5(L)^A(V) |
| BN-0185 | G(V)^G(L)G(L)a^g^t^5(x)^5(x)^t^t^g^a^g^T(L)5(L)^T(V) |
| BN-0186 | T(V)^T(L)5(L)g^g^a^a^g^g^t^g^a^5(x)^5(L)T(L)^5(V) |
| BN-0187 | G(V)^G(L)A(L)t^t^5(x)^a^g^t^5(x)^t^g^g^G(L)T(L)^5(V) |
| BN-0188 | 5(V)^G(L)T(L)g^t^t^g^a^a^a^t^t^5(x)^T(L)G(L)^5(V) |
| BN-0189 | G(V)^5(L)G(L)t^g^t^t^g^a^a^a^t^t^5(L)T(L)^G(V) |
| BN-0190 | G(V)^A(L)G(L)t^5(x)^5(x)^t^t^g^a^g^t^5(x)^T(L)G(L)^G(V) |
| BN-0191 | G(V)^G(L)A(L)g^t^5(x)^5(x)^t^t^g^a^g^t^5(L)T(L)^G(V) |
| BN-0192 | T(V)^G(L)G(L)g^a^g^t^5(x)^5(x)^t^t^g^a^G(L)T(L)^5(V) |
| BN-0193 | G(V)^T(L)T(L)5(×)^a^g^5(×)^t^t^g^g^g^t^A(L)G(L)^G(V) |
| BN-0194 | 5(V)^5(L)G(L)t^g^t^a^g^a^a^g^a^g^G(L)T(L)^5(V) |
| BN-0195 | G(V)^5(L)A(L)t^a^a^g^a^a^a^g^5(x)^t^5(L)A(L)^T(V) |
| BN-0196 | G(V)^G(L)5(L)a^t^a^a^g^a^a^a^e^5(x)^T(L)5(L)^A(V) |
| BN-0197 | 5(V)^G(L)G(L)a^a^g^g^t^g^a^5(x)^5(x)^t^5(L)A(L)^T(V) |
| BN-0198 | T(V)^G(L)5(L)t^t^g^g^t^g^a^5(x)^a^5(x)^5(L)5(L)^5(V) |
| BN-0199 | G(V)^T(L)^G(L)^T(Mo)^t^g^a^a^a^t^t^5(x)^t^G(L)^5(L)^A(V) |
| BN-0200 | G(V)^G(L)^G(L)^A(Mo)^g^t^5(x)^5(x)^t^t^g^a^g^T(L)^5(L)^T(V) |
| BN-0201 | T(V)^T(L)^5(L)^G(Mo)^g^a^a^g^g^t^g^a^5(x)^5(L)^T(L)^5(V) |
| BN-0202 | G(V)^G(L)^A(L)^T(Mo)^t^5(x)^a^g^t^5(x)^t^g^g^G(L)^T(L)^5(V) |
| BN-0203 | 5(V)^G(L)^T(L)^G(Mo)^t^t^g^a^a a^t^t^5(x)^T(L)^G(L)^5(V) |
| BN-0204 | G(V)^5(L)^G(L)^T(Mo)^g^t^t^g^a^a^a^t^t^5(L)^T(L)^G(V) |
| BN-0205 | G(V)^A(L)^G(L)^T(Mo)^5(x)^5(x)^t^t^g^a^g^t^5(x)^T(L)^G(L)^G(V) |
| BN-0206 | G(V)^G(L)^A(L)^G(Mo)^t^5(x)^5(x)^t^t^g^a^g^t^5(L)^T(L)^G(V) |
| BN-0207 | T(v)^G(L)^G(L)^G(Mo)^a^g^t^5(x)^5(x)^t^t^g^a^G(L)^T(L)^5(V) |
| BN-0208 | G(V)^T(L)^T(L)^5(Mo)^a^g^5(x)^t^t^g^g^g^t^A(L)^G(L)^G(V) |
| BN-0209 | 5(V)^5(L)^G(L)^T(Mo)^g^t^a^g^a^a^g^a^g^G(L)^T(L)^5(V) |
| BN-0210 | G(V)^5(L)^A(L)^T(Mo)^a^a^g^a^a^a^g^5(x)^t^5(L)^A(L)^T(V) |
| BN-0211 | G(V)^G(L)^5(L)^A(Mo)^t^a^a^g^a^a^a^g^5(x)^T(L)^5(L)^A(V) |
| BN-0212 | 5(v)^G(L)^G(L)^A(Mo)^a^g^g^t^g^a^5(x)^5(x)^t^5(L)^A(L)^T(V) |
| BN-0213 | T(V)^G(L)^5(L)^T(Mo)^t^g^g^t^g^a^5(x)^a^5(x)^5(L)^5(L)^5(V) |
| BN-0214 | G(V)^T(L)^G(L)^T(Mo)^T(Mo)^g^a^a^a^t^t^5(x)^t^G(L)^5(L)^A(V) |
| BN-0215 | G(V)^G(L)^G(L)^A(Mo)^G(Mo)^t^5(x)^5(x)^t^t^g^a^g^T(L)^5(L)^T(V) |
| BN-0216 | T(V)^T(L)^5(L)^G(Mo)^G(Mo)^a^a^g^g^t^g^a^5(x)^5(L)^T(L)^5(V) |
| BN-0217 | G(V)^G(L)^A(L)^T(Mo)^T(Mo)^5(x)^a^g^t^5(x)^t^q^g^G(L)^T(L)^5(V) |
| BN-0218 | 5(V)^G(L)^T(L)^G(Mo)^T(Mo)^t^g^a^a^a^t^t^5(x)^T(L)^G(L)^5(V) |
| BN-0219 | G(V)^5(L)^G(L)^T(Mo)^G(Mo)^t^t^g^a^a^a^t^t^5(L)^T(L)^G(V) |
| BN-0220 | G(V)^A(L)^G(L)^T(Mo)^5(Mo)^5(x)^t^t^g^a^g^t^5(x)^T(L)^G(L)^G(V) |
| BN-0221 | G(V)^G(L)^A(L)^G(Mo)^T(Mo)^5(x)^5(x)^t^t^g^a^g^t^5(L)^T(L)^G(V) |
| BN-0222 | T(V)^G(L)^G(L)^G(Mo)^A(Mo)^g^t^5(×)^5(×)^t^t^g^a^G(L)^T(L)^5(V) |
| BN-0223 | G(V)^T(L)^T(L)^5(Mo)^A(Mo)^g^5(x)^t^t^g^g^g^t^A(L)^G(L)^G(V) |
| BN-0224 | 5(V)^5(L)^G(L)^T(Mo)^G(Mo)^t^a^g^a^a^g^a^g^G(L)^T(L)^5(V) |
| BN-0225 | G(V)^5(L)^A(L)^T(Mo)^A(Mo)^a^g^a^a^a^g^5(x)^t^5(L)^A(L)^T(V) |
| BN-0226 | G(V)^G(L)^5(L)^A(Mo)^T(Mo)^a^a^g^a^a^a^g^5(x)^T(L)^5(L)^A(V) |
| BN-0227 | 5(V)^G(L)^G(L)^A(Mo)^A(Mo)^g^g^t^g^a^5(x)^5(x)^t^5(L)^A(L)^T(V) |
| BN-0228 | T(V)^G(L)^5(L)^T(Mo)^T(Mo)^g^g^t^g^a^5(x)^a^5(x)^5(L)^5(L)^5(V) |

Positions in a sequence of SEQ ID NO: 1 being human RasGRP4 mRNA and/or SEQ ID NO: 2 being human RasGRP4 pre-mRNA targeted by the antisense oligonucleotides shown in Tables 1 to 5, and sequence numbers and nucleobase sequences corresponding to the antisense oligonucleotides are shown in Tables 6 to 10.

In Tables 6 to 10, the "SEQ1 START" means "SEQ ID NO: 1 start site" and represents the position number of a nucleoside on a side closest to a 5' side targeted by an antisense oligonucleotide in SEQ ID NO: 1. The "SEQ1 END" means "SEQ ID NO: 1 termination site" and represents the position number of a nucleoside on a side closest to a 3' side targeted by an antisense oligonucleotide in SEQ ID NO: 1.

The "SEQ2 START" means "SEQ ID NO: 2 start site" and represents the position number of a nucleoside on a side closest to a 5' side targeted by an antisense oligonucleotide in SEQ ID NO: 2. The "SEQ2 END" means "SEQ ID NO: 2 termination site" and represents the position number of a nucleoside on a side closest to a 3' side targeted by an antisense oligonucleotide in SEQ ID NO: 2.

Each antisense oligonucleotide is targeted to either of human RasGRP4 mRNA represented by SEQ ID NO: 1 and/or human RasGRP4 pre-mRNA represented by SEQ ID NO: 2.

In Tables 6 to 10, "-" (hyphen) indicates that the antisense oligonucleotide does not target human RasGRP4 mRNA represented by SEQ ID NO: 1 or human RasGRP4 pre-mRNA represented by SEQ ID NO: 2 with 100% complementarity. The "BASE SEQUENCE" represents a nucleobase sequence of an antisense oligonucleotide.

The "SEQ No. of BASE SEQ" represents a sequence number corresponding to a nucleobase sequence of an antisense oligonucleotide. The "SEQ No. of compd." represents a sequence number unique to each antisense oligonucleotide.

**[Table 6]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0001 | 124 | 216 | 231 | 4817 | 4832 | TGGGGCATGTCTTGTG | 3 |
| BN-0002 | 125 | 237 | 252 | 4838 | 4853 | CCTTGCTGATTTCCCG | 4 |
| BN-0003 | 126 | 501 | 516 | - | - | TCAGCCAGTACCTGAC | 5 |
| BN-0004 | 127 | 683 | 698 | 6938 | 6953 | CTTTTTGCCCAGGCCT | 6 |
| BN-0005 | 128 | 753 | 768 | 7008 | 7023 | ACTCCAGGTAGGTGAG | 7 |
| BN-0006 | 129 | 774 | 789 | 7029 | 7044 | TGATAGCCTGGAAGGA | 8 |
| BN-0007 | 130 | 891 | 906 | 8441 | 8456 | TCAGCACCATCACCTG | 9 |
| BN-0008 | 131 | 939 | 954 | 8489 | 8504 | GAATGAACTTGTCCAG | 10 |
| BN-0009 | 132 | 974 | 989 | 9746 | 9761 | GAAATTCTGCAGCTGG | 11 |
| BN-0010 | 133 | 979 | 994 | 9751 | 9766 | GTGTTGAAATTCTGCA | 12 |
| BN-0011 | 134 | 1033 | 1048 | 9805 | 9820 | TCCTTGAGTCTGGAGA | 13 |
| BN-0012 | 135 | 1038 | 1053 | 9810 | 9825 | GGGAGTCCTTGAGTCT | 14 |
| BN-0013 | 136 | 1114 | 1129 | 11814 | 11829 | GCGTAGTTGTTGTGGG | 15 |
| BN-0014 | 137 | 1186 | 1201 | 11886 | 11901 | AGGTCCTTGAGGTGCA | 16 |
| BN-0015 | 138 | 1252 | 1267 | 11952 | 11967 | TTCAGCTTGGGTAGGT | 17 |
| BN-0016 | 139 | 1332 | 1347 | 12032 | 12047 | GCAGCAGATCCTCATT | 18 |
| BN-0017 | 140 | 1365 | 1380 | 13438 | 13453 | TGTAGAAGAGGTCCAG | 19 |
| BN-0018 | 141 | 1369 | 1384 | 13442 | 13457 | TCCGTGTAGAAGAGGT | 20 |
| BN-0019 | 142 | 1400 | 1415 | 13473 | 13488 | CCGGGCATAAGAAAGC | 21 |
| BN-0020 | 143 | 1446 | 1461 | 13615 | 13630 | GTGCATTGAAGGGGGA | 22 |
| BN-0021 | 144 | 1525 | 1540 | 13694 | 13709 | ACCAGCTGCTCCACAT | 23 |
| BN-0022 | 145 | 1538 | 1553 | - | - | GAACACAGACTCCACC | 24 |
| BN-0023 | 146 | 1591 | 1606 | 13903 | 13918 | CGCTCAAAGTCCTCCT | 25 |
| BN-0024 | 147 | 1668 | 1683 | 14094 | 14109 | CTCTGCTGAAGGATCC | 26 |
| BN-0025 | 148 | 1719 | 1734 | 14145 | 14160 | CCAACTTGGAGCAGAT | 27 |
| BN-0026 | 149 | 1761 | 1776 | 14187 | 14202 | TTCGGAAGGTGACCTC | 28 |
| BN-0027 | 150 | 1818 | 1833 | 15534 | 15549 | AGCCTTGCTTGGTGAC | 29 |
| BN-0028 | 151 | 1894 | 1909 | 15705 | 15720 | GGCCTCTTCTTACATT | 30 |
| BN-0029 | 152 | 2051 | 2066 | 15978 | 15993 | GGATTCAGTCTGGGTC | 31 |
| BN-0030 | 153 | - | - | 640 | 655 | GGGGGAAGGAAGAGGC | 32 |
| BN-0031 | 154 | - | - | 679 | 694 | GGAAAGAGGAACTGCC | 33 |
| BN-0032 | 155 | - | - | 967 | 982 | CTCCCTGAAGCATCCA | 34 |
| BN-0033 | 156 | - | - | 1037 | 1052 | AGCTCAAACTCCAGGG | 35 |
| BN-0034 | 157 | - | - | 1231 | 1246 | AGGAGTGGTTTAAGAG | 36 |
| BN-0035 | 158 | - | - | 1498 | 1513 | AGTGACTTACCTAAGG | 37 |
| BN-0036 | 159 | 244 | 259 | 4845 | 4860 | GCCATGACCTTGCTGA | 38 |
| BN-0037 | 160 | - | - | 5575 | 5590 | CATGACCTTGGACAGG | 39 |
| BN-0038 | 161 | - | - | 5802 | 5817 | GGATATGAGGTCAGCA | 40 |
| BN-0039 | 162 | - | - | 5918 | 5933 | GAGTTCTGGTCAAGGC | 41 |
| BN-0040 | 163 | - | - | 6395 | 6410 | TTGGAAGTGGGATTAT | 42 |
| BN-0041 | 164 | - | - | 6544 | 6559 | GTGCTGAGCTAGGGGG | 43 |
| BN-0042 | 165 | - | - | 6639 | 6654 | TCAGCCAGTACCTGAG | 44 |
| BN-0043 | 166 | - | - | 6771 | 6786 | GGCTGACTCACAGGTC | 45 |
| BN-0044 | 167 | 666 | 681 | 6921 | 6936 | GGCTGCTCATTGGGAG | 46 |
| BN-0045 | 168 | - | - | 7033 | 7048 | ACCGTGATAGCCTGGA | 47 |
| BN-0046 | 169 | - | - | 7206 | 7221 | TGGCCTCTTTAACTCA | 48 |
| BN-0047 | 170 | 889 | 904 | 8439 | 8454 | AGCACCATCACCTGCA | 49 |
| BN-0048 | 171 | - | - | 9361 | 9376 | CTCTGAGAGTCACACA | 50 |
| BN-0049 | 172 | - | - | 9439 | 9454 | TTGGGGAATAGACAGG | 51 |
| BN-0050 | 173 | - | - | 9632 | 9647 | AGGCATGTGTGGACCA | 52 |

**[Table 7]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0051 | 174 | 964 | 979 | 9736 | 9751 | AGCTGGTGGAGCCTCT | 53 |
| BN-0052 | 175 | 968 | 983 | 9740 | 9755 | CTGCAGCTGGTGGAGC | 54 |
| BN-0053 | 176 | 969 | 984 | 9741 | 9756 | TCTGCAGCTGGTGGAG | 55 |
| BN-0054 | 177 | 970 | 985 | 9742 | 9757 | TTCTGCAGCTGGTGGA | 56 |
| BN-0055 | 178 | 971 | 986 | 9743 | 9758 | ATTCTGCAGCTGGTGG | 57 |
| BN-0056 | 179 | 972 | 987 | 9744 | 9759 | AATTCTGCAGCTGGTG | 58 |
| BN-0057 | 180 | 976 | 991 | 9748 | 9763 | TTGAAATTCTGCAGCT | 59 |
| BN-0058 | 181 | 977 | 992 | 9749 | 9764 | GTTGAAATTCTGCAGC | 60 |
| BN-0059 | 182 | 978 | 993 | 9750 | 9765 | TGTTGAAATTCTGCAG | 61 |
| BN-0060 | 183 | 980 | 995 | 9752 | 9767 | CGTGTTGAAATTCTGC | 62 |
| BN-0061 | 184 | 981 | 996 | 9753 | 9768 | GCGTGTTGAAATTCTG | 63 |
| BN-0062 | 185 | 982 | 997 | 9754 | 9769 | AGCGTGTTGAAATTCT | 64 |
| BN-0063 | 186 | 1036 | 1051 | 9808 | 9823 | GAGTCCTTGAGTCTGG | 65 |
| BN-0064 | 187 | 1037 | 1052 | 9809 | 9824 | GGAGTCCTTGAGTCTG | 66 |
| BN-0065 | 188 | 1039 | 1054 | 9811 | 9826 | TGGGAGTCCTTGAGTC | 67 |
| BN-0066 | 189 | 1040 | 1055 | 9812 | 9827 | ATGGGAGTCCTTGAGT | 68 |
| BN-0067 | 190 | 1041 | 1056 | 9813 | 9828 | CATGGGAGTCCTTGAG | 69 |
| BN-0068 | 191 | - | - | 9870 | 9885 | TTGGAGCTTTGGGGAG | 70 |
| BN-0069 | 192 | 1245 | 1260 | 11945 | 11960 | TGGGTAGGTGCAGGCG | 71 |
| BN-0070 | 193 | 1246 | 1261 | 11946 | 11961 | TTGGGTAGGTGCAGGC | 72 |
| BN-0071 | 194 | 1247 | 1262 | 11947 | 11962 | CTTGGGTAGGTGCAGG | 73 |
| BN-0072 | 195 | 1248 | 1263 | 11948 | 11963 | GCTTGGGTAGGTGCAG | 74 |
| BN-0073 | 196 | 1249 | 1264 | 11949 | 11964 | AGCTTGGGTAGGTGCA | 75 |
| BN-0074 | 197 | 1250 | 1265 | 11950 | 11965 | CAGCTTGGGTAGGTGC | 76 |
| BN-0075 | 198 | 1251 | 1266 | 11951 | 11966 | TCAGCTTGGGTAGGTG | 77 |
| BN-0076 | 199 | 1253 | 1268 | 11953 | 11968 | GTTCAGCTTGGGTAGG | 78 |
| BN-0077 | 200 | 1368 | 1383 | 13441 | 13456 | CCGTGTAGAAGAGGTC | 79 |
| BN-0078 | 201 | 1395 | 1410 | 13468 | 13483 | CATAAGAAAGCTCATA | 80 |
| BN-0079 | 202 | 1396 | 1411 | 13469 | 13484 | GCATAAGAAAGCTCAT | 81 |
| BN-0080 | 203 | 1397 | 1412 | 13470 | 13485 | GGCATAAGAAAGCTCA | 82 |
| BN-0081 | 204 | 1398 | 1413 | 13471 | 13486 | GGGCATAAGAAAGCTC | 83 |
| BN-0082 | 205 | 1399 | 1414 | 13472 | 13487 | CGGGCATAAGAAAGCT | 84 |
| BN-0083 | 206 | 1401 | 1416 | 13474 | 13489 | CCCGGGCATAAGAAAG | 85 |
| BN-0084 | 207 | 1523 | 1538 | 13692 | 13707 | CAGCTGCTCCACATGC | 86 |
| BN-0085 | 208 | 1526 | 1541 | 13695 | 13710 | CACCAGCTGCTCCACA | 87 |
| BN-0086 | 209 | 1527 | 1542 | 13696 | 13711 | CCACCAGCTGCTCCAC | 88 |
| BN-0087 | 210 | 1528 | 1543 | 13697 | 13712 | TCCACCAGCTGCTCCA | 89 |
| BN-0088 | 211 | - | - | 13850 | 13865 | GAACACAGACTTCAGA | 90 |
| BN-0089 | 212 | 1586 | 1601 | 13898 | 13913 | AAAGTCCTCCTGAGAG | 91 |
| BN-0090 | 213 | 1587 | 1602 | 13899 | 13914 | CAAAGTCCTCCTGAGA | 92 |
| BN-0091 | 214 | 1588 | 1603 | 13900 | 13915 | TCAAAGTCCTCCTGAG | 93 |
| BN-0092 | 215 | 1589 | 1604 | 13901 | 13916 | CTCAAAGTCCTCCTGA | 94 |
| BN-0093 | 216 | 1590 | 1605 | 13902 | 13917 | GCTCAAAGTCCTCCTG | 95 |
| BN-0094 | 217 | 1592 | 1607 | 13904 | 13919 | TCGCTCAAAGTCCTCC | 96 |
| BN-0095 | 218 | 1593 | 1608 | 13905 | 13920 | GTCGCTCAAAGTCCTC | 97 |
| BN-0096 | 219 | 1670 | 1685 | 14096 | 14111 | CTCTCTGCTGAAGGAT | 98 |
| BN-0097 | 220 | 1744 | 1759 | 14170 | 14185 | TGGAAGGTGTGCAGGA | 99 |
| BN-0098 | 221 | 1753 | 1768 | 14179 | 14194 | GTGACCTCATGGAAGG | 100 |
| BN-0099 | 222 | 1754 | 1769 | 14180 | 14195 | GGTGACCTCATGGAAG | 101 |
| BN-0100 | 223 | 1756 | 1771 | 14182 | 14197 | AAGGTGACCTCATGGA | 102 |

**[Table 8]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0101 | 224 | 1757 | 1772 | 14183 | 14198 | GAAGGTGACCTCATGG | 103 |
| BN-0102 | 225 | 1758 | 1773 | 14184 | 14199 | GGAAGGTGACCTCATG | 104 |
| BN-0103 | 226 | 1759 | 1774 | 14185 | 14200 | CGGAAGGTGACCTCAT | 105 |
| BN-0104 | 227 | 1760 | 1775 | 14186 | 14201 | TCGGAAGGTGACCTCA | 106 |
| BN-0105 | 228 | - | - | 14868 | 14883 | TAGAGATTTGGAGGAT | 107 |
| BN-0106 | 229 | 1813 | 1828 | 15529 | 15544 | TGCTTGGTGACACCCC | 108 |
| BN-0107 | 230 | 1814 | 1829 | 15530 | 15545 | TTGCTTGGTGACACCC | 109 |
| BN-0108 | 231 | 1815 | 1830 | 15531 | 15546 | CTTGCTTGGTGACACC | 110 |
| BN-0109 | 232 | 1816 | 1831 | 15532 | 15547 | CCTTGCTTGGTGACAC | 111 |
| BN-0110 | 233 | 1817 | 1832 | 15533 | 15548 | GCCTTGCTTGGTGACA | 112 |
| BN-0111 | 234 | 1819 | 1834 | 15535 | 15550 | TAGCCTTGCTTGGTGA | 113 |
| BN-0112 | 235 | 1820 | 1835 | 15536 | 15551 | GTAGCCTTGCTTGGTG | 114 |
| BN-0113 | 236 | 1821 | 1836 | 15537 | 15552 | GGTAGCCTTGCTTGGT | 115 |
| BN-0114 | 237 | 1822 | 1837 | 15538 | 15553 | CGGTAGCCTTGCTTGG | 116 |
| BN-0115 | 238 | 1823 | 1838 | 15539 | 15554 | GCGGTAGCCTTGCTTG | 117 |
| BN-0116 | 239 | 2042 | 2057 | 15787 | 15802 | CTAGGTCTTACCACAG | 118 |
| BN-0117 | 240 | 2049 | 2064 | 15969 | 15984 | CTGGGTCCAGGCATGG | 119 |
| BN-0118 | 241 | 2050 | 2065 | 15976 | 15991 | ATTCAGTCTGGGTCCA | 120 |
| BN-0119 | 242 | - | - | 15977 | 15992 | GATTCAGTCTGGGTCC | 121 |
| BN-0120 | 243 | - | - | 16578 | 16593 | TGGCCTTATTCTTGCT | 122 |
| BN-0121 | 244 | 3059 | 3074 | 17775 | 17790 | CAGGGGTGTTGGGGTT | 123 |

**[Table 9]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0124 | 245 | 979 | 994 | 9751 | 9766 | GTGTTGAAATTCTGCA | 12 |
| BN-0125 | 246 | 1038 | 1053 | 9810 | 9825 | GGGAGTCCTTGAGTCT | 14 |
| BN-0126 | 247 | 1761 | 1776 | 14187 | 14202 | TTCGGAAGGTGACCTC | 28 |
| BN-0127 | 248 | 2051 | 2066 | 15978 | 15993 | GGATTCAGTCTGGGTC | 31 |
| BN-0128 | 249 | 980 | 995 | 9752 | 9767 | CGTGTTGAAATTCTGC | 62 |
| BN-0129 | 250 | 981 | 996 | 9753 | 9768 | GCGTGTTGAAATTCTG | 63 |
| BN-0130 | 251 | 1036 | 1051 | 9808 | 9823 | GAGTCCTTGAGTCTGG | 65 |
| BN-0131 | 252 | 1037 | 1052 | 9809 | 9824 | GGAGTCCTTGAGTCTG | 66 |
| BN-0132 | 253 | 1039 | 1054 | 9811 | 9826 | TGGGAGTCCTTGAGTC | 67 |
| BN-0133 | 254 | 1253 | 1268 | 11953 | 11968 | GTTCAGCTTGGGTAGG | 78 |
| BN-0134 | 255 | 1368 | 1383 | 13441 | 13456 | CCGTGTAGAAGAGGTC | 79 |
| BN-0135 | 256 | 1396 | 1411 | 13469 | 13484 | GCATAAGAAAGCTCAT | 81 |
| BN-0136 | 257 | 1397 | 1412 | 13470 | 13485 | GGCATAAGAAAGCTCA | 82 |
| BN-0137 | 258 | 1759 | 1774 | 14185 | 14200 | CGGAAGGTGACCTCAT | 105 |
| BN-0138 | 259 | 1813 | 1828 | 15529 | 15544 | TGCTTGGTGACACCCC | 108 |
| BN-0139 | 260 | 979 | 994 | 9751 | 9766 | GTGTTGAAATTCTGCA | 12 |
| BN-0140 | 261 | 1038 | 1053 | 9810 | 9825 | GGGAGTCCTTGAGTCT | 14 |
| BN-0141 | 262 | 1761 | 1776 | 14187 | 14202 | TTCGGAAGGTGACCTC | 28 |
| BN-0142 | 263 | 2051 | 2066 | 15978 | 15993 | GGATTCAGTCTGGGTC | 31 |
| BN-0143 | 264 | 980 | 995 | 9752 | 9767 | CGTGTTGAAATTCTGC | 62 |
| BN-0144 | 265 | 981 | 996 | 9753 | 9768 | GCGTGTTGAAATTCTG | 63 |
| BN-0145 | 266 | 1036 | 1051 | 9808 | 9823 | GAGTCCTTGAGTCTGG | 65 |
| BN-01 46 | 267 | 1037 | 1052 | 9809 | 9824 | GGAGTCCTTGAGTCTG | 66 |
| BN-0147 | 268 | 1039 | 1054 | 9811 | 9826 | TGGGAGTCCTTGAGTC | 67 |
| BN-0148 | 269 | 1253 | 1268 | 11953 | 11968 | GTTCAGCTTGGGTAGG | 78 |
| BN-0149 | 270 | 1368 | 1383 | 13441 | 13456 | CCGTGTAGAAGAGGTC | 79 |
| BN-0150 | 271 | 1396 | 1411 | 13469 | 13484 | GCATAAGAAAGCTCAT | 81 |
| BN-0151 | 272 | 1397 | 1412 | 13470 | 13485 | GGCATAAGAAAGCTCA | 82 |
| BN-0152 | 273 | 1759 | 1774 | 14185 | 14200 | CGGAAGGTGACCTCAT | 105 |
| BN-0153 | 274 | 1813 | 1828 | 15529 | 15544 | TGCTTGGTGACACCCC | 108 |
| BN-0154 | 275 | 979 | 994 | 9751 | 9766 | GTGTTGAAATTCTGCA | 12 |
| BN-0155 | 276 | 1038 | 1053 | 9810 | 9825 | GGGAGTCCTTGAGTCT | 14 |
| BN-0156 | 277 | 1761 | 1776 | 14187 | 14202 | TTCGGAAGGTGACCTC | 28 |
| BN-0157 | 278 | 2051 | 2066 | 15978 | 15993 | GGATTCAGTCTGGGTC | 31 |
| BN-0158 | 279 | 980 | 995 | 9752 | 9767 | CGTGTTGAAATTCTGC | 62 |
| BN-0159 | 280 | 981 | 996 | 9753 | 9768 | GCGTGTTGAAATTCTG | 63 |
| BN-0160 | 281 | 1036 | 1051 | 9808 | 9823 | GAGTCCTTGAGTCTGG | 65 |
| BN-0161 | 282 | 1037 | 1052 | 9809 | 9824 | GGAGTCCTTGAGTCTG | 66 |
| BN-0162 | 283 | 1039 | 1054 | 9811 | 9826 | TGGGAGTCCTTGAGTC | 67 |
| BN-0163 | 284 | 1253 | 1268 | 11953 | 11968 | GTTCAGCTTGGGTAGG | 78 |
| BN-0164 | 285 | 1368 | 1383 | 13441 | 13456 | CCGTGTAGAAGAGGTC | 79 |
| BN-0165 | 286 | 1396 | 1411 | 13469 | 13484 | GCATAAGAAAGCTCAT | 81 |
| BN-0166 | 287 | 1397 | 1412 | 13470 | 13485 | GGCATAAGAAAGCTCA | 82 |
| BN-0167 | 288 | 1759 | 1774 | 14185 | 14200 | CGGAAGGTGACCTCAT | 105 |
| BN-0168 | 289 | 1813 | 1828 | 15529 | 15544 | TGCTTGGTGACACCCC | 108 |
| BN-0169 | 290 | 979 | 994 | 9751 | 9766 | GTGTTGAAATTCTGCA | 12 |
| BN-0170 | 291 | 1038 | 1053 | 9810 | 9825 | GGGAGTCCTTGAGTCT | 14 |
| BN-0171 | 292 | 1761 | 1776 | 14187 | 14202 | TTCGGAAGGTGACCTC | 28 |
| BN-0172 | 293 | 2051 | 2066 | 15978 | 15993 | GGATTCAGTCTGGGTC | 31 |
| BN-0173 | 294 | 980 | 995 | 9752 | 9767 | CGTGTTGAAATTCTGC | 62 |

**[Table 10]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0174 | 295 | 981 | 996 | 9753 | 9768 | GCGTGTTGAAATTCTG | 63 |
| BN-0175 | 296 | 1036 | 1051 | 9808 | 9823 | GAGTCCTTGAGTCTGG | 65 |
| BN-0176 | 297 | 1037 | 1052 | 9809 | 9824 | GGAGTCCTTGAGTCTG | 66 |
| BN-0177 | 298 | 1039 | 1054 | 9811 | 9826 | TGGGAGTCCTTGAGTC | 67 |
| BN-0178 | 299 | 1253 | 1268 | 11953 | 11968 | GTTCAGCTTGGGTAGG | 78 |
| BN-0179 | 300 | 1368 | 1383 | 13441 | 13456 | CCGTGTAGAAGAGGTC | 79 |
| BN-0180 | 301 | 1396 | 1411 | 13469 | 13484 | GCATAAGAAAGCTCAT | 81 |
| BN-0181 | 302 | 1397 | 1412 | 13470 | 13485 | GGCATAAGAAAGCTCA | 82 |
| BN-01 82 | 303 | 1759 | 1774 | 14185 | 14200 | CGGAAGGTGACCTCAT | 105 |
| BN-01 83 | 304 | 1813 | 1828 | 15529 | 15544 | TGCTTGGTGACACCCC | 108 |
| BN-01 84 | 305 | 979 | 994 | 9751 | 9766 | GTGTTGAAATTCTGCA | 12 |
| BN-0185 | 306 | 1038 | 1053 | 9810 | 9825 | GGGAGTCCTTGAGTCT | 14 |
| BN-0186 | 307 | 1761 | 1776 | 14187 | 14202 | TTCGGAAGGTGACCTC | 28 |
| BN-0187 | 308 | 2051 | 2066 | 15978 | 15993 | GGATTCAGTCTGGGTC | 31 |
| BN-0188 | 309 | 980 | 995 | 9752 | 9767 | CGTGTTGAAATTCTGC | 62 |
| BN-0189 | 310 | 981 | 996 | 9753 | 9768 | GCGTGTTGAAATTCTG | 63 |
| BN-0190 | 311 | 1036 | 1051 | 9808 | 9823 | GAGTCCTTGAGTCTGG | 65 |
| BN-0191 | 312 | 1037 | 1052 | 9809 | 9824 | GGAGTCCTTGAGTCTG | 66 |
| BN-0192 | 313 | 1039 | 1054 | 9811 | 9826 | TGGGAGTCCTTGAGTC | 67 |
| BN-0193 | 314 | 1253 | 1268 | 11953 | 11968 | GTTCAGCTTGGGTAGG | 78 |
| BN-0194 | 315 | 1368 | 1383 | 13441 | 13456 | CCGTGTAGAAGAGGTC | 79 |
| BN-0195 | 316 | 1396 | 1411 | 13469 | 13484 | GCATAAGAAAGCTCAT | 81 |
| BN-0196 | 317 | 1397 | 1412 | 13470 | 13485 | GGCATAAGAAAGCTCA | 82 |
| BN-0197 | 318 | 1759 | 1774 | 14185 | 14200 | CGGAAGGTGACCTCAT | 105 |
| BN-0198 | 319 | 1813 | 1828 | 15529 | 15544 | TGCTTGGTGACACCCC | 108 |
| BN-0199 | 320 | 979 | 994 | 9751 | 9766 | GTGTTGAAATTCTGCA | 12 |
| BN-0200 | 321 | 1038 | 1053 | 9810 | 9825 | GGGAGTCCTTGAGTCT | 14 |
| BN-0201 | 322 | 1761 | 1776 | 14187 | 14202 | TTCGGAAGGTGACCTC | 28 |
| BN-0202 | 323 | 2051 | 2066 | 15978 | 15993 | GGATTCAGTCTGGGTC | 31 |
| BN-0203 | 324 | 980 | 995 | 9752 | 9767 | CGTGTTGAAATTCTGC | 62 |
| BN-0204 | 325 | 981 | 996 | 9753 | 9768 | GCGTGTTGAAATTCTG | 63 |
| BN-0205 | 326 | 1036 | 1051 | 9808 | 9823 | GAGTCCTTGAGTCTGG | 65 |
| BN-0206 | 327 | 1037 | 1052 | 9809 | 9824 | GGAGTCCTTGAGTCTG | 66 |
| BN-0207 | 328 | 1039 | 1054 | 9811 | 9826 | TGGGAGTCCTTGAGTC | 67 |
| BN-0208 | 329 | 1253 | 1268 | 11953 | 11968 | GTTCAGCTTGGGTAGG | 78 |
| BN-0209 | 330 | 1368 | 1383 | 13441 | 13456 | CCGTGTAGAAGAGGTC | 79 |
| BN-0210 | 331 | 1396 | 1411 | 13469 | 13484 | GCATAAGAAAGCTCAT | 81 |
| BN-0211 | 332 | 1397 | 1412 | 13470 | 13485 | GGCATAAGAAAGCTCA | 82 |
| BN-0212 | 333 | 1759 | 1774 | 14185 | 14200 | CGGAAGGTGACCTCAT | 105 |
| BN-0213 | 334 | 1813 | 1828 | 15529 | 15544 | TGCTTGGTGACACCCC | 108 |
| BN-0214 | 335 | 979 | 994 | 9751 | 9766 | GTGTTGAAATTCTGCA | 12 |
| BN-0215 | 336 | 1038 | 1053 | 9810 | 9825 | GGGAGTCCTTGAGTCT | 14 |
| BN-0216 | 337 | 1761 | 1776 | 14187 | 14202 | TTCGGAAGGTGACCTC | 28 |
| BN-0217 | 338 | 2051 | 2066 | 15978 | 15993 | GGATTCAGTCTGGGTC | 31 |
| BN-0218 | 339 | 980 | 995 | 9752 | 9767 | CGTGTTGAAATTCTGC | 62 |
| BN-0219 | 340 | 981 | 996 | 9753 | 9768 | GCGTGTTGAAATTCTG | 63 |
| BN-0220 | 341 | 1036 | 1051 | 9808 | 9823 | GAGTCCTTGAGTCTGG | 65 |
| BN-0221 | 342 | 1037 | 1052 | 9809 | 9824 | GGAGTCCTTGAGTCTG | 66 |
| BN-0222 | 343 | 1039 | 1054 | 9811 | 9826 | TGGGAGTCCTTGAGTC | 67 |
| BN-0223 | 344 | 1253 | 1268 | 11953 | 11968 | GTTCAGCTTGGGTAGG | 78 |
| BN-0224 | 345 | 1368 | 1383 | 13441 | 13456 | CCGTGTAGAAGAGGTC | 79 |
| BN-0225 | 346 | 1396 | 1411 | 13469 | 13484 | GCATAAGAAAGCTCAT | 81 |
| BN-0226 | 347 | 1397 | 1412 | 13470 | 13485 | GGCATAAGAAAGCTCA | 82 |
| BN-0227 | 348 | 1759 | 1774 | 14185 | 14200 | CGGAAGGTGACCTCAT | 105 |
| BN-0228 | 349 | 1813 | 1828 | 15529 | 15544 | TGCTTGGTGACACCCC | 108 |

### [Evaluation Example 1] Residual rate of human RasGRP4 mRNA in THP1 cells (human monocytic leukemia cell line)

An effect of an antisense oligonucleotide targeting a RasGRR4 nucleic acid on a RasGRP4 RNA transcript was tested in vitro.

The THP-1 cell suspension adjusted to 2,000,000 cells/mL with PBS (phosphate buffered saline) was dispensed in 1 mL aliquots into 1.5 mL tubes, and PBS was removed after centrifugation. Thereafter, the cells were resuspended in 0.1 mL of Nucleofector (registered trademark) solution V (mounted on Nucleofector Kit V manufactured by LONZA) containing an antisense oligonucleotide (150 pmol) or control siRNA (150 pmol) and then transferred to a cuvette, and the antisense oligonucleotide or the control siRNA was introduced into cells by Nucleofector (registered trademark) (manufactured by LONZA, program: V-001). Subsequently, 0.5 mL of pre-warmed RPMI-1640 medium (RP10 medium manufactured by Sigma) containing 10% heat-inactivated fetal bovine serum (manufactured by Biological Industries) and penicillin/streptomycin (manufactured by Gibco) was added, then the mixture was transferred to a 6-well plate containing 1 mL of RP10 medium, and incubated. After 48 hours, RNA was isolated from the cells using RNeasy kit (manufactured by QIAGEN), and then reverse-transcribed with QuantiTect Reverse Transcription Kit (manufactured by QIAGEN) to prepare cDNA. The prepared cDNA was used to measure the RasGRP4 gene expression level by quantitative real-time PCR (LightCycler 96 system, manufactured by Roche) with SYBR (registered trademark) Green 1. In the real-time PCR, the housekeeping gene Glyceraldehyde-3-phosphate dehydrogenase [GAPDH] mRNA level was also simultaneously quantified, and the RasGRP4 mRNA level with respect to the GAPDH mRNA level was evaluated as the RasGRP4 expression level. The results are shown in Table 11 as percent expression of RasGRP4 with respect to the control siRNA-treated cells. As the control siRNA, Silencer (registered trademark) Negative Control No. 2 siRNA (product No. (catalog No.) AM4637, purchased from Thermo Fisher Scientific Life Sciences Solutions) was used. The control siRNA was evaluated as a negative control.

**[Table 11]**

| Compd. No. | mRNA remain % |
|---|---|
| BN-0002 | 32 |
| BN-0003 | 66 |
| BN-0004 | 52 |
| BN-0006 | 78 |
| BN-0008 | 69 |
| BN-0009 | 16 |
| BN-0010 | 14 |
| BN-0011 | 27 |
| BN-0012 | 9 |
| BN-0013 | 45 |
| BN-0014 | 71 |
| BN-0015 | 20 |
| BN-0017 | 24 |
| BN-0018 | 19 |
| BN-0019 | 14 |
| BN-0020 | 29 |
| BN-0021 | 10 |
| BN-0022 | 21 |
| BN-0023 | 16 |
| BN-0024 | 22 |
| BN-0025 | 36 |
| BN-0026 | 10 |
| BN-0027 | 9 |
| BN-0028 | 21 |
| BN-0029 | 2 |
| BN-0030 | 55 |
| BN-0032 | 34 |
| BN-0033 | 50 |
| BN-0034 | 51 |
| BN-0036 | 38 |
| BN-0037 | 42 |
| BN-0038 | 31 |
| BN-0039 | 41 |
| BN-0040 | 38 |
| BN-0041 | 42 |
| BN-0042 | 26 |
| BN-0043 | 40 |
| BN-0044 | 37 |
| BN-0045 | 29 |
| BN-0046 | 23 |
| BN-0047 | 31 |
| BN-0048 | 20 |
| BN-0049 | 66 |
| BN-0050 | 29 |
| BN-0051 | 26 |
| BN-0052 | 63 |
| BN-0053 | 56 |
| BN-0054 | 39 |
| BN-0055 | 27 |
| BN-0056 | 35 |
| BN-0057 | 61 |
| BN-0058 | 50 |
| BN-0059 | 67 |
| BN-0060 | 12 |
| BN-0061 | 8 |
| BN-0062 | 21 |
| BN-0063 | 13 |
| BN-0064 | 6 |
| BN-0065 | 15 |
| BN-0066 | 33 |
| BN-0067 | 36 |
| BN-0069 | 53 |
| BN-0070 | 47 |
| BN-0071 | 27 |
| BN-0072 | 29 |
| BN-0073 | 23 |
| BN-0074 | 23 |
| BN-0075 | 12 |
| BN-0076 | 23 |
| BN-0077 | 18 |
| BN-0078 | 43 |
| BN-0079 | 15 |
| BN-0080 | 8 |
| BN-0081 | 36 |
| BN-0082 | 24 |
| BN-0083 | 49 |
| BN-0084 | 26 |
| BN-0085 | 70 |
| BN-0093 | 76 |
| BN-0094 | 52 |
| BN-0095 | 29 |
| BN-0097 | 62 |
| BN-0098 | 63 |
| BN-0100 | 36 |
| BN-0101 | 46 |
| BN-0102 | 13 |
| BN-0103 | 18 |
| BN-0104 | 27 |
| BN-0105 | 9 |
| BN-0106 | 12 |
| BN-0107 | 50 |
| BN-0108 | 22 |
| BN-0109 | 44 |
| BN-0110 | 21 |
| BN-0111 | 41 |
| BN-0112 | 37 |
| BN-0113 | 20 |
| BN-0114 | 22 |
| BN-0115 | 28 |
| BN-0116 | 77 |
| BN-0117 | 45 |
| BN-0118 | 43 |
| BN-0119 | 28 |
| BN-0120 | 44 |
| BN-0121 | 41 |

### [Evaluation Example 2] Caspase 3/7 activation in HepG2 cells

HepG2 cells were seeded at a density of 10,000 cells/well in a 96-well plate, and after approximately 24 hours, an antisense oligonucleotide was added such that a final concentration thereof was 30 nM or 100 nM using Lipofectamine (registered trademark) 3000 (manufactured by Thermo Fisher Scientific). After approximately 24 hours, the caspase 3/7 activity was quantified by Caspase-Glo (registered trademark) 3/7 Assay (manufactured by Promega Corporation).

In the present invention, an antisense oligonucleotide having low caspase 3/7 activity is desirable.

### [Evaluation Example 3] Residual rate of mouse RasGRP4 mRNA in primary mouse bone marrow-derived macrophages

An effect of an antisense oligonucleotide targeting a RasGRP4 nucleic acid on a RasGRP4 RNA transcript was tested in vitro.

Bone marrow-derived macrophages collected from a C57BL/6J mice (8 weeks old, male and female) were cultured for 7 days, then seeded on a 12-well plate at 250,000 cells/mL per well, and cultured overnight. On the following day, an antisense oligonucleotide (4 pmol) or control siRNA (4 pmol) was incubated with Lipofectamine (registered trademark) RNAiMAX Transfection Reagent (manufactured by Thermo Fisher Scientific) at room temperature for 20 minutes in an OptiMEM medium (manufactured by Sigma). An aliquot of 0.1 mL of the solution was added to the wells seeded with cells, followed by incubation at 37°C overnight. On the following day, the culture medium was substituted with fresh culture medium, and incubation was continued at 37°C. After 48 hours, RNA was isolated from the cells using RNeasy kit (manufactured by QIAGEN), and then reverse-transcribed with QuantiTect Reverse Transcription Kit (manufactured by QIAGEN) to prepare cDNA. The prepared cDNA was used to measure the RasGRP4 gene expression level by quantitative real-time PCR (LightCycler 96 system, manufactured by Roche) with SYBR (registered trademark) Green 1. In the real-time PCR, the housekeeping gene Glyceraldehyde-3-phosphate dehydrogenase [GAPDH] mRNA level was also simultaneously quantified, and the RasGRP4 mRNA level with respect to the GAPDH mRNA level was evaluated as the RasGRP4 expression level. The results are shown in Table 12 as percent expression of RasGRP4 with respect to the control siRNA-treated cells. As the control siRNA, Silencer (registered trademark) Negative Control No. 2 siRNA (product No. (catalog No.) AM4637, purchased from Thermo Fisher Scientific Life Sciences Solutions) was used. The control siRNA was evaluated as a negative control.

**[Table 12]**

| Compd. No. | mRNA remain % | Compd. No. | mRNA remain % |
|---|---|---|---|
| BN-0009 | 71 | BN-0062 | 78 |
| BN-0010 | 45 | BN-0063 | 31 |
| BN-0012 | 71 | BN-0064 | 28 |
| BN-0015 | 60 | BN-0065 | 20 |
| BN-0017 | 65 | BN-0073 | 34 |
| BN-0018 | 56 | BN-0074 | 36 |
| BN-0018 | 44 | BN-0075 | 58 |
| BN-0021 | 70 | BN-0076 | 41 |
| BN-0022 | 76 | BN-0077 | 48 |
| BN-0023 | 67 | BN-0078 | 57 |
| BN-0024 | 61 | BN-0079 | 38 |
| BN-0026 | 62 | BN-0080 | 65 |
| BN-0027 | 64 | BN-0081 | 69 |
| BN-0028 | 40 | BN-0082 | 55 |
| BN-0028 | 60 | BN-0095 | 58 |
| BN-0037 | 58 | BN-0100 | 66 |
| BN-0040 | 59 | BN-0102 | 68 |
| BN-0042 | 59 | BN-0103 | 43 |
| BN-0046 | 44 | BN-0105 | 73 |
| BN-0047 | 54 | BN-0106 | 41 |
| BN-0048 | 54 | BN-0110 | 51 |
| BN-0060 | 68 | BN-0113 | 58 |
| BN-0061 | 57 | BN-0114 | 55 |

### (Manufacturing Example 2)

A nucleic acid compound shown in Table 13 was prepared using an automatic nucleic acid synthesizer NTS M-8-SE (manufactured by Nihon Techno Service Co., Ltd.).

**[Table 13]**

| Compd.No | Chemical structure | SEQ No.of Compd. |
|---|---|---|
| C16-BN-0028 | C16-G(V)^G(L)^5(L)^5(x)^t^5(x)^t^t^5(x)^t^t^a^5(x)^A(L)^T(L)^T(V) | 350 |

In Table 13, "C16-" means that a moiety obtained by removing a hydrogen atom from the 5'-terminal hydroxy group is bonded to a group represented by the following formula.

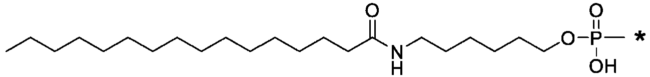

In the formula, * represents the bonding position to the oligonucleotide, that is, * is an oxygen atom of a hydroxy group at the 5' terminal.

C16-BN-0028 is an antisense oligonucleotide in which the group ("C16-") is bonded to BN-0028.

### [Evaluation Example 4] Residual rate of mouse RasGRP4 mRNA in muscle tissue of myositis induced mice

C-protein-induced myositis (CIM), a polymyositis model, was induced in C57BL/6J mice (8 to 10 weeks old, female). CIM was induced by intradermal administration of a recombinant human skeletal muscle C-protein fragment (200 µg/body) together with complete Freund's adjuvant (CFA) at four sites in total of both sides of the tail base and both hind limb bases, intradermal administration of CFA at both forelimb bases, and intraperitoneal administration of pertussis toxin (250 ng/body). C16-BN-0028 was subcutaneously administered once at 50 mg/kg on day 7 after the antigen immunization, and the mice were dissected 7 days after the administration, and the quadriceps femoris muscle was extracted. In the negative control group, PBS being an administration vehicle was subcutaneously administered at 10 ml/kg.

The quadriceps femoris muscle was immersed in TRI Reagent (Cosmo Bio), the tissue was homogenized with TissueLyser II, bromochloropropane was added and mixed, the aqueous layer was recovered after centrifugation, and RNA was isolated using KingFisher Apex (Thermo Fisher Scientific). Thereafter, the RasGRP4 gene expression level was measured using One-step PrimeScript III RT-qPCR Mix (Takara Bio Inc.). In the real-time PCR, the housekeeping gene Hypoxanthine Phosphoribosyltransferase 1 [HPRT1] mRNA level was also simultaneously quantified, and the RasGRP4 mRNA level with respect to the HPRT1 mRNA level was evaluated as the RasGRP4 expression level.

As a result, the mRNA level in the C16-BN-0028 administration group was 20 to 40% as compared with the mRNA of the negative control group. The RasGRP4 gene expression inhibitory action was exhibited in vivo.

### (Manufacturing Example 3)

Nucleic acid compounds shown in Table 14 were prepared using an automatic nucleic acid synthesizer NTS M-8-SE (manufactured by Nihon Techno Service Co., Ltd.).

**[Table 14]**

| Compd. No. | Chemical structure |
|---|---|
| BN-0291 | T(L)^G(L)^G(V)^5(x)^5(x)^t^5(x)^t^t^t^a^a^5(x)^T(V)^5(L)^A(L) |
| BN-0292 | T(V)^G(L)^G(L)^5(x)^5(x)^t^5(x)^t^t^t^a^a^5(V)^T(L)^5(L)^A(V) |
| BN-0293 | T(V)^G(L)^G(L)^5(V)^5(x)^t^5(x)^t^t^t^a^a^5(x)^T(L)^5(L)^A(V) |
| BN-0294 | T(V)^G(L)^G(L)^5(V)^5(V)^t^5(x)^t^t^t^a^a^5(x)^T(L)^5(L)^A(V) |
| BN-0295 | T(V)^G(L)^G(L)^5(Mo)^5(x)^t^5(x)^t^t^t^a^a^5(x)^T(L)^5(L)^A(V) |
| BN-0296 | T(V)^G(L)^G(L)^5(Mo)^5(Mo)^t^5(x)^t^t^t^a^a^5(x)^T(L)^5(L)^A(V) |
| BN-0297 | T(V)^G(L)G(L)5(x)^5(x)^t^5(x)^t^t^t^a^a^5(x)^T(L)5(L)^A(V) |

Positions in a sequence of SEQ ID NO: 1 being human RasGRP4 mRNA and/or SEQ ID NO: 2 being human RasGRP4 pre-mRNA targeted by the antisense oligonucleotides shown in Table 14, and sequence numbers and nucleobase sequences corresponding to the antisense oligonucleotides are shown in Table 15.

**[Table 15]**

| Compd. No. | SEQ No. of compd. | SEQ1 STRAT | SEQ1 END | SEQ2 STRAT | SEQ2 END | BASE SEQUENCE | SEQ No. of BASE SEQ |
|---|---|---|---|---|---|---|---|
| BN-0291 | 351 | - | - | 7206 | 7221 | TGGCCTCTTTAACTCA | 48 |
| BN-0292 | 352 | - | - | 7206 | 7221 | TGGCCTCTTTAACTCA | 48 |
| BN-0293 | 353 | - | - | 7206 | 7221 | TGGCCTCTTTAACTCA | 48 |
| BN-0294 | 354 | - | - | 7206 | 7221 | TGGCCTCTTTAACTCA | 48 |
| BN-0295 | 355 | - | - | 7206 | 7221 | TGGCCTCTTTAACTCA | 48 |
| BN-0296 | 356 | - | - | 7206 | 7221 | TGGCCTCTTTAACTCA | 48 |
| BN-0297 | 357 | - | - | 7206 | 7221 | TGGCCTCTTTAACTCA | 48 |

### [Evaluation Example 5] Residual rate of human RasGRP4 mRNA in THP1 cells (human monocytic leukemia cell line)

An effect of an antisense oligonucleotide targeting a RasGRP4 nucleic acid on a RasGRP4 RNA transcript was tested in vitro.

The same evaluation method as in Evaluation Example 1 was used. The results are shown in Table 16 as percent expression of RasGRP4 with respect to the control siRNA-treated cells. As the control siRNA, Silencer (registered trademark) Negative Control No. 2 siRNA (product No. (catalog No.) AM4637, purchased from Thermo Fisher Scientific Life Sciences Solutions) was used. The control siRNA was evaluated as a negative control.

**[Table 16]**

| Compd. No. | mRNA remain % |
|---|---|
| BN-0128 | 27 |
| BN-0136 | 17 |
| BN-0151 | 48 |
| BN-0166 | 22 |
| BN-0181 | 49 |
| BN-0188 | 72 |
| BN-0195 | 23 |
| BN-0196 | 7 |
| BN-0201 | 73 |
| BN-0204 | 78 |
| BN-0210 | 46 |
| BN-0211 | 24 |
| BN-0214 | 48 |
| BN-0217 | 41 |
| BN-0225 | 21 |
| BN-0226 | 23 |
| BN-0293 | 37 |
| BN-0294 | 51 |
| BN-0295 | 31 |
| BN-0296 | 33 |

### [Evaluation Example 6] Residual rate of human RasGRP4 mRNA in TF-1 cells (human leukemia cell line)

An effect of an antisense oligonucleotide targeting a RasGRP4 nucleic acid on a RasGRP4 RNA transcript was tested in vitro.

TF-1 cells were seeded at a density of 40,000 cells/well in a 96-well plate, and an antisense oligonucleotide (100 nM) was added using Lipofectamine RNAiMAX Transfection Reagent (Thermo Fisher Scientific). After approximately 24 hours, RNA was isolated from the cells using RNeasy Mini kit (manufactured by QIAGEN), and then reverse-transcribed with High-Capacity cDNA Reverse Transcription Kit with RNase Inhibitor (Applied Biosystems) to prepare cDNA. The RasGRP4 gene expression level was measured by quantitative real-time PCR with TaqMan Gene Expression Assays (Thermo Fisher Scientific) using the prepared cDNA. In the real-time PCR, the housekeeping gene GAPDH mRNA level was also simultaneously quantified, and the RasGRP4 mRNA level with respect to the GAPDH mRNA level was evaluated as the RasGRP4 expression level. The results are shown in Table 17 as percent expression of RasGRP4 with respect to the antisense oligonucleotide-untreated cells.

**[Table 17]**

| Compd. No. | mRNA remain % |
|---|---|
| BN-0128 | 30 |
| BN-0136 | 49 |
| BN-0151 | 51 |
| BN-0166 | 42 |
| BN-0181 | 45 |
| BN-0196 | 41 |
| BN-0210 | 43 |
| BN-0211 | 43 |
| BN-0214 | 51 |
| BN-0225 | 41 |
| BN-0226 | 64 |
| BN-0293 | 22 |
| BN-0294 | 23 |
| BN-0295 | 20 |
| BN-0296 | 20 |

### [Evaluation Example 7] Residual rate of mouse RasGRP4 mRNA in mouse macrophage RAW264.7 cells

An effect of an antisense oligonucleotide targeting a RasGRP4 nucleic acid on a RasGRP4 RNA transcript was tested in vitro.

RAW264.7 cells were seeded at a density of 20,000 cells/well in a 96-well plate and cultured overnight. On the following day, a nucleic acid compound was added using Lipofectamine RNAiMAX Transfection Reagent (Thermo Fisher Scientific). After approximately 48 hours, RNA was isolated from the cells using RNeasy kit (manufactured by QIAGEN), and then reverse-transcribed with High-Capacity cDNA Reverse Transcription Kit with RNase Inhibitor (Applied Biosystems) to prepare cDNA. The RasGRP4 gene expression level was measured by quantitative real-time PCR with TaqMan Gene Expression Assays (Thermo Fisher Scientific) using the prepared cDNA. In the real-time PCR, the housekeeping gene Hypoxanthine phosphoribosyltransferase 1 [HPRT1] mRNA level was also simultaneously quantified, and the RasGRP4 mRNA level with respect to the HPRT1 mRNA level was evaluated as the RasGRP4 expression level. The results are shown in Table 18 as percent expression of RasGRP4 with respect to the antisense oligonucleotide-untreated cells.

**[Table 18]**

| Compd. No. | mRNA remain % |
|---|---|
| BN-0128 | 40 |
| BN-0136 | 65 |
| BN-0151 | 63 |
| BN-0166 | 50 |
| BN-0181 | 45 |
| BN-0196 | 46 |
| BN-0210 | 76 |
| BN-0211 | 35 |
| BN-0214 | 64 |
| BN-0226 | 38 |
| BN-0293 | 43 |
| BN-0294 | 48 |
| BN-0295 | 42 |
| BN-0296 | 56 |

### (Manufacturing Example 4)

Nucleic acid compounds shown in Table 19 were prepared using an automatic nucleic acid synthesizer NTS M-8-SE (manufactured by Nihon Techno Service Co., Ltd.).

**[Table 19]**

| Compd. No. | Chemical structure | SEQ No. of Compd. |
|---|---|---|
| C16-BN-0019 | C16-5(v)^5(L)^G(L)^g^g^5(x)^a^t^a^a^g^a^a^A(L)^G(L)^5(V) | 358 |
| C16-BN-0026 | C16-T(V)^T(L)^5(L)^g^g^a^a^g^g^t^g^a^5(x)^5(L)^T(L)^5(V) | 359 |
| C16-BN-0181 | C16-G(V)^G(L)^5(L)^A(V)^T(V)^a^a^g^a^a^a^g^5(x)^T(L)^5(L)^A(V) | 360 |
| C16-BN-0196 | C16-G(V)^G(L)5(L)a^t^a^a^g^a^a^a^g^5(x)^T(L)5(L)^A(V) | 361 |
| C16-BN-0211 | C16-G(V)^G(L)^5(L)^A(Mo)^t^a^a^g^a^a^a^g^5(x)^T(L)^5(L)^A(V) | 362 |
| BN-0306 | C16-G(V)^G(L)^5(L)^t^a^5(x)^t^a^5(x)^g^5(x)^5(x)^g^T(L)^5(L)^A(V) | 363 |

In Table 19, "C16-" is the same as in Table 13. C16-BN-0019 is an antisense oligonucleotide in which the group ("C16-") is bonded to BN-0019, C16-BN-0026 is an antisense oligonucleotide in which the group ("C16-") is bonded to BN-0026, C16-BN-0181 is an antisense oligonucleotide in which the group ("C16-") is bonded to BN-0181, C16-BN-0196 is an antisense oligonucleotide in which the group ("C16-") is bonded to BN-0196, and C16-BN-0211 is an antisense oligonucleotide in which the group ("C16-") is bonded to BN-0211. BN-0306 is an antisense oligonucleotide that is non-complementary to human and mouse RasGRP4 and was manufactured as a negative control.

### [Evaluation Example 8] Residual rate of human RasGRP4 mRNA in THP1 cells (human monocytic leukemia cell line)

An effect of an antisense oligonucleotide targeting a RasGRP4 nucleic acid on a RasGRP4 RNA transcript was tested in vitro.

A THP-1 cell suspension adjusted to 1,000,000 cells/mL with RPMI-1640 medium (manufactured by Gibco, hereinafter referred to as RP10 medium) containing 10% heat-inactivated fetal bovine serum (manufactured by Gibco), penicillin/streptomycin (manufactured by Gibco), and 0.05 mM 2-mercaptoethanol (manufactured by FUJIFILM Wako Pure Chemical Corporation) was dispensed in 1 mL aliquots into 1.5 mL tubes, and the supernatant was removed after centrifugation. Thereafter, the cells were resuspended in 0.1 mL of Nucleofector (registered trademark) solution V (mounted on Nucleofector Kit V manufactured by LONZA) containing an antisense oligonucleotide (150 pmol) and then transferred to a cuvette, and the antisense oligonucleotide was introduced into cells by Nucleofector (registered trademark) (manufactured by LONZA, program: V-001). Subsequently, 0.5 mL of pre-warmed RP10 medium was added, and then the mixture was transferred to a 12-well plate containing 1 mL RP10 medium and incubated. After approximately 48 hours, RNA was isolated from the cells using RNeasy kit (manufactured by QIAGEN), and then reverse-transcribed with High-Capacity cDNA Reverse Transcription Kit with RNase Inhibitor (Applied Biosystems) to prepare cDNA. The RasGRP4 gene expression level was measured by quantitative real-time PCR with TaqMan Gene Expression Assays (Thermo Fisher Scientific) using the prepared cDNA. In the real-time PCR, the housekeeping gene GAPDH mRNA level was also simultaneously quantified, and the RasGRP4 mRNA level with respect to the GAPDH mRNA level was evaluated as the RasGRP4 expression level. The results are shown in Table 20 as percent expression of RasGRP4 with respect to the antisense oligonucleotide-untreated cells.

**[Table 20]**

| Compd. No. | mRNA remain % |
|---|---|
| C16-BN-0181 | 28 |
| C16-BN-0196 | 23 |
| C16-BN-0211 | 22 |
| BN-0306 | 114 |

### [Evaluation Example 9] Residual rate of human RasGRP4 mRNA in TF-1 cells (human leukemia cell line)

Antisense oligonucleotides were tested at various concentrations using the same evaluation method as in Evaluation Example 6. The results are calculated as a concentration (IC₅₀ value) at which an RNA transcript level was 50% with respect to untreated control cells and are shown in Table 21.

**[Table 21]**

| Compd. No. | IC₅₀ (nM) |
|---|---|
| C16-BN-0181 | 6.7 |
| C16-BN-0196 | 10 |
| C16-BN-0211 | 11 |
| BN-0306 | >100 |

### [Evaluation Example 10] Residual rate of mouse RasGRP4 mRNA in mouse macrophage RAW264.7 cells

An effect of an antisense oligonucleotide targeting a RasGRP4 nucleic acid on a RasGRP4 RNA transcript was tested at various concentrations.

RAW264.7 cells were seeded at a density of 20,000 cells/well in a 96-well plate and cultured overnight. On the following day, the entire amount of the culture medium was replaced with an FBS-free medium, and a nucleic acid compound was added. After approximately 48 hours, RNA was isolated from the cells using RNeasy kit (manufactured by QIAGEN), and then reverse-transcribed with High-Capacity cDNA Reverse Transcription Kit with RNase Inhibitor (Applied Biosystems) to prepare cDNA. The RasGRP4 gene expression level was measured by quantitative real-time PCR with TaqMan Gene Expression Assays (Thermo Fisher Scientific) using the prepared cDNA. In the real-time PCR, the housekeeping gene Hypoxanthine phosphoribosyltransferase 1 [HPRT1] mRNA level was also simultaneously quantified, and the RasGRP4 mRNA level with respect to the HPRT1 mRNA level was evaluated as the RasGRP4 expression level. The results are calculated as a concentration (IC₅₀ value) at which an RNA transcript level was 50% with respect to untreated control cells and are shown in Table 22.

**[Table 22]**

| Compd. No. | IC₅₀ (nM) |
|---|---|
| C16-BN-0181 | 166 |
| C16-BN-0196 | 208 |
| C16-BN-0211 | 189 |
| BN-0306 | >1000 |

All publications or parts thereof, patents, and patent applications cited herein are hereby incorporated by reference in their entirety.

### Industrial Applicability

The antisense oligonucleotide of the present invention inhibits RasGRP4 gene expression, and therefore is useful for treating, preventing, and/or ameliorating a disease for which inhibition of RasGRP4 gene expression is effective, particularly myositis such as polymyositis, dermatomyositis, and rheumatoid arthritis.

The sequences of SEQ ID NOs: 1 and 2 are known, and the respective sequences are shown below.
(Sequence of SEQ ID NO: 1) 
CTCTTTCCTTCCCAAGCACCGAGGAGGCCCCAGCTCCCTAGGGGCTGAGAAGCTGGAGTCCTGGGCAAGGGGAGGAGCTGAGC
CCTACTCTTGCAAGACCCCCGGCCTCCTCACCCCACGCGGGAAGCATGAACAGAAAAGACAGTAAGAGGAAGTCCCACCAGGA
ATGCACCGGAAAAATAGGAGGGCGAGGCCGGCCCCGCCAAGTGCGCCGCCACAAGACATGCCCCAGCCCTCGGGAAATCAGCA
AGGTCATGGCTTCCATGAACCTGGGCCTGCTGAGTGAGGGCGGCTGCAGCGAAGATGAGCTGCTGGAGAAATGCATCCAGTCC
TTCGATTCAGCTGGCAGCCTGTGCCACGAGGACCACATGCTCAACATGGTGCTGGCCATGCACAGCTGGGTGCTGCCGTCCGC
CGACCTGGCTGCCCGCCTGCTGACCTCATACCAGAAGGCCACAGGGGACACCCAGGAGCTGAGACGGCTGCAGATCTGTCACC
TGGTCAGGTACTGGCTGATGCGACACCCTGAGGTGATGCACCAGGATCCCCAGCTAGAAGAAGTCATAGGTCGTTTCTGGGCC
ACCGTGGCCCGGGAGGGCAACTCAGCCCAGAGAAGACTGGGAGACTCTTCTGACCTCCTGAGCCCTGGTGGCCCTGGCCCCCC
ACTCCCAATGAGCAGCCCAGGCCTGGGCAAAAAGCGCAAAGTGTCCTTGCTTTTCGACCACTTGGAGACGGGGGAGCTGGCTC
AGCACCTCACCTACCTGGAGTTCCGGTCCTTCCAGGCTATCACGCCCCAGGACCTGCGGAGCTACGTTTTGCAGGGCTCAGTA
CGAGGCTGCCCGGCCCTGGAGGGCTCCGTAGGTCTCAGCAACAGCGTGTCCCGCTGGGTGCAGGTGATGGTGCTGAGCCGTCC
CGGGCCCCTACAGCGTGCACAGGTGCTGGACAAGTTCATTCACGTGGCACAGAGGCTCCACCAGCTGCAGAATTTCAACACGC
TGATGGCAGTCACAGGGGGCCTGTGTCACAGTGCCATCTCCAGACTCAAGGACTCCCATGCCCACCTGAGCCCTGACAGCACC
AAGGCCCTCCTGGAGCTCACTGAGCTCCTTGCCTCCCACAACAACTACGCCCGCTACCGCCGCACCTGGGCTGGCTGCGCGGG
TTTCCGGCTGCCTGTACTGGGCGTGCACCTCAAGGACCTGGTGTCCCTGCATGAGGCACAGCCCGACAGGTTGCCTGACGGCC
GCCTGCACCTACCCAAGCTGAACAACCTCTACCTGCGGCTGCAGGAGCTGGTGGCCCTCCAAGGGCAGCATCCACCCTGCAGC
GCCAATGAGGATCTGCTGCACCTGCTCACGCTCTCCCTGGACCTCTTCTACACGGAAGACGAGATCTATGAGCTTTCTTATGC
CCGGGAGCCGCGTTGTCCCAAGAGCCTGCCACCCTCCCCCTTCAATGCACCTCTGGTGGTGGAGTGGGCCCCTGGTGTGACAC
CCAAGCCGGACAGGGTCACACTGGGTCGGCATGTGGAGCAGCTGGTGGAGTCTGTGTTCAAGAATTATGACCCTGAAGGCCGA
GGAACAATCTCTCAGGAGGACTTTGAGCGACTCTCGGGCAATTTTCCCTTCGCCTGCCATGGGCTTCACCCACCCCCACGCCA
GGGGAGAGGATCCTTCAGCAGAGAGGAGCTGACAGGGTACCTGCTCCGGGCCAGCGCCATCTGCTCCAAGTTGGGCCTGGCCT
TCCTGCACACCTTCCATGAGGTCACCTTCCGAAAGCCTACCTTCTGCGACAGCTGCAGTGGCTTCCTCTGGGGTGTCACCAAG
CAAGGCTACCGCTGTCGGGAGTGCGGGCTGTGTTGCCACAAACACTGCAGAGACCAGGTGAAGGTAGAATGTAAGAAGAGGCC
AGGGGCCAAGGGCGATGCAGGACCCCCCGGAGCTCCTGTCCCATCCACACCAGCTCCCCATGCCAGCTGTGGCTCCGAGGAAA
ATCACTCCTACACGCTATCCCTGGAGCCTGAGACTGGGTGCCAGCTTCGCCATGCCTGGACCCAGACTGAATCCCCACACCCT
TCCTGGGAAACAGATACGGTCCCCTGCCCGGTGATGGACCCACCATCAACTGCATCCTCCAAGCTGGATTCCTAGACATCTCT
TGGTCTCCTCTCTTCCTCACTCCCTTCCCCCAGTCAGTCCTGAGTCCTGCCGTCAGACTCTGGCAGGGCTCCCAGAGGTAGGC
CTCCAGAGGCATCCGCCTTCCCATCCACACTGCCTTTAGTGGTACGTCCGTCATCTTTTTCCCTGGAAGTGACTTTCCTCTTT
TGCATCCTGGTGGATGCTAATCCTGCTCCCTTTCCCTGCACTTAGTATCTGTGCGGCTGTGTGTTGGATACATTTAGACTCAG
GCCTCATTCTACCAACTCCCTACATCTCCATTTCTTTTTTTTTTTGAGACAGAGTCTTGCTCTGTCACCCAGGCTGGAGTGCA
ATGATGCAATCTCAGCTCACTGCAACTTCCGCCTCCTGGGTTCAAGTGATTCTTCTGCCTCAGCCTCCCAAGTAGCTGGGATT
ACAGGCACGCGCCACCATGCCCGGCTAATTTTTGTATTTTTAGTAGAGACGGGGTTTCCCCATGTTGGCCAAGATGGTCTCAA
ACTCCTGACCTCATGATCCACCAGCCTCAGCCTCCCAAAGTGCTGGGAATACAGGCGTGAGCCACCACGCCCAGCTCCTGTTT
TTCTGCATAGAATGTGCTCCCTCTGACAGCCTATACTTTTTCCCTCTTTATCTCGTTGAACGTCTGTCTTTCCTCAACAGGAG
CATCAACTCCAGGAGAACAGGGATTTCTATCAATGTCATTCACTGCTGCATCCCCAGTGCCTAGAACAGGGCTGGCAGGTGGT
AGGCGCCTGACAGATGCTTGTCTGATGCCTGAATGTCTCCTTACCCATGCCCACGGCACAGGATAGATGTGCTATAGGGCAAA
GAACTTTGAGGGTCGAGCAGCAGGGGCCCATTCAGTCCCAGGGAGCAGAGACCCCCCCAACCCCTTCACAAACCCCAACACCC
CTGACTTGGCCCCCACAGAGAGAGGTCCTACAGCTGTCATAAATTAAATTTATTCTCTGGA
(Sequence of SEQ ID NO: 2) 
TTTTGTAGAGACGGGGTTTTGTCACGTTGTTCAAGCTGGTCTCAAACTCTTAGACTCAAGCGATCCGCCTGCCTTGGCCTCCCAAAGTGCTG
GATGACAGGCGTGAGCCCCTGTGAGCCCCCGTGCCTGGTCCGGTATGTAATTTTTTTTAAGGTAACAGTGGATTGTGTGAGGATTAAATGAG
TTAATATTTTTAAAGTCCTTACAGCAGTGTGTGGTACACAGTAGGTGCTATGTGGAGGTTTGTTACAATAAAGTCAGAAAGTGAAAATGTTT
TCCAGGGTACAAATGGTATCACTTAAACTAGGAATAAGAGACGCCTATGAAGAAATATACAAAATACTATGTCTTGTGAGTTTCCATTCAAA
CCTTCATGAATTATATATATGAATATGTCCTTCTAGACAGACAGACTTATCTTTTTGAGTGAGTGTGTGTGTTTTTACATAAATATCAGTCA
TGCTTTAAAAAGAAAAGCCCCTTCTTTTATTGAAGTATGACAGTGACGGGTCTCGACACCGAAAGATGGGGGAGATAGAGAGCCCGCCGGGG
AGCTTGAGGGTGGGAGAGCCAGCGATGCCGGAAACCTCAGCAGAAGGGATCCCCGTGTGGGGGAGGGGGGCCTTCGGTCTCTCCCCAGCCTC
TTCCTTCCCCCTGGCCTGAGGGTGGGTGGGGAGGGGCAGTTCCTCTTTCCTTCCCAAGCACCGAGGAGGCCCCAGCTCCCTAGGGGCTGAGA
AGCTGGAGTCCTGGGCAAGGGGAGGAGCTGAGCCCTACTCTTGCAAGACCCCCGGCCTCCTCACCCCACGCGGGAAGCATGAACAGAAAAGA
CAGTAAGAGGTGAGAGGACCCTCCCCAGGGAGCCCGGCAGCACCCTGAGGCCTCCCTCTGCATGGGAGGGCTGGGGAGATCACTCGCCAGGG
TGGCAGGATTCCTGACTTCTTTGGGATCAGGCGCAGGGGGGCCAGGTGGATGCTTCAGGGAGTCTGGAGCCAGCTACGGCTGCAGTCGCTTG
TATGTGGCATCTGTACCGTGTTGTCCCTGGAGTTTGAGCTGCTCCGTGCCCCTTTTCCACTCCTCATCACCCCACCTCCATCCCTGGCCTGG
GCTGAGTGTCTCTGCCCTCCCTGGGGGCTTCCTGGTTCCCCATCCCCGTGGACTGGAAACTCCAGGCTCCTGCAGGGCCTGGCCCAGGAGAG
GGGAGTGTGGCACAGGGCAAGGCGACTCCCCTGCCTCTTAAACCACTCCTGGCTGTTCCAGACCCCTACAGGGGGCTGCAGGACAGGGCCTA
GTGGCTGGCTGTGCGGCCGGTTCTGGAATGGGCTTCATCTCCTGGGCAAGCTGGCCCTTCTGTCCCCACAGAGGCAGATGGTGGGTGGCTCC
CTAGCCTGGGTAACTCCTCCTGCCACCCCCAGCACCACTCTGATGGGCAAAGGGCTTTCCTCCCCACCCAGCAGTTTAGTGGTAGACTCCTG
GCTGCCTAATTTCCTAGCTGTGTGACCTTAGGTAAGTCACTTAACTGCTCTGTGTCTCGGGTTTTTCTTCTGTCAGTAGAACAATCATATCC
TCTACCTCGGAGAGGCCTGTAAGTACTCAATGAGACACTCTAGACCAGTGGTTCTCAAACTGGAGCGAGCCTCAGAACTGTCTGGAGGGCTT
GTAAAATACAGGTTGCTGGGCTTACCTTTAAAGTGTTTGGTTTTTTTTTTTTTGTTTTTTTTTTTTGAGACGGAGTCTCGCTCTGCCGCCAG
GCTGGAGTGCAGTGGCACAATCTTGGCTCACTGCAACCTCTGCCTCCCGGGTTCAAGCGATCATCCTGCCTCAGCCTCCCAAGTAGCTGGGA
CTACAGGTGTGCACCACCACACCCAGCTACTTTTTTGTATTTTTAGTAGAGATGGGGTTTCACCATGTTGGGCAGGATGGTCTCGATCTCTT
GACCTCGTGATCCACCCACCTCGGCCTCCCAAAGTGCTGGGATTAGAGGCATGAGCCACCACCCTTAGCCTTAAAGTGTTTGATTTTGTAGG
TCTGGGTTGGGACCTGAGAATCTGTATTGATATCCAGTTCCCAGGTGATGCTTTTGGTACTAGCTGGGTGCCCACACTTAGGGAAATGCTGA
CTTAGTTAGAATGCTCCTTCCTTAGCCTAGGACATATATAATACTTTAAAATGTCCCTTCTAGTAACGATGGCTCATGCCTCTAATCCCAGC
ACTTTGGGAGGATAAGACAAGGAGAACTTGAAGCCAGGAGTTCTAGACCAGCCTGAGAAATATAGTGAGACCCCCCCCCCCATTGACACACA
CCCAAACACACACACAAAATTAGCTGGGTATGGTCGTGCACACCTGTAGTCCCAGCTACCTGGGAGACTAAGGCAGGAGGATCACTCGAGTC
CAGGAGTTCAAGGTTACAATGAGCTTTGATTGCACAACTGTGCTCCAGCCTGGGAGACAGAGCAAGACCCTGTCTGAAAAAGGGTAGAGGTG
GGAGCCTGGCGTGATGGCTCACACCTGTAATCCCAGCACTTTCAGAGGCAAAGGTGGGAGGATCAGTTGGGCCCAGGAGTTTGAAACCAGCC
TGGCCAACATGGTGAAACCCCATTTCTACTAAAAATACAAAAATTAGCTCGGCGTGGTGGCACGTGCCTGTAGTCCCAACTACTTGGGAGGC
TGAGGCAGGAGAATTGCTTGAACCCGGGAGGTGGAGGTTGCAGTGAGCTGAGATCGCACCACTGCACTCCAGCCTGGGTGACAGAGTGAGAC
CCTGTCTCCAAAAAAAGAAAGAAAGAAAGAAAAAAGAAATCCCTGGAGAAGGTCCAAGGACTATCCTCATTTTTCCTTCGAGGAAATTGAGC
CCCAGAGAAATTAAGTGACTAACACGAAGTCACAACAGCTGGCAAAAATTTCTGGATTCTACTTTCGTCTGAAGCTTGGATTCAGCAGCCTC
CCTGCTCCCACCATCCTTGTCTCTCCATTACCCTCTTCCCCTATCAGGAACAGCAGCCTCTGGGAGGCACCAGGCAGAGGGAGAATTATTAT
TACTATTATTACTATTATTATTATTATTATTTTGAGACTAAGACTCGCTCTGTTGCCCAGGCTGGAGTGCAGTGGCACGATCTCGGCTCACT
GCAACCTCCGCCTCCTGGGTTCAAGCAAGTCTTCTGCCTCAGCCTCCTGAGTAGCTGGGATTACAGGAGCCCACCACCACGCCTGGCTAATT
TTTGTATTTTTAATAGAGATGGGGTTTCACCATGTTGGCCAGGCTGGTCTCGAACTCCTGACCTCAAGTGATCCACCCACCTCAGCCTCCCA
AAGTGCTGGGACTACAGGCGTGAGCCACTGTGACTGGCCTCTCTTTTTAATTTTTTTATTATTATTTTTGAAACAGAAATCACTCTGTCACC
CAGGTTGGAGCACAGTGATGCAGTTCCGGCTCACTGCAGCCTCCACCTCCTGGGTTCAAGCGACCCTCCTGCCTCAGCCTCCTGAGTAGCTG
GGATTACAGGCGCCCGCCATCACGCCTGACCAATTTTTGCATTTTTAGTAGCGATGGGGGTTTCACCATGTTGGCCGGGGTGGTCTCGAACT
CCTGGCCTCAAGTGATCCACCCACCTTGGCCTCCCAAAGCTTTGGGATTACAGGCGTGAGCCATCACACTGGTCCAAGAGCCTTGCTCTCTT
GAGGGCTGTGGGAGGAAGGAGGGGAGGGTGTTAGAGACCAAACGGGAGCCCTGAGGAGGCAATTCCTGAAGGCAGGTCCTCCCATGCCTCTG
CCTGGGGAGGACAGAAGAGATTCCAGGGGTCTTCTCCCGAGTCCCTGGTAGGCCAGGAGTAATCCTGGGATCTGCCAAAAGGAAGTCTCTTC
CAGGATGCCCTGGAAGCAGTCAAGAGGAAGTTGGGGGCAATGCCTGGGGAAGGAGTTCTTATGATTCCCTGAGGGGTCTTGAGTGGTCCCAG
GGGTCTTCTACTGTGATTCCCTGGGAGATTCAGAGCAGTCCTGAGCGGCGGTCCCCAGGCTTTTTGGCATCAGGGACCAGTTTTGTGGAAGG
GGGTGGGGATGGTGTGGGGATGATTCAAGTTAATTACATGTATCCTGTGCTTTATTTTTATTATTATTACATTGTAATATATAATGAAATAG
TTACGCAGCTCACCATAATGGAGAATCAGTGGGAGCCCTGAGCTTGTTTTCCTGCAACTGGATGCTCCCATCTGGGGGTGATGAGAGACAGT
GACAGATCATCAGGCATTAGGCATCAGGCATCATAAACAGCACGCAGCCTAGATCATCAGGCATCATAAACAGCACGCAGCCTAGATCCCTC
GCATGTGAAGTTCACAATAGGGTTCATGCTCCTAAGAGAGTCTAATGCCCCAGCTGATCTGACAGGAGGTGGAGCTCAGGCGGGGGTGCTTG
CTTGCCCGCTGGTTCCTAACAGGCCACGGACTGGTACCTGTCTGTGTCCCAGGGGCTGGGGACCCCTGGTCTTGAGGATCTTCCTCTCTGAT
TCCCTGAAGGGAGCAGGAATATCCCTGGGAGTTTGCTTGTGGGAGAGGGCTCCCATGATTCCCCCAGGGATCTGGAATATTCCTGGGGATTC
TCTCTAGAGTCCCTGGTAGGTCAGGGGTAACCCTGGGGCTTTGCCTTTAGGAAGCCTCTCCCGTGGTGCCTTGGGGCATTAGAGTCAAAAGG
AATTCTAGGGTCTGTCCGAAAGAAGGTGCTCCCATGACTCCTTGGGGGCTGTGCCCACAGGAAGTCCCACCAGGAATGCACCGGAAAAATAG
(Sequence of SEQ ID NO: 2 (Continued)) 
GAGGGCGAGGCCGGCCCCGCCAAGTGCGCCGCCACAAGACATGCCCCAGCCCTCGGGAAATCAGCAAGGTCATGGCTTCCATGAACCTGGGC
CTGCTGAGTGAGGGCGGCTGCAGCGAAGATGAGCTGCTGGAGAAATGCATCCAGTCCTTCGGTAAGTGTCCTCTCCCCAGCAGCCTCCCTGG
CCCTAACCTCGGGCTCAGCACTCGCTTCTCTCCACCTCAGTTTTTGGAGGTAAAATGAGAACAGGGCTGGGTGGAATGAAACTCAATCACTA
CATTAGAGATAACAGAAACACCTGCATTTCTTCTTTCCTGAAATGGAAATATATACATTTATATATATATATAATATATGTGTATATTATAT
ATATTTATATAAATCTTTTTTCTTTTCTTTTCTTTTTCTTTTGAGACAGAGTCTCGCTCTGTTACCCAGGCTGGAGTGCAGTGGCCTGATCT
CAGCTCACTGCAACCCCCACCTCCTGGGTTCAAGAGATTCTCCTGCCTCAGCTTCCCGAGTAGCTAGGATTACAGGCACATGCCACCAGGCC
CAGCTAATTTTTGTGTTTTTAGTAGAAACGGTGTTTCACCATGTTGGCCAGACTTGTCCCGAACTCCTGGCCTCATATGATCCGCCCGATTC
AGCCTCCCAAAGTGCTGGGATTACAGGTGTGAGCCACTGTGCCCGGCCAGTCTGGTGCTATTTCAATCTTCAGCAAGACTTGCCAGGGGTGA
TGCTGCCCCATGCACAATACAGATAAGGAAACTGAGGCACAGGAGGTTAGGTTGCCTGTCCAAGGTCATGCTGGGGATCACCGTTAGAGCTG
GTCTTTGAACCAGGTGGTCCGGCAGGATCTGGAGCTACGCTCTGCATTGCTTGAGGGCCATTCTGTCACAGAGTACCCCTCCTTCCACCCCA
GATTCAGCTGGCAGCCTGTGCCACGAGGACCACATGCTCAACATGGTGCTGGCCATGCACAGCTGGGTGCTGCCGTCCGCCGACCTGGCTGC
CCGCCTGCTGACCTCATATCCTCGAGGACTGTGAGCTATGGGTGGAGGGCAGAGGGCAGAGTCGTGAGAACCTGGGCAATGGGAATGGGGAG
CAGGCAGGTCATGGATCCTGGAAAACAGAGCCTTGACCAGAACTCAAATACCAGAAGGCCACAGGGGACACCCAGGAGCTGAGACGGCTGCA
GATCTGTCACCTGGTCAGGTAGGGCTGGGCCGAGATCCCCACTCTCAGGACCCACGACTTCCATCCTGCTTCCCCCTCAAAAAACATTCCTC
AGAGAATCCCCAGGCCAGTTCCCACAGACCCAACCAGCCCCAGAGGCCAAAATCCCACTTCTGAGACACCTGTAGCTTGGTTCCTAAGACCC
TCTTAGTTCCGTTCCTTAGAACCCCCACTTCCTAGAGACCCCCCTCACCAACCAGATCCCATAGACCCTCCTAGCTGGTGCCCAGAGACCTC
CAACTCTGATCCCCAAAATCTCTACCTCCCACAGACCCCAGCCTAGCCCATAAGACCCCCTCATTCTGTCCCTCCAAAAATTTCACCCCCGG
AAGCCCCCAGTCTAGTTCCTAGGACGCTCCACGATCTTGCTTCCCAATAATCCCACTTCCAAGTGACTCCCCGGTCTGTTTCCCACAGATCC
CCCCAGTCTCTGTTAAGAGACCCCCATCCTACTCCAGAGATTCTCCAAGCCTGGTGCCCCTCCAAACCCCAACCCTCCAGGGACCCCCACAC
ACATTTAGAGACCCCCTAGCTCAGCACCTAGAAATTCCTCAGCCCAAAATACCCCTTGTAGAGACCTTCGCCACCCCCGGCCTCATCTCACC
ATCTCCAAACCACTCTCAGGTACTGGCTGATGCGACACCCTGAGGTGATGCACCAGGATCCCCAGCTAGAAGAAGTCATAGGTCGTTTCTGG
GCCACCGTGGCCCGGGAGGGCAACTCAGCCCAGAGAAGACTGGGAGACTCTTCTGACCTGTGAGTCAGCCCTGTGCTCCCCTCTTCCCTGCC
CCATCGCCATTCTGCCATCTCCCTCTATATCCCCAAGCCCTCAACTGTGAGCCACTCCAATACCCTGCCCCTCTTGGCCCCCAGCCTGAGCC
CTGGTGGCCCTGGCCCCCCACTCCCAATGAGCAGCCCAGGCCTGGGCAAAAAGCGCAAAGTGTCCTTGCTTTTCGACCACTTGGAGACGGGG
GAGCTGGCTCAGCACCTCACCTACCTGGAGTTCCGGTCCTTCCAGGCTATCACGGTGAGGACCCCCTCCCATCCCCGTCCCAAGCAGGGGAG
ACACTGGGGTAGGGGAGGGATTGGCTGGGGGAAGGTAGGACTGAGAGGACACCTGCATGGTCATTCGGCAGAGATCTCAAAGTCTTAAACTC
AAACCTGTATGCTAGAAGGTCCAGGAAAATGAGTTAAAGAGGCCAAGGCAGGCAGATCGCCTGAGGTCAGGAGTTTGAGACCAGCCTGGCCA
ACATGGTGAAACCCCATCTCTACTAAAAATACAAAAATTATCCAGGTGTGGTGGTGCATGCCTGTAATCCCAGCTACTTGGGAGGGTGAGGC
AGGAGAATCACTTGAACCTCAGGAGGCGGAGATTACAGTGAGCTGAGATCACACCACTGCACTCCAGCCTGGGCGACAGAGCGAGATTCTGT
CTCGAAAAACAACAACAACAAAAAAGTAAAATAAATAAATAAAAGGCTTATTGCAGCGACTGGCACTTAGTAAGTGCTCAAAATATTTTATC
ATTTATTCAACAAGCATTTAAATTCATGGTGCCTCAGTTTCCTCATCTGTAAAAAGGGGATATGCACAGTTCCTGCCTTGCAGGAACAGTGC
CTGGTCCAGGGTAAATGCTATCTGCTTGAACCATTTGAAATTGTCATATATTACCTACCCAAGGATCAGTTTCATGTGCTTCAACATATATA
TGCATTAGCTTTTGCCATCTGGACATCATCATTTCAAGGGAAGGTGGGGATCTGTTTCCTTCATGTGAAACTGTCCCGATTGTATGTGTGTG
TTGTGTGTGTGAGTGTGTATGCAAATATACTCAGTGTAGTGGATGTGGGTATGAAATGAACACTCCTTGGGCAGAACAGATAGAGGTTGATT
TCAATTGATCTTCCCCAGCCTTCCCTTTGCTTACTGTATACCAGTCAGGTGTACAGTTAAGAGCATATAGACGCCCACCCTGACTCACACCT
GTGTGACCCTGGGCAAATTACCTATCCTCTATGTGCCTCAACTGTCTCACCTCTAAAATGGAATGATACTCACCTCATAGAGTTATGAGGAT
TAAATTATTTAATATGATTACTTGTCAAATATAGTGCCTGGCTATATTGAAACATGCCTCCTCAGATGGGAGAGGAGCATGGTGACGTTAAG
GACATGTAGCAGCCTGAGGGTTGGGGGACAGATGACAAAGGTCACTCCGGCTAGACCAGGCTAGGACATCAGAGATCGTGACTGCTAGTTGG
ATGCCATGGAAGGGCAGGGCCTGAGCGCCACGGCCCACCTTGACACCCACCTCGCTCCCAGCCCCAGGACCTGCGGAGCTACGTTTTGCAGG
GCTCAGTACGAGGCTGCCCGGCCCTGGAGGGCTCCGTAGGTCTCAGCAACAGCGTGTCCCGCTGGGTGCAGGTGATGGTGCTGAGCCGTCCC
GGGCCCCTACAGCGTGCACAGGTGCTGGACAAGTTCATTCACGTGGCACAGGTGAGGCCCGCCCCTTGGGGCTGCGAACTCCACTGCAGGCA
GCCACCTGGTCCTTCCCCTTCCAGACCCTCGACCCCACAGGGTCATCCCGGCGGTGACGTCACGCATCTCCCGGCCCCGCCCCCGAGGCCAC
AGCCTGGGCATTCCAACCAAGGACCGTAACTTCTTCTTTGGCCTTGATGTCACCGCGTCGCACCCACCGTGGCCCCACCCCTTGGCCCTAGA
CGTGACAGGCTCCCAAGCCCTATGCCCGAATATTCTATCCCCAGGTCAATGAGAGTGTCTCTTCCATGCCCCCACCTTTGTGGGGCATGGAA
GCCCGAGGCTTCAATCCCCAGTCCCCAGGCCCCAGCGGTCGCTTCGGGATTGTGCATCGCTCCCCATTGCGTCCGGGCTCTCCCCGCCCCGC
CCCATTGTGTTACGCCCTTCTCTTCCCCTCCCTTCCCCCTCCTTTCCGGACACCTTCTCGGCCTTCTGGGGAAAAGGGAAGCCCTACGGCCC
CGCTGTGGCCGCGCCCCCTTGCGTATTTCCACGCAGACCACGCCCTTCCTCTGGTGTCTTCCCTGGCTTGCTTACTCCTGCAGGGCCCCTGA
GACCTGGGGGTCTCGACGTCCGCCTGGGGTGTCCTTAGTCTGAGCATTTTCCATTCCTCCCTGGCCCCACTCTCTCCACAGCAACCTCTGCC
GAAATTTGTCAAAAGGAGGGCAAGGGAATTCCCCAGCTGCCCTGGTGGCCCCACACCTGTCTAGCATCTGTCTGACTCCTCCATATCTCTCT
GGCCCTATTCACCTCTGCCTGGCTCCCAGCTCCTGCCCAGAGCCTGCACCTTCTTGCCTCCCCGCACCTGTGTGACTCTCAGAGCAGTCAGA
CCCCCAGATGGCTGACACCCTGACATGTCTGAAGCCTCCTCCACCTGACACACACCTGTCTATTCCCCAAATCCGCCCAACTTCTCGTACTT
CCTGTCTCCCCAGATTTTCTAACCTCGTATCAGTCAATCACTTGCTTGACCCTCCCGGCTCAGATAGCCATCTGACACCCCTGTGAGGCCCA
CACATGGCATGTCCCACTCTTGCCGCACACACCCGCCTTCTCTCACCTGTCTGATCCCCACATTGGTCCACACATGCCTGGGACCCCACCTC
AACTGGGATGCTGTCATCTGAACTCCTGACTTGCCCTCCCTCCCGTGACGGCCCTGTGTGCATGCTTCTCTTCCTAGAGGCTCCACCAGCTG
(Sequence of SEQ ID NO: 2 (Continued)) 
CAGAATTTCAACACGCTGATGGCAGTCACAGGGGGCCTGTGTCACAGTGCCATCTCCAGACTCAAGGACTCCCATGCCCACCTGAGCCCTGA
CAGCACCAAGGTGAATCCCACTACCCTCCCCAAAGCTCCAAGTGGTCAGCTGTCTTGAGCCCCCACACCTAATTCCCCAAGCCCTGTCCATT
CCTGCCCAGGGGTCCTGGTACCACATGAAGTCCTTGCTCCTCCAAATCCCAGACCCCCAGGCTCTGCCAGCATTCCTCCTCCCTGTCATCCA
TCCACTCATGGTGCTGATCCCTCTCTGCTTTCAGTGTGTCTGGTCCGGAGCTGGACTGTGAGGGAGACAGATGGGAGACAGGCTAGCAGAGA
ACTTTGCAGTCCCCATGGGGAGGACTGGCTGAGGTCAGAAAGTGGCAGGAAATTAAGCCGAAGATGCCCTGGGAAGATCCTATAGCGCCTTG
TAAGATGGGCTAAGCTAGGGAAGTTTTGAGAGTGATGACACACCCTTGATAAAATCTGGGTTTTTAAGAAATAATTATAGGCTGGGTACAGT
GTGGCTCACACTTGTAATCCCAGCGTTTTGGGAGGCCAAGATTGGAGGATCACTTTAGCCCAGGTGTTTCAGACCAGCCTGGGCAACATAGC
GAGACCCCATCTCCAGAAATTTTTTTTTTTTTTTTTTTTTTTTTTTGAGACAGAGTCTCGCTCTTGTCGCCCAGGCTGGAGTGCAGTGGTGC
AATCTCGGCTCACTGCAACCTCTGCCTCCCGGATTCAGGTAATTCTCCTGCCCCAGCCTCCCGAATAGCTGGGATTACAGGGGTGCACCACC
ATGCCCTGCTAATTTTGTATTTTTAGTAGAGACAGGGTTTTACCATGTTGGCCAGGCTGGTCTCAAACTCCTGACCTAAGGCGATCCACCCA
CCTTGGCCTCCAAAAGTGCTGGGATTACAGGTGTGAGCCTCCGCGCCTGGCTGAAAAAAAATTTAAAAATAAATTAGCCAGGGATGGTGGCC
CATGCCTGTAGTCCCAGCTACTTGGGAGGCTGAGGGGGAGGACTGCTGGAGCCTCCCCCTCAGCCTTTTCATTGTTGTTTTTTGAGACAGAG
TCTCACTCTATCTCCCAGGCTGGAGTACAGTGTCGAAATCTTGGCTCACTGAAGCCTCCACCTCCCAGGTTCAAGCGATTCGCCTGCCTCAG
ACTCCCGAGTAGCTGGGATTACAGGCCTGCACCACCAGACCCAGTTAATTTTTGTATTTTTAGTAGAGACAGGTTTTTACCATGTTGCCCAG
GCTGTTCTCGAACTCCTGACCTCAGATGATCCGCCTGCCTCGGCCTCTCAAAATGCTGGGATTACAGGTGTGAGCCACCGTGCCCAGCCATC
AAGCTCAGGAGTTTAAGGCTGCAATGAGCTGTGATCATGACACTGTACTCCAGCCTGGGTGACAGAGTGAGACCCTAGCCTAAAAAATAAAT
AAATAAAAAGGCCGGTCTTGATGGCTAACGCCTGTAATCCCAACACTTTGGGAGGCCGAGGCAGGCAGATCACCTGAGGTGAGGAGTTTGAG
ACCAGCCTGGCCAACATGGTAAAACCCTGTCTCTACTAAAAATACAAAATTAGCCTGGCGTGGTGGCTCAATGCCTGTAATCCCAGCTTCTT
GGGAGGCTGAGGCAGGAGAATCGCTTGAACCCAGGAGGTGGAGGTGGCAGTAAGCCGAGATCACGCCACTGCACTCTAGCCTGGGCAAGACA
GCAAAACTCCATCTCAAAAAAAAAAAAAAAAAAATCAAAAAGAAAGAAAGATAAAAGAAATCATTTTAGCCAAAGAATGGGGGATTGTGGAA
CTTCTCTGAGATGGGGACCCAAGAGGAAGAGCAGTTTGGGGGAGATGCTGAGACCGGCATGGGATATGGAAGGGCTCCAGAGGTTTCCACAA
TGCCACTGGGTCCCTGAAGGCCTAGAGCCCACACAGGAGGCTGCTCAGGGGATGGGAGTGTCCTGATAACGCCCCAATCCCATGTCCCTCCT
CCCCAGGCCCTCCTGGAGCTCACTGAGCTCCTTGCCTCCCACAACAACTACGCCCGCTACCGCCGCACCTGGGCTGGCTGCGGGGGTTTCGG
GCTGCCTGTACTGGGCGTGCACCTCAAGGACCTGGTGTCCCTGCATGAGGCACAGCCCGACAGGTTGCCTGACGGCCGCCTGCACCTACCCA
AGCTGAACAACCTCTACCTGCGGCTGCAGGAGCTGGTGGCCCTCCAAGGGCAGCATCCACCCTGCAGCGCCAATGAGGATCTGCTGCACCTG
CTCACGGTGAGCCCCCCTGGCCCCGCCCAGGCTGGGCTTCCAAGGGTACTGAGATATAGGGTCCTGGGTGTGTGAGTGTCTCCAGGACCAGG
ATAGAAAAGTGCTGGAGTGCATGGGTTTTGAAGCCAGAACTTCTAGGTTTTTTTCTAGCTGCGTGATCTTGGGCAAGTCACTGAACCCTTAA
GCCTCAGTTTCCTTATCCACAAAATGGGAATAATAAAAGGACCCAGCCGGGCGCAGTGGCTCACGCCTGTGATCCCAACACTTTGGGACGCT
GAGGTGGGCAGATCACCTGAGGTCAGGAGTTCGAGACCAGCCTGACCAACATGGAGAAACCCCGTCTCTACTAAAAATACAAAATTAGCCAG
GAGTGGTGGTGCGTGCCTGTAATCCCAGCTACTTGGGAGGCTGAGGCAGGAGAATTGCTTGAACCCGGGAGGTGGAGGTTGCAGTGAGCTGA
GATTGCGCCATTGCGCCATTGCACCCCAGCCTGGGCAAGAAGAGCGAAACTCCTTCTCAAAATAAATAAATAAATAAAAATAAAATAAAATA
AAAGGACCCACTTCAGAGGGTGGTTGAGGAGAGACAGCCAATGACATCGTTCAAAGAATGTCTTTAAGACAGTGTCTAGCCGGGCGCAGTGG
CTCATGCCTCTAATCCTAGCACTTTGGGAGGCTGAGGTAGGCGGATTGCCTGAGGAGTTTGAGACCAGCCTAGGCAACATGGTGAAACCCCA
TCTCTACTAAAAATACAAAAAATTAGCCTGGCATAGTGGCACACGCCTGTAATCCCAGCTACTTGGGAGGCTGAGGCATGAGAATCGCTTGA
ACCTGGGAGGCGGAGGTTGCAGTGAGCCAAGATCGTACCACTACACTCCAGCCTGGGTGACAGAGCAAGACTCTGTCTCAAAAAACGACAGT
ATATGGAGCAGAGTGAGCTCTGTATTCTATTTAAGTAACATGTTTTTTCCCCACTTCCTCCAATGACTCAAGTATCCATTGATCTGAGCTCT
GGAATTGCTTTGGGGTTCACATTCTTAAACTCCAAGGTTCTGAGCTTCTCATGCTGATAGCCCAAAATCTCAAGCTTTGACATATCCAAGAA
TCTAAGCAAAAGGCTACTAACAGTTATTGATCATTGACTGTGTGCCAGGTACCTCATTCCACCTTACAACCCTATGAGGTCGGGACCCTTAC
TGTGCCCATTCTGTAGATTGGGAAACTGAGGCCCAGAGAGGTGAGGTCCCTTGCCCACTGTCCCAGAGCAGTAATGCCAGGATTTGAACCCA
GGAGGGGCTTTGGGAAGAGCCTGCGAGTGGGTGTGGGGGTCTGGGGCAGGGCTATAGATGGGAGGATCCGTAACACCCCTGTCTGCTCCAGC
TCTCCCTGGACCTCTTCTACACGGAAGACGAGATCTATGAGCTTTCTTATGCCCGGGAGCCGCGTTGTCCCAAGAGCCTGGTGAGACCCAAC
CCCTGGCCTCGGACTCCCCCAATTACCTCGAACCCCCCCTAAGCCTATGCAGTGACTAACTGTGCTCCTCTGTCCCCGCCCCAGCCACCCTC
CCCCTTCAATGCACCTCTGGTGGTGGAGTGGGCCCCTGGTGTGACACCCAAGCCGGACAGGGTCACACTGGGTCGGCATGTGGAGCAGCTGG
TGGAGGTAAGGAGGGGAGGGTGGCCCGGCCGCCAGCAGCCAGGAGGGAAGACATCTGTAGAACCCTGAGCCTGGAGGTGGGGACTTATGGCC
ATGGGGATCATATTTCGACAACTGGGCCTTATCTGCTTCCCCACACTCCTCTGAAGTCTGTGTTCAAGAATTATGACCCTGAAGGCCGAGGA
ACAATCTCTCAGGAGGACTTTGAGCGACTCTCGGGCAATTTTCCCTTCGCCTGCCATGGGCTTCACCCACCCCCACGCCAGGGGTAAGTGGT
TGTGACTCCTGGATCCTCCGAAGATGAGGACACTGGGGTTGCCTGGACTCCTGGGTCTGGAGGACGTTGGGGCAGGTCATGCTGAGGCTCAG
TTTCTGCCCCCAGGAGAGGATCCTTCAGCAGAGAGGAGCTGACAGGGTACCTGCTCCGGGCCAGCGCCATCTGCTCCAAGTTGGGCCTGGCC
TTCCTGCACACCTTCCATGAGGTCACCTTCCGAAAGCCTACCTTCTGCGACAGCTGCAGTGGCTTCGTGAGCACCCCACCCCATCACCACCC
TTAGGTTCCATCTGTCCTGAAGCCGGCAGTCCGCAAACCCCAGATAACCCCCAAATTCCAGAAATCCTCCAGCTTCAAATCATCCTGTTTCA
TATAAACCCTAAACACCAGACATTCCCTATATCCTGGATAATCTCCAAATGCCAGATAATCCCAAAATCCCAGATAATGTCCACCCCAGACA
ATTGCTGAACCATGGATAATACCTAAAACTCTAGATAGGAGGCCAGGAGTGGTGGCTCACGCCTGTAATCCCAGCACTTGGGGAGGCTGAGG
TGGGCGGATTGCTTGAGCTCAAGAGTTCAAGACCAGCCTAGGGAACATAGCAAGAGAGACCCCGTCTCTACTAAAAATACAAAAATTAGCTG
GATGTGGTGGTGCATACCTGTGATCCCAGCTACTCAGGAGGCTGAGGCACGAGAATTGCTTGAACCCAGGAGGTGGAGGTTGCATGAGCCAA
(Sequence of SEQ ID NO: 2 (Continued))

GATCGCAGCACTGCACTCCAGCTTGGGTGACAGAGCGAGATTCTGTCTCAAAAACAACAACAACAACAACAACAACAACAACAACAACA
ACAAGAACAAACCTGGATAATCTCCAAACCGGGTAGATTATGTTCAAACAGAATCATCCTCCAAATCTCTAGACTCCAGATCATTCAAACTC
TGAATAACTTCCAAATTCAAGAGATCCTCAAACTCTGGCTAGTGGATCAATCTCTCAACCTATAACCCGAGACTAATTCACAAACCTTGGAT
TAGTTCCCACATTCATTTATTCATTCATGAAATATTTACTGAGCACCTCCTGTGTGCCAGGTAATCCTAGGCCCTGGGGTTACAGCAGGAAT
ACAGCAGACAACTCTTGCCCATAGAATTTGCATTTTAGTAACTCTAAATTTTACCTAAATCTAATATCTGAGGTGATTATCTAAACTTGAGA
TAATCTCCAAGTTTCCTTTATTTATTTTGTTAAGAGATGGGTCTTGCTCTATTACCCAGGCTGGAGTGCCGTGGTACGATCATGGTTCATTG
CAGCCTCCAATCCTGAGCTCAAGCGATCCTCCCACCTTGGCCTCCCAACCTCCCACCTTGGCGTCTCAAAGTGCTGGGATTACTGGCATGAG
CCACTGTACCCCACCCACATTCCTTTAATTCCAGTTAAATCTCTAGCCTTGAGAATTTCAAAACCTGGGTAGCTTCCACACTTCCACTCCCC
AGCTGCCCTGCCAATCCACAGCCCTCCCTCTCCTAGGATGGGTAACCTTTTCTCCTTTCTCTTCCCAGCTCTGGGGTGTCACCAAGCAAGGC
TACCGCTGTCGGGGTGAGTGGGGTGTCAGTGAGTGGGAGGGAAGCCTTGGCCCGCAGGCTGGAAGAATGGATGACACAGATCGGACCCCTGC
CGCTGCCTTCCCCCAGAGTGCGGGCTGTGTTGCCACAAACACTGCAGAGACCAGGTGAAGGTAGAATGTAAGAAGAGGCCAGGGGCCAAGGG
CGATGCAGGACCCCCCGGAGCTCCTGTCCCATCCACACCAGCTCCCCATGCCAGCTGTGGTAAGACCTAGAGCACACCGGTGCTGGGAAGGG
GCCTGGGGGAAAGGGGTTGAGTCCAAGGTCAAGAAGAGGTCCTTCCCATCAGACCCACATCCCTTCCATCCACCCACAAACAGGCTCCGAGG
AAAATCACTCCTACACGCTATCCCTGGAGCCTGAGACTGGGTGCCAGCTTCGCCATGCCTGGACCCAGACTGAATCCCCACACCCTTCCTGG
GAAACAGATACGGTGAGGAGAAACCGCCCCTACCCCCAAGTGGATACAGGGGACACAGGCCATTGGCGTGGGGAAGACTGGACAGATTTGGA
GACCCATAGAAAGTACAAATATGCAGAAGTCCTTGACTGCTGGGGTAGGAGGGTATGGATACTGGTTACTTTTTAATATTGATAGAAAAATA
GGCTGGGTGTGGTGGCTCATGCTTGTAATCCCAGGACTTCGGGGAGGCTGAGGTGGGAGGATCACTTGAGGTCAGAAGTTCGAGATCAGCCT
GGCCAACGTGATAAAACCCTATCTCTACTAAAAATACAAAAATTAGCCAAGTGTGATGGCGGGTGCCTGTAGTCCCAGCTACACGGGAGGCT
GAGGCAGGAGAATCACTTGAGCCCGGGAGGCAGAGGCTGTAGTGAGCTGAGATTGTGCCACTGCACACCAGCCTAGGTGACAGAGCAAGACC
CTGTCTCAAAAAAAAAAAAAAAAGAAAAATAGAAGTTTCAACATGTTGGGGGTAGGGTGGGGACAATAACCACTAGGAGAACAGAAAAAAGG
ATTCTAGCCAATAAGATAGCAAGAATAAGGCCAAATATATCAGGAGCTACTGTACGGGTTAATGGAGTAAACGCCACTATTTGAGGGCAGAG
ACTCTCAGATTGGGTTACAAAAAGGATTCCACAGGGGACGTTCATTCATCCACTTACCAGTCTGGGCAGGGAAGTGTTCCTGGCAGTGAAGC
GTCTCCTAGACATCATATGCTCCCCAGATCATCTGTCATCTTTCTTCCTCCCTTCCTCCTTCCAGGTCCCCTGCCCGGTGATGGACCCACCA
TCAACTGCATCCTCCAAGCTGGATTCCTAGACATCTCTTGGTCTCCTCTCTTCCTCACTCCCTTCCCCCAGTCAGTCCTGAGTCCTGCCGTC
AGACTCTGGCAGGGCTCCCAGAGGTAGGCCTCCAGAGGCATCCGCCTTCCCATCCACACTGCCTTTAGTGGTACGTCCGTCATCTTTTTCCC
TGGAAGTGACTTTCCTCTTTTGCATCCTGGTGGATGCTAATCCTGCTCCCTTTCCCTGCACTTAGTATCTGTGCGGCTGTGTGTTGGATACA
TTTAGACTCAGGCCTCATTCTACCAACTCCCTACATCTCCATTTCTTTTTTTTTTTGAGACAGAGTCTTGCTCTGTCACCCAGGCTGGAGTG
CAATGATGCAATCTCAGCTCACTGCAACTTCCGCCTCCTGGGTTCAAGTGATTCTTCTGCCTCAGCCTCCCAAGTAGCTGGGATTACAGGCA
CGCGCCACCATGCCCGGCTAATTTTTGTATTTTTAGTAGAGACGGGGTTTCCCCATGTTGGCCAAGATGGTCTCAAACTCCTGACCTCATGA
TCCACCAGCCTCAGCCTCCCAAAGTGCTGGGAATACAGGCGTGAGCCACCACGCCCAGCTCCTGTTTTTCTGCATAGAATGTGCTCCCTCTG
ACAGCCTATACTTTTTCCCTCTTTATCTCGTTGAACGTCTGTGCTTTCCTCAACAGGAGCATCAACTCCAGGAGAACAGGGATTTGTATCAAT
GTCATTCACTGCTGCATCCCCAGTGCCTAGAACAGGGCTGGCAGGTGGTAGGCGCCTGACAGATGCTTGTCTGATGCCTGAATGTCTCCTTA
CCCATGCCCACGGCACAGGATAGATGTGCTATAGGGCAAAGAACTTTGAGGGTCGAGCAGCAGGGGCCCATTCAGTCCCAGGGAGCAGAGAC
CCCCCCAACCCCTTCACAAACCCCAACACCCCTGACTTGGCCCCCACAGAGAGAGGTCCTACAGCTGTCATAAATTAAATTTATTCTCTGGA
GAACATGGTTCTTGGTATTGACGTGAATGGGAGCAGGGCATTTGGGGACTCAGGAATAGGAGATGGGGGGCTTGGGTGCCAAATACCAAGGA
AAGCCTTAAACGGACTCCCCAGAGATTACCGATAGCATTAGCATCTCATGGAGGACCCCCCGCCCCCGACCACCAGTCCCCCTTTACTTCTT
CTTCTTCTTCTTGCGACCCCAACACTGGGGGCCTCCATCCTCTCTTCGGCTCCTCCAGGTGGGCATGGGAGGCTCCAGCTCATTTGGGCGGC
CCCCCCGGTCTGGAACGTTGCCCATAAGCACCTGAGAGTTTATGAGATTTGAGTTGCAGAGAAAAAAAGTATCTTGGACAAGGGGGCCAGAG
AGCGAAGGGAACAGCATGAGGCTGAAGAAGAAACAGCAGATGTTGACTATTTGAGTACAGTGGCTGGGGGAGGGGGGACTGAACTGGTGGAA
GATACTAGCATGAGCTTTCTTTTTTCTTTTCTTTTTTTTGAGACGGAGTCTCACTCTGTTGCCCAGGCTGGAGTGCAGTGGTGTGATCTCAG
CTCACTGCAACCTCCACCTACCGGGTTCAAGAGATTCTCCTGCCTCAGCCTCCCA

## Claims

1. A compound comprising an antisense oligonucleotide that inhibits RasGRP4 gene expression, or a pharmacologically acceptable salt thereof.

2. A compound comprising an antisense oligonucleotide which consists of 8 to 80 nucleosides complementary to a portion in a nucleobase sequence selected from the group consisting of nucleobase sequences represented by nucleobase position numbers:
237 to 252, 244 to 259, 501 to 516, 666 to 681, 683 to 698, 774 to 789, 889 to 904, 939 to 954, 964 to 979, 968 to 983, 969 to 984, 970 to 985, 971 to 986, 972 to 987, 974 to 989, 976 to 991, 977 to 992, 978 to 993, 979 to 994, 980 to 995, 981 to 996, 982 to 997, 1033 to 1048, 1036 to 1051, 1037 to 1052, 1038 to 1053, 1039 to 1054, 1040 to 1055, 1041 to 1056, 1114 to 1129, 1186 to 1201, 1245 to 1260, 1246 to 1261, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1395 to 1410, 1396 to 1411, 1397 to 1412, 1398 to 1413, 1399 to 1414, 1400 to 1415, 1401 to 1416, 1446 to 1461, 1523 to 1538, 1525 to 1540, 1526 to 1541, 1538 to 1553, 1590 to 1605, 1591 to 1606, 1592 to 1607, 1593 to 1608, 1668 to 1683, 1719 to 1734, 1744 to 1759, 1753 to 1768, 1756 to 1771, 1757 to 1772, 1758 to 1773, 1759 to 1774, 1760 to 1775, 1761 to 1776, 1813 to 1828, 1814 to 1829, 1815 to 1830, 1816 to 1831, 1817 to 1832, 1818 to 1833, 1819 to 1834, 1820 to 1835, 1821 to 1836, 1822 to 1837, 1823 to 1838, 1894 to 1909, 2042 to 2057, 2049 to 2064, 2050 to 2065, 2051 to 2066, and 3059 to 3074 of SEQ ID NO: 1, wherein
the antisense oligonucleotide is at least 80% complementary to the portion in the selected nucleobase sequence of SEQ ID NO: 1, or
a compound comprising an antisense oligonucleotide which consists of 8 to 80 nucleosides complementary to a portion in a nucleobase sequence selected from the group consisting of nucleobase sequences represented by nucleobase position numbers:
640 to 655, 967 to 982, 1037 to 1052, 1231 to 1246, 4838 to 4853, 4845 to 4860, 5575 to 5590, 5802 to 5817, 5918 to 5933, 6395 to 6410, 6544 to 6559, 6639 to 6654, 6771 to 6786, 6921 to 6936, 6938 to 6953, 7029 to 7044, 7033 to 7048, 7206 to 7221, 8439 to 8454, 8489 to 8504, 9361 to 9376, 9439 to 9454, 9632 to 9647, 9736 to 9751, 9740 to 9755, 9741 to 9756, 9742 to 9757, 9743 to 9758, 9744 to 9759, 9746 to 9761, 9748 to 9763, 9749 to 9764, 9750 to 9765, 9751 to 9766, 9752 to 9767, 9753 to 9768, 9754 to 9769, 9805 to 9820, 9808 to 9823, 9809 to 9824, 9810 to 9825, 9811 to 9826, 9812 to 9827, 9813 to 9828, 11814 to 11829, 11886 to 11901, 11945 to 11960, 11946 to 11961, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13468 to 13483, 13469 to 13484, 13470 to 13485, 13471 to 13486, 13472 to 13487, 13473 to 13488, 13474 to 13489, 13615 to 13630, 13692 to 13707, 13694 to 13709, 13695 to 13710, 13902 to 13917, 13903 to 13918, 13904 to 13919, 13905 to 13920, 14094 to 14109, 14145 to 14160, 14170 to 14185, 14179 to 14194, 14182 to 14197, 14183 to 14198, 14184 to 14199, 14185 to 14200, 14186 to 14201, 14187 to 14202, 14868 to 14883, 15529 to 15544, 15530 to 15545, 15531 to 15546, 15532 to 15547, 15533 to 15548, 15534 to 15549, 15535 to 15550, 15536 to 15551, 15537 to 15552, 15538 to 15553, 15539 to 15554, 15705 to 15720, 15787 to 15802, 15969 to 15984, 15976 to 15991, 15977 to 15992, 15978 to 15993, 16578 to 16593, and 17775 to 17790 of SEQ ID NO: 2, wherein
the antisense oligonucleotide is at least 80% complementary to the portion in the selected nucleobase sequence of SEQ ID NO: 2, or a pharmacologically acceptable salt thereof.

3. The compound or the pharmacologically acceptable salt thereof according to claim 2, comprising an antisense oligonucleotide which consists of 8 to 80 nucleosides complementary to a portion in a nucleobase sequence selected from the group consisting of nucleobase sequences represented by nucleobase position numbers:
237 to 252, 244 to 259, 501 to 516, 666 to 681, 683 to 698, 774 to 789, 889 to 904, 939 to 954, 964 to 979, 968 to 983, 969 to 984, 970 to 985, 971 to 986, 972 to 987, 974 to 989, 976 to 991, 977 to 992, 978 to 993, 979 to 994, 980 to 995, 981 to 996, 982 to 997, 1033 to 1048, 1036 to 1051, 1037 to 1052, 1038 to 1053, 1039 to 1054, 1040 to 1055, 1041 to 1056, 1114 to 1129, 1186 to 1201, 1245 to 1260, 1246 to 1261, 1247 to 1262, 1248 to 1263, 1249 to 1264, 1250 to 1265, 1251 to 1266, 1252 to 1267, 1253 to 1268, 1365 to 1380, 1368 to 1383, 1369 to 1384, 1395 to 1410, 1396 to 1411, 1397 to 1412, 1398 to 1413, 1399 to 1414, 1400 to 1415, 1401 to 1416, 1446 to 1461, 1523 to 1538, 1525 to 1540, 1526 to 1541, 1538 to 1553, 1590 to 1605, 1591 to 1606, 1592 to 1607, 1593 to 1608, 1668 to 1683, 1719 to 1734, 1744 to 1759, 1753 to 1768, 1756 to 1771, 1757 to 1772, 1758 to 1773, 1759 to 1774, 1760 to 1775, 1761 to 1776, 1813 to 1828, 1814 to 1829, 1815 to 1830, 1816 to 1831, 1817 to 1832, 1818 to 1833, 1819 to 1834, 1820 to 1835, 1821 to 1836, 1822 to 1837, 1823 to 1838, 1894 to 1909, 2042 to 2057, 2049 to 2064, 2050 to 2065, 2051 to 2066, and 3059 to 3074 of SEQ ID NO: 1, wherein
the antisense oligonucleotide is at least 80% complementary to the portion in the selected nucleobase sequence of SEQ ID NO: 2.

4. The compound or the pharmacologically acceptable salt thereof according to claim 2, comprising an antisense oligonucleotide which consists of 8 to 80 nucleosides complementary to a portion in a nucleobase sequence selected from the group consisting of nucleobase sequences represented by nucleobase position numbers:
640 to 655, 967 to 982, 1037 to 1052, 1231 to 1246, 4838 to 4853, 4845 to 4860, 5575 to 5590, 5802 to 5817, 5918 to 5933, 6395 to 6410, 6544 to 6559, 6639 to 6654, 6771 to 6786, 6921 to 6936, 6938 to 6953, 7029 to 7044, 7033 to 7048, 7206 to 7221, 8439 to 8454, 8489 to 8504, 9361 to 9376, 9439 to 9454, 9632 to 9647, 9736 to 9751, 9740 to 9755, 9741 to 9756, 9742 to 9757, 9743 to 9758, 9744 to 9759, 9746 to 9761, 9748 to 9763, 9749 to 9764, 9750 to 9765, 9751 to 9766, 9752 to 9767, 9753 to 9768, 9754 to 9769, 9805 to 9820, 9808 to 9823, 9809 to 9824, 9810 to 9825, 9811 to 9826, 9812 to 9827, 9813 to 9828, 11814 to 11829, 11886 to 11901, 11945 to 11960, 11946 to 11961, 11947 to 11962, 11948 to 11963, 11949 to 11964, 11950 to 11965, 11951 to 11966, 11952 to 11967, 11953 to 11968, 13438 to 13453, 13441 to 13456, 13442 to 13457, 13468 to 13483, 13469 to 13484, 13470 to 13485, 13471 to 13486, 13472 to 13487, 13473 to 13488, 13474 to 13489, 13615 to 13630, 13692 to 13707, 13694 to 13709, 13695 to 13710, 13902 to 13917, 13903 to 13918, 13904 to 13919, 13905 to 13920, 14094 to 14109, 14145 to 14160, 14170 to 14185, 14179 to 14194, 14182 to 14197, 14183 to 14198, 14184 to 14199, 14185 to 14200, 14186 to 14201, 14187 to 14202, 14868 to 14883, 15529 to 15544, 15530 to 15545, 15531 to 15546, 15532 to 15547, 15533 to 15548, 15534 to 15549, 15535 to 15550, 15536 to 15551, 15537 to 15552, 15538 to 15553, 15539 to 15554, 15705 to 15720, 15787 to 15802, 15969 to 15984, 15976 to 15991, 15977 to 15992, 15978 to 15993, 16578 to 16593, and 17775 to 17790 of SEQ ID NO: 2, wherein
the antisense oligonucleotide is at least 80% complementary to the portion in the selected nucleobase sequence of SEQ ID NO: 2.

5. The compound or the pharmacologically acceptable salt thereof according to claim 2, comprising an antisense oligonucleotide which consists of 8 to 80 nucleosides, and having a nucleobase sequence including at least 8 contiguous nucleobases of any one of nucleobase sequences of SEQ ID NOs: 3 to 123.

6. The compound or the pharmacologically acceptable salt thereof according to claim 5, comprising an antisense oligonucleotide having a nucleobase sequence including any one of nucleobase sequences of SEQ ID NOs: 3 to 123.

7. The compound or the pharmacologically acceptable salt thereof according to claim 5 or 6, comprising an antisense oligonucleotide having any one of nucleobase sequences of SEQ ID NOs: 3 to 123.

8. The compound or the pharmacologically acceptable salt thereof according to any one of claims 5 to 7, comprising an antisense oligonucleotide which consists of 8 to 80 nucleosides and having a nucleobase sequence including at least 8 contiguous nucleobases of any one nucleobase sequence selected from the group consisting of SEQ ID NOs:
4, 5, 6, 8, 10, 11, 12, 13, 14, 15, 16, 17, 19, 20, 21, 22, 23, 24, 25, 26, 27, 28, 29, 30, 31, 32, 34, 35, 36, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 71, 72, 73, 74, 75, 76, 77, 78, 79, 80, 81, 82, 83, 84, 85, 86, 87, 95, 96, 97, 99, 100, 102, 103, 104, 105, 106, 107, 108, 109, 110, 111, 112, 113, 114, 115, 116, 117, 118, 119, 120, 121, 122, and 123.

9. The compound or the pharmacologically acceptable salt thereof according to any one of claims 1 to 8, wherein the antisense oligonucleotide includes a phosphorothioate bond.

10. The compound or the pharmacologically acceptable salt thereof according to any one of claims 1 to 9, wherein the antisense oligonucleotide includes at least one nucleoside selected from the group consisting of a 2'-modified nucleoside and a 2'-4'-bridged nucleoside.

11. The compound or the pharmacologically acceptable salt thereof according to claim 10, wherein the 2'-4'-bridged nucleoside is at least one selected from the group consisting of LNA, ENA, cEt, AmNA, scpBNA, and GuNA.

12. The compound or the pharmacologically acceptable salt thereof according to claim 11, wherein the 2'-4'-bridged nucleoside is LNA.

13. The compound or the pharmacologically acceptable salt thereof according to any one of claims 10 to 12, wherein the 2'-modified nucleoside is at least one selected from the group consisting of a 2'-O-MCE nucleoside, a 2'-O-MOE nucleoside, a 2'-O-NMA nucleoside, and a 2'-O-Me nucleoside.

14. The compound or the pharmacologically acceptable salt thereof according to claim 13, wherein the 2'-modified nucleoside is a 2'-O-MCE nucleoside.

15. The compound or the pharmacologically acceptable salt thereof according to any one of claims 1 to 14, wherein the antisense oligonucleotide includes 5-methylcytosine.

16. The compound or the pharmacologically acceptable salt thereof according to any one of claims 1 to 15, wherein
the antisense oligonucleotide includes a gap segment, a 5' wing segment, and a 3' wing segment,
the gap segment includes at least two nucleosides selected from 5'-modified nucleosides and deoxyribonucleosides, and a 5'-terminal and a 3'-terminal of the gap segment are independently 5'-modified nucleosides or deoxyribonucleosides,
the nucleoside at the 3'-terminal of the 5' wing segment is a 2'-modified nucleoside or a 2'-4'-bridged nucleoside, and is linked to the 5'-terminal of the gap segment, and
the nucleoside at the 5'-terminal of the 3' wing segment is a 2'-modified nucleoside or a 2'-4'-bridged nucleoside, and is linked to the 3'-terminal of the gap segment.

17. The compound or the pharmacologically acceptable salt thereof according to claim 16, wherein
the gap segment includes 5 to 30 deoxyribonucleosides,
the 5' wing segment and the 3' wing segment each independently include 1 to 10 sugar-modified nucleosides independently selected from the group consisting of a 2'-modified nucleoside and a 2'-4'-bridged nucleoside,
each of the wing segments includes at least one phosphorothioate bond, and
each of cytosines of the gap segment and each wing segment is replaced with 5-methylcytosine.

18. The compound or the pharmacologically acceptable salt thereof according to claim 17, wherein
the gap segment includes 8 to 12 deoxyribonucleosides,
the 5' wing segment and the 3' wing segment each independently include 2 to 5 sugar-modified nucleosides independently selected from the group consisting of LNA and a 2'-O-MCE nucleoside, and include at least one 2'-O-MCE nucleoside, and
the gap segment includes at least one phosphorothioate bond.

19. The compound or the pharmacologically acceptable salt thereof according to any one of claims 16 to 18, wherein the 5' wing segment and the 3' wing segment are each independently selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, VVVL, VLLVV, and VVLLV, in which L represents a 2'-4'-bridged nucleoside, and V represents a 2'-modified nucleoside.

20. The compound or the pharmacologically acceptable salt thereof according to any one of claims 1 to 19, wherein the antisense oligonucleotide consists of 15 to 25 nucleosides.

21. The compound or the pharmacologically acceptable salt thereof according to any one of claims 1 to 20, wherein the compound comprising the antisense oligonucleotide includes a functional molecule.

22. The compound or the pharmacologically acceptable salt thereof according to claim 21, wherein the functional molecule is a group derived from a molecule having a function of delivering an antisense oligonucleotide to a target site.

23. The compound or the pharmacologically acceptable salt thereof according to claim 21 or 22, wherein the functional molecule is a lipid selected from the group consisting of cholesterol, a vitamin, a steroid, C5-30 saturated fatty acids, and C5-30 unsaturated fatty acids.

24. The compound or the pharmacologically acceptable salt thereof according to any one of claims 1 to 20, wherein the compound consists of the antisense oligonucleotide.

25. The salt according to any one of claims 1 to 24, wherein the pharmacologically acceptable salt is a sodium salt.

26. A medicinal drug comprising, as an active ingredient, the compound or the pharmacologically acceptable salt according to any one of claims 1 to 25.

27. A drug for treating, preventing, and/or ameliorating a disease or a condition for which a RasGRP4 gene expression inhibitory action is effective, comprising, as an active ingredient, the compound or the pharmacologically acceptable salt according to any one of claims 1 to 25.

28. A RasGRP4 gene expression inhibitor comprising, as an active ingredient, the compound or the pharmacologically acceptable salt according to any one of claims 1 to 25.
